# EUROPÄISCHE PATENTSCHRIFT

(11) **EP 2 114 937 B1**
(45) Veröffentlichungstag und Bekanntmachung des Hinweises auf die Patenterteilung: **29.06.2011**
(21) Anmeldenummer: 08706994.4
(22) Anmeldetag: 11.01.2008
(51) Int. Cl.: C07D 417/14, A61K 31/5415, A61P 3/10, C07D 417/12, C07D 513/04

(54) **PHENOTHIAZIN DERIVATE, VERFAHREN ZU IHRER HERSTELLUNG UND IHRE VERWENDUNG ALS ARZNEIMITTEL**
PHENOTHIAZIN DERIVATES, METHOD FOR THE PRODUCTION THEREOF AND USE THEREOF AS PHARMACEUTICALS
DÉRIVÉ DE PHÉNOTHIAZINE, PROCÉDÉ POUR SA FABRICATION ET UTILISATION DE CELUI-CI EN TANT QUE MÉDICAMENT

(30) Priorität: 26.01.2007 DE 102007005045; 31.08.2007 EP 07291062
(43) Veröffentlichungstag der Anmeldung: 11.11.2009
(73) Patentinhaber: Sanofi-Aventis, 75013 Paris (FR)
(72) Erfinder: KEIL, Stefanie, 65926 Frankfurt am Main (DE); DEFOSSA, Elisabeth, 65926 Frankfurt am Main (DE); SCHOENAFINGER, Karl, 63755 Alzenau (DE); SCHMOLL, Dieter, 65926 Frankfurt am Main (DE); DIETRICH, Axel, 65926 Frankfurt am Main (DE); KUHLMANN, Johanna, 65926 Frankfurt am Main (DE); ENGEL, Karl-Christian, 65926 Frankfurt am Main (DE)
(74) Vertreter: Then, Johann
(86) Internationale Anmeldenummer: PCT/EP2008/000163
(87) Internationale Veröffentlichungsnummer: WO 2008/089892

(56) Entgegenhaltungen:
- WO-A-02/062772
- US-A1- 2005 288 279

## Beschreibung

Die Erfindung betrifft substituierte Phenothiazine sowie deren physiologisch verträgliche Salze.

Es sind bereits Penothiazin Derivate wie zum Beispiel Chlorpromazin (3-(2-Chlor-4*a*,10*a-*dihydro-10*H*-phenothiazin-10-yl)- *N,N*-dimethylpropan- 1-amin) als Neuroleptika bekannt.

WO 02/062 772 beschreibt 10-alkylierte Phenotiazin-Derivate Zur Behandlung von Diabetes II. US 2005/028279 beschreibt halogeniate Phenotiazine gegen übergewicht.

Der Erfindung lag die Aufgabe zugrunde, Verbindungen zur Behandlung von Diabetes zu entwickeln. Diese Verbindungen sollen inbesondere den Blutzuckerspiegel absenken.

Die Erfindung betrifft daher Verbindungen der Formel I, 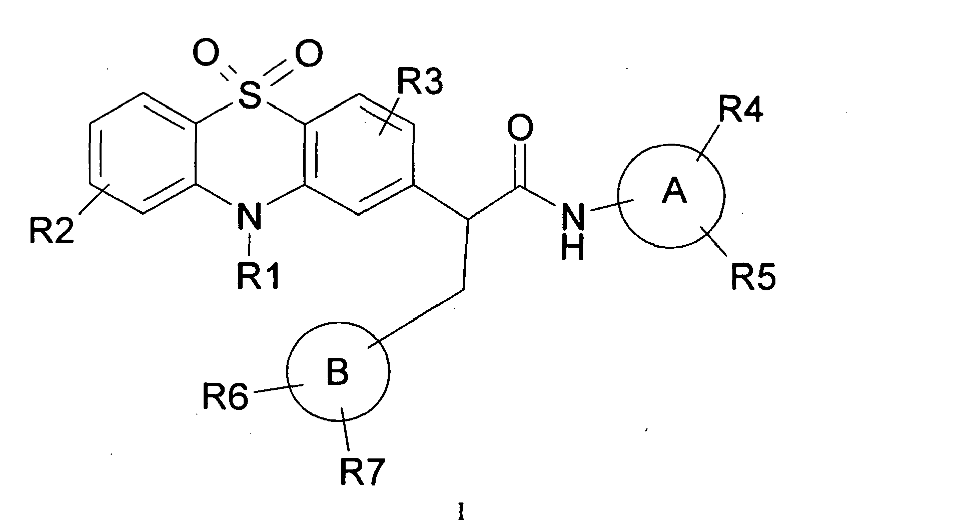 worin bedeuten
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl
- R2, R3: unabhängig voneinander H, F, Cl, Br, CN, NO₂, (C₀-C₆)-Alkylen-COO-(C₀-C₆)- Alkyl, (C₀-C₆)-Alkylen-O-(C₀-C₆)-Alkyl, (C₁-C₆)-Alkyl, (C₀-C₆)-Alkylen-CO- (C₁-C₆)-Alkyl, (C₀-C₆)-Alkylen-Phenyl, SCF₃, SF₅, SCH₃;
- R4, R5: unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁- C₆)-Alkyl, (C₀-C₆)-Alkylen-CO-(C₁-C₆)-Alkyl, (C₀-C₆)-Alkylen-CONH(C₀-C₆)- Alkyl, (C₀-C₆)-Alkylen-CON[(C₀-C₆)-Alkyl]₂, (C₀-C₆)-Alkylen-NH(C₀-C₆)- Alkyl, (C₀-C₆)-Alkylen-NH-COO-(C₀-C₆)-Alkyl, (C₀-C₆)-Alkylen-CON[(C₀-C₆)- Alkyl]-O-(C₀-C₆)-Alkyl, (C₀-C₆)-Alkylen-N[(C₀-C₆)-Alkyl]₂, (C₀-C₆)-Alkylen- Aryl, SF₅, (C₀-C₆)-Alkyl-S(O)ₓ(C₁-C₆)-Alkyl, S(O)ₓ(C₁-C₆)-Alkylen-COO-(C₀- C₆)-Alkyl, S(O)ₓ(C₂-C₆)-Alkylen-O-(C₀-C₆)-Alkyl, -SO₂-NH-(C₀-C₆)-Alkyl, - SO₂-N-[(C₀-C₆)-Alkyl]₂, S(O)ₓ(C₀-C₆)-Alkylen-Heterocyclus, S(O)ₓ(C₁-C₆)- Alkylen-CO-Heterocyclus, -NH-SO₂-(C₁-C₆)-Alkyl, (C₀-C₆)-Alkylen-Cycloalkyl, (C₀-C₆)-Alkylen-Heterocyclus, (C₀-C₆)-Alkylen-Aryl;
- x: 0, 1, 2;
- R6, R7: unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₀-C₆)- Alkyl, (C₀-C₆)-Alkylen-Aryl, SF₅, S(O)ₓ-(C₁-C₆)-Alkyl;
- A: 5 bis 10 gliedriger Heterocyclus, wobei der Heterocyclus mit einem weiteren 5 bis 10 gliedrigen Ring anneliert sein kann;
- B: 4 bis 8 gliedriger Cycloalkylring, ein 4 bis 10 gliedriger Heterocyclus oder ein 6 bis 10 gliedriger Arylring;
sowie deren physiologisch verträgliche Salze.

Bevorzugt sind Verbindungen der Formel I, worin ein oder mehrere Reste folgende Bedeutung haben:
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
- R2, R3: unabhängig voneinander H, F, Cl, Br, CN, NO₂, (C₀-C₆)-Alkylen-COO-(C₀-C₆)- Alkyl, (C₀-C₆)-Alkylen-O-(C₀-C₆)-Alkyl, (C₁-C₆)-Alkyl, (C₀-C₆)-Alkylen-CO- (C₁-C₆)-Alkyl, (C₀-C₆)-Alkylen-Phenyl, SCF₃, SF₅, SCH₃;
- R4, R5: unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁- C₆)-Alkyl, (C₀-C₆)-Alkylen-CO-(C₁-C₆)-Alkyl, (C₀-C₆)-Alkylen-CONH(C₀-C₆)- Alkyl, (C₀-C₆)-Alkylen-CON[(C₀-C₆)-Alkyl]₂, (C₀-C₆)-Alkylen-NH(C₀-C₆)- Alkyl, (C₀-C₆)-Alkylen-NH-COO-(C₀-C₆)-Alkyl, (C₀-C₆)-Alkylen-CON[(C₀-C₆)- Alkyl]-O-(C₀-C₆)-Alkyl, (C₀-C₆)-Alkylen-N[(C₀-C₆)-Alkyl]₂, (C₀-C₆)-Alkylen- Aryl, SF₅, (C₀-C₆)-Alkyl-S(O)ₓ(C₁-C₆)-Alkyl, S(O)ₓ(C₁-C₆)-Alkylen-COO-(C₀- C₆)-Alkyl, S(O)ₓ(C₂-C₆)-Alkylen-O-(C₀-C₆)-Alkyl, -SO₂-NH-(C₀-C₆)-Alkyl, - SO₂-N-[(C₀-C₆)-Alkyl]₂, S(O)ₓ(C₀-C₆)-Alkylen-Heterocyclus, S(O)ₓ(C₁-C₆)- Alkylen-CO-Heterocyclus, -NH-SO₂-(C₁-C₆)-Alkyl, (C₀-C₆)-Alkylen-Cycloalkyl, (C₀-C₆)-Alkylen-Heterocyclus, (C₀-C₆)-Alkylen-Aryl;
- x: 0, 1, 2;
- R6, R7: unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₁-C₆)- Alkyl, (C₀-C₆)-Alkylen-Aryl, SF₅, S(O)ₓ-(C₁-C₆)-Alkyl;
- A: 5 bis 10 gliedriger Heterocyclus, der in alpha Stellung eine -C=N- Bindung enthält, wobei der Heterocyclus mit einem weiteren 5 bis 10 gliedrigen Ring anneliert sein kann;
- B: 4 bis 8 gliedriger Cycloalkylring, ein 4 bis 10 gliedriger Heterocyclus oder ein 6 bis 10 gliedriger Arylring;
sowie deren physiologisch verträgliche Salze.

Besonders bevorzugt sind Verbindungen der Formel I, worin ein oder mehrere Reste folgende Bedeutung haben:
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
- R2, R3: H;
- R4, R5: unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁- C₆)-Alkyl, (C₀-C₆)-Alkylen-CO-(C₁-C₆)-Alkyl, (C₀-C₆)-Alkylen-CONH(C₀-C₆)- Alkyl, (C₀-C₆)-Alkylen-CON[(C₀-C₆)-Alkyl]₂, (C₀-C₆)-Alkylen-NH(C₀-C₆)- Alkyl, (C₀-C₆)- Alkylen-NH-COO-(C₀-C₆)-Alkyl, (C₀-C₆)-Alkylen-CON[(C₀-C₆)- Alkyl]-O-(C₀-C₆)-Alkyl, (C₀-C₆)-Alkylen-N[(C₀-C₆)-Alkyl]₂, (C₀-C₆)-Alkylen- Aryl, SF₅, (C₀-C₆)-Alkyl-S(O)ₓ(C₁-C₆)-Alkyl, S(O)ₓ(C₁-C₆)-Alkylen-COO-(C₀- C₆)-Alkyl, S(O)ₓ(C₂-C₆)-Alkylen-O-(C₀-C₆)-Alkyl, -SO₂-NH-(C₀-C₆)-Alkyl, - SO₂-N-[(C₀-C₆)-Alkyl]₂, S(O)ₓ(C₀-C₆)-Alkylen-Heterocyclus, S(O)ₓ(C₁-C₆)- Alkylen-CO-Heterocyclus, -NH-SO₂-(C₁-C₆)-Alkyl, (C₀-C₆)-Alkylen-Cycloalkyl, (C₀-C₆)-Alkylen-Heterocyclus, (C₀-C₆)-Alkylen-Aryl;
- x: 0, 1, 2;
- R6, R7: unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₁-C₆)- Alkyl, (C₀-C₆)-Alkylen-Aryl, SF₅, S(O)ₓ-(C₁-C₆)-Alkyl;
- A: ein 5 gliedriger Heterocyclus, der in alpha Stellung eine -C=N- Bindung enthält, wobei der Heterocyclus mit einem weiteren 5 bis 10 gliedrigen Ring anneliert sein kann;
- B: 4 bis 8 gliedriger Cycloalkylring, ein 4 bis 10 gliedriger Heterocyclus oder ein 6 bis 10 gliedriger Arylring;
sowie deren physiologisch verträgliche Salze.

Ganz besonders bevorzugt sind Verbindungen der Formel I, worin ein oder mehrere Reste folgende Bedeutung haben:
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
- R2, R3: H;
- R4, R5: unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁- C₆)-Alkyl, (C₀-C₆)-Alkylen-CO-(C₀-C₆)-Alkyl, (C₀-C₆)-Alkylen-CONH(C₀-C₆)- Alkyl, (C₀-C₆)-Alkylen-CON[(C₀-C₆)-Alkyl]₂, (C₀-C₆)-Alkylen-NH(C₀-C₆)- Alkyl, (C₀-C₆)-Alkylen-NH-COO-(C₀-C₆)-Alkyl, (C₀-C₆)-Alkylen-CON[(C₀-C₆)- Alkyl]-O-(C₀-C₆)-Alkyl, (C₀-C₆)-Alkylen-N[(C₀-C₆)-Alkyl]₂, (C₀-C₆)-Alkylen- Aryl, SF₅, (C₀-C₆)-Alkyl-S(O)ₓ(C₁-C₆)-Alkyl, S(O)ₓ(C₁-C₆)-Alkylen-COO-(C₀- C₆)-Alkyl, S(O)ₓ(C₂-C₆)-Alkylen-O-(C₀-C₆)-Alkyl, -SO₂-NH-(C₀-C₆)-Alkyl, - SO₂-N-[(C₀-C₆)-Alkyl]₂, S(O)ₓ(C₀-C₆)-Alkylen-Heterocyclus, S(O)ₓ(C₁-C₆)- Alkylen-CO-Heterocyclus, -NH-SO₂-(C₁-C₆)-Alkyl, (C₀-C₆)-Alkylen-Cycloalkyl, (C₀-C₆)-Alkylen-Heterocyclus, (C₀-C₆)-Alkylen-Aryl;
- x: 0, 1, 2;
- R6, R7: unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₁-C₆)- Alkyl, (C₀-C₆)-Alkylen-Aryl, SF₅, S(O)ₓ-(C₁-C₆)-Alkyl;
- A: Thiazol-2-yl, Pyrazol-3-yl, Pyridin-2-yl, Oxazol-2-yl, Isoxazol-3-yl, Imidazol-2- yl, [1,2,4]Thiadiazol-3-yl, [1,2,4]Thiadiazol-5-yl, [1,3,4]Thiadiazol-3-yl, Pyridin-2-yl, Pyrimidin-2-yl, Pyrimidin-4-yl, Pyrazin-2-yl, [1,2,4]Triazin-3-yl, [1,2,4]Triazin-6-yl, Thiazolo[4,5-b]pyridin-2-yl, Thieno[2,3- d]thiazol-2-yl, Benzothiazol-2-yl, Benzooxazol-2-yl, Benzimidazol-2-yl, Chinolin-2-yl, Isochinolin-3-yl, 4,5,6,7-Tetrahydro-benzothiazol-2-yl, 4,5,6,7- Tetrahydro-benzooxazol-2-yl, 4,5,6,7-Tetrahydro-benzimidazol-2-yl, 4,5,6,7- Tetrahydro-pyrazolo[1,5-a]pyridin-2-yl, 5,6-Dihydro-4H-cyclopentathiazol-2-yl, 4,5-Dihydro-thiazol-2-yl;
- B: Cyclopentyl, Cyclohexyl, Cyclohexenyl, Tetrahydrofuranyl, Tetrahydropyranyl, Tetrahydro-thiopyranyl, Tetrahydrothiophen, Piperidinyl, Phenyl, Pyridyl, Furanyl, Thiophenyl, Thiazolyl, Indanyl, Oxetanyl, Oxazolyl, Pyrazolyl, Isoxazolyl;
sowie deren physiologisch verträgliche Salze.

Weiter ganz besonders bevorzugt sind Verbindungen der Formel I, worin ein oder mehrere Reste folgende Bedeutung haben:
- R1: H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
- R2, R3: H;
- R4, R5: unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁- C₆)-Alkyl, (C₀-C₆)-Alkylen-CO-(C₁-C₆)-Alkyl, (C₀-C₆)-Alkylen-CONH(C₀-C₆)- Alkyl, (C₀-C₆)-Alkylen-CON[(C₀-C₆)-Alkyl]₂, (C₀-C₆)-Alkylen-NH(C₀-C₆)- Alkyl, (C₀-C₆)-Alkylen-NH-COO-(C₀-C₆)-Alkyl, (C₀-C₆)-Alkylen-CON[(C₀-C₆)- Alkyl]-O-(C₀-C₆)-Alkyl, (C₀-C₆)-Alkylen-N[(C₀-C₆)-Alkyl]₂, (C₀-C₆)-Alkylen- Aryl, SF₅, (C₀-C₆)-Alkyl-S(O)ₓ(C₁-C₆)-Alkyl, S(O)ₓ(C₁-C₆)-Alkylen-COO-(C₀- C₆)-Alkyl, S(O)ₓ(C₂-C₆)-Alkylen-O-(C₀-C₆)-Alkyl, -SO₂-NH-(C₀-C₆)-Alkyl, - SO₂-N-[(C₀-C₆)-Alkyl]₂, S(O)ₓ(C₀-C₆)-Alkylen-Heterocyclus, S(O)ₓ(C₁-C₆)- Alkylen-CO-Heterocyclus, -NH-SO₂-(C₁-C₆)-Alkyl, (C₀-C₆)-Alkylen-Cycloalkyl, (C₀-C₆)-Alkylen-Heterocyclus, (C₀-C₆)-Alkylen-Aryl;
- x: 0, 1, 2;
- R6, R7: unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀-C₆)- Alkylen-O-CO-(C₁-C₆)-Alkyl, (C₁-C₆)-Alkyl, (C₀-C₆)-Alkylen-CO-(C₁-C₆)- Alkyl, (C₀-C₆)-Alkylen-Aryl, SF₅, S(O)ₓ-(C₁-C₆)-Alkyl;
- A: Thiazol-2-yl, Pyrazol-3-yl, Pyridin-2-yl, Oxazol-2-yl, Isoxazol-3-yl, Imidazol-2- yl, [1,2,4]Thiadiazol-3-yl, [1,2,4]Thiadiazol-5-yl, [1,3,4]Thiadiazol-3-yl , Pyridin-2-yl, Pyrimidin-2-yl, Pyrimidin-4-yl, Pyrazin-2-yl, [1,2,4]Triazin-3-yl, [1,2,4]Triazin-6-yl, Thiazolo[4,5-b]pyridin-2-yl, Thieno[2,3- d]thiazol-2-yl, Benzothiazol-2-yl, Benzooxazol-2-yl, Benzimidazol-2-yl, Chinolin-2-yl, Isochinolin-3-yl, 4,5,6,7-Tetrahydro-benzothiazol-2-yl, 4,5,6,7- Tetrahydro-benzooxazol-2-yl, 4,5,6,7-Tetrahydro-benzimidazol-2-yl, 4,5,6,7- Tetrahydro-pyrazolo[1,5-a]pyridin-2-yl, 5,6-Dihydro-4H-cyclopentathiazol-2-yl, 4,5-Dihydro-thiazol-2-yl;
- B: Cyclopentyl, Cyclohexyl, Tetrahydrofuranyl, Tetrahydropyranyl, Tetrahydro- thiopyranyl, Piperidinyl, Phenyl, Pyridyl, Furanyl, Thiophenyl, Thiazolyl, Oxazolyl, Pyrazolyl, Isoxazolyl;
sowie deren physiologisch verträgliche Salze.

Weiter ganz besonders bevorzugt sind Verbindungen der Formel I, worin ein oder mehrere Reste folgende Bedeutung haben:
- R1: H, (C₁-C₆)-Alkyl;
- R2, R3: H;
- R4: H, F, Cl, Br, (C₀-C₆)-Alkylen-COO-(C₀-C₆)-Alkyl, (C₀-C₆)-Alkylen-O-(C₀-C₆)- Alkyl, =O, (C₁-C₆)-Alkyl, (C₀-C₆)-Alkylen-Aryl;
- R5: H;
- R6: H;
- R7: H, F, Cl, Br, =O, =N-O-(C₀-C₆)-Alkyl, (C₁-C₆)-Alkyl;
- A: Thiazol-2-yl, Pyrazol-3-yl, Isoxazol-3-yl, [1,2,4]Thiadiazol-3-yl, [1,2,4]Thiadiazol-5-yl, [1,3,4]Thiadiazol-3-yl, Thiazolo[4,5- b]pyridin-2-yl, Thieno[2,3-d]thiazol-2-yl;
- B: Cyclopentyl, Cyclohexyl, Cyclohexenyl, Tetrahydrofuranyl, Tetrahydropyranyl, Tetrahydrothiophenyl, Oxetanyl, Piperidinyl, Indanyl;
sowie deren physiologisch verträgliche Salze.

In einer Ausführungsform sind Verbindungen der Formel I bevorzugt, in denen R1 gleich H ist.

In einer Ausführungsform sind Verbindungen der Formel I bevorzugt, in denen R1 gleich Methyl ist.

In einer Ausführungsform sind Verbindungen der Formel I bevorzugt, in denen A gleich Thiazol-2-yl ist.

In einer Ausführungsform sind Verbindungen der Formel I bevorzugt, in denen A gleich Pyrazol-3-yl ist.

In einer Ausführungsform sind Verbindungen der Formel I bevorzugt, in denen A gleich Isoxazol-3-yl ist.

In einer Ausführungsform sind Verbindungen der Formel I bevorzugt, in denen A gleich [1,2,4]Thiadiazol-3-yl, [1,2,4]Thiadiazol-5-yl oder [1,3,4]Thiadiazol-3-yl ist.

In einer Ausführungsform sind Verbindungen der Formel I bevorzugt, in denen A gleich Pyridazin-2-yl ist.

In einer Ausführungsform sind Verbindungen der Formel I bevorzugt, in denen A gleich Thiazolo[4,5-b]pyridin-2-yl ist.

In einer Ausführungsform sind Verbindungen der Formel I bevorzugt, in denen A gleich Thieno[2,3-d]thiazol-2-yl ist.

In einer Ausführungsform sind Verbindungen der Formel I bevorzugt, in denen B gleich Cyclopentyl ist.

In einer Ausführungsform sind Verbindungen der Formel I bevorzugt, in denen B gleich Cyclohexyl ist.

In einer Ausführungsform sind Verbindungen der Formel I bevorzugt, in denen B gleich Cyclohexenyl ist.

In einer Ausführungsform sind Verbindungen der Formel I bevorzugt, in denen B gleich Tetrahydrofuranyl ist.

In einer Ausführungsform sind Verbindungen der Formel I bevorzugt, in denen B gleich Tetrahydropyranyl ist.

In einer Ausführungsform sind Verbindungen der Formel I bevorzugt, in denen B gleich, Tetrahydrothiophenyl ist.

In einer Ausführungsform sind Verbindungen der Formel I bevorzugt, in denen B gleich, Oxetanyl ist.

In einer Ausführungsform sind Verbindungen der Formel I bevorzugt, in denen B gleich, Piperidinyl ist.

In einer Ausführungsform sind Verbindungen der Formel I bevorzugt, in denen B gleich, Indanyl ist.

In einer Ausführungsform sind Verbindungen der Formel I bevorzugt, in denen R6 gleich H ist.

In einer Ausführungsform sind Verbindungen der Formel I bevorzugt, in denen R6 gleich (C₁-C₆)-Alkyl ist.

In einer Ausführungsform sind Verbindungen der Formel I bevorzugt, in denen R6 gleich =O ist.

In einer Ausführungsform sind Verbindungen der Formel I bevorzugt, in denen R6 gleich -OH ist.

In einer Ausführungsform sind Verbindungen der Formel I bevorzugt, in denen R6 gleich =NOH ist.

In einer Ausführungsform sind Verbindungen der Formel I bevorzugt, in denen R6 gleich F, Cl oder Br ist.

In einer Ausführungsform sind Verbindungen der Formel I bevorzugt, in denen R7 gleich H ist.

In einer Ausführungsform sind Verbindungen der Formel I bevorzugt, in denen R7 gleich =O ist.

In einer Ausführungsform sind Verbindungen der Formel I bevorzugt, in denen R4 gleich F, Cl oder Br ist.

In einer Ausführungsform sind Verbindungen der Formel I bevorzugt, in denen R4 gleich H ist.

In einer Ausführungsform sind Verbindungen der Formel I bevorzugt, in denen R4 gleich (C₁-C₆)-Alkyl ist.

In einer Ausführungsform sind Verbindungen der Formel I bevorzugt, in denen R4 gleich =O ist.

In einer Ausführungsform sind Verbindungen der Formel I bevorzugt, in denen R4 gleich (C₀-C₆)-Alkylen-COO-(C₀-C₆)-Alkyl ist.

In einer Ausführungsform sind Verbindungen der Formel I bevorzugt, in denen R4 gleich (C₀-C₆)-Alkylen-O-(C₀-C₆)-Alkyl ist.

In einer Ausführungsform sind Verbindungen der Formel I bevorzugt, in denen R4 gleich Benzyl ist.

Die Erfindung bezieht sich auf Verbindungen der Formel I, in Form ihrer Racemate, racemischen Mischungen und reinen Enantiomere sowie auf ihre Diastereomere und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Unter der Defintion (C₀-C₆)-Alkylen- wird verstanden, dass entweder eine Bindung oder eine (C₁-C₆)-Alkylen Gruppe vorhanden sein kann.

Unter der Definition -(C₀-C₆)-Alkyl wird verstanden, dass entweder ein Wasserstoff oder eine (C₁-C₆)-Alkyl Gruppe vorhanden sein kann.

Unter "Anellierung" oder "anelliert" wird verstanden, dass ein weiteres Ringssystem ankondensiert ist. Das weitere ankondensierte Ringssystem kann aromatisch oder nichtaromatisch und carbocyclisch oder heterocyclisch sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamolsalz (2-Amino-2-hydroxymethyl-1,3-propandiol), Diethanolaminsalz, Lysinsalz oder Ethylendiaminsalz.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können als physiologisch funktionelle Derivate der Verbindungen der Formel I vorliegen. Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C6)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.

Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂),-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH, NH(C₁-C₆)-Alkyl, N[(C₁-C₆)-Alkyl]₂, SO₂-CH₃, COOH, COO-(C₁-C₆)-Alkyl oder CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl, N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus,; PO₃H₂, SO₃H, SO-NH₂, SO₂H(C₁-C₆)-Alkyl, SON[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl)-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyctus, wobei n = 0 - 6 sein kann, wobei der Arylrest oder heterocyclische Rest ein bis 3-fach substituiert sein kann mit F, Cl, Br, I, OH, CF₃, NO₂ CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Unter Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ringsystem anelliert ist..

Geeignete "Heterocyclen" bzw. "heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydrobenzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterocyclen.

Die Heterocyclen bzw. heterocyclischen Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C6)-Alkyl, O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C6)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ wobei n = 0 - 6 sein kann und der Arylrest oder heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₂-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N[(C₁-C₆)-Alkyl]-CO-(C₁-C₆)-Alkyl, N[(C₁-C₆)-Alkyl]-COO-(C₁-C₆)-Alkyl, N[(C₁-C₆)₋Alkyl]-CO-Aryl, N[(C₁-C₆)-Alkyl]-CO-Heterocyclus, N(C₁-C₆)-Alkyl-COO-Aryl, N[(C₁-C₆)-Alkyl]-COO-Heterocyclus, N[(C₁-C₆)-Alkyl]-CO-NH-(C₁-C₆)-Alkyl), N[(C₁-C₆)-Alkyl]-CO-NH-Aryl, N[(C₁-C6)-Alkyl]-CO-NH-Heterocyclus, N[(C₁-C₆)-Alkyl]-CO-N-[(C₁-C₆)-Alkyl]₂, N[(C₁-C₆)-Alkyl]-CO-N[(C₁-C₆)-Alkyl)]-Aryl, N[(C₁-C₆)-Alkyl]-CO-N[(C₁-C₆)-Alkyl]-Heterocyclus, N[(C₁-C₆)-Alkyl]-CO-N-(Aryl)₂, N[(C₁-C₆)-Alkyl]-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl, N(Aryl)-CO-NH-Aryl, N(Heterocyclus)-CO-NH-Aryl, N(Aryl)-CO-N-[(C₁-C₆)-Alkyl]₂, N(Heterocyclus)-CO-N-[(C₁-C₆)-Alkyl]₂, N(Aryl)-CO-N[(C₁-C₆)-Alkyl]-Aryl, N(Heterocyclus)-CO-N[(C₁-C₆)-Alkyl]-Aryl, N(Aryl)-CO-N-(Aryl)₂, N(Heterocyclus)-CO-N-(Aryl)₂, Aryl, O-(CH₂)ₙAryl, O-(CH₂)ₙ-Heterocyclus, wobei n = 0 - 6 sein kann, wobei der Arylrest oder heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N[(C₁-C₆)-Alkyl]₂, SO₂-CH₃, COOH, COO-(C₁-C₆)-Alkyl oder CONH₂.

Verbindungen der Formel I aktivieren den Glucosestoffwechsel in Glucokinase-exprimierenden Zellen. Sie sind daher gut zur Behandlung und Verhinderung erhöhter Blutzuckerspiegel, Obesitas und des metabolischen Syndroms geeignet (Sagen et al. Diabetes 55, 1713-1722, Levin et al. Diabetes (2006), S122-S130, Matschinsky et al (2006) 55, 1-12).

Aufgrund der Aktivierung von Glucokinase können die Verbindungen der Formel I auch zur Behandlung bzw. Prävention weiterer Krankheiten und Leiden in einem Säugetier, vorzugsweise einem Menschen, geeignet sein, die durch erhöhten Blutzuckerspiegel, Übergewicht oder durch verringerte Aktivität von Glucokinase hervorgerufen werden.

Die Verbindungen der vorliegenden Erfindung eignen sich insbesondere zur Behandlung und/oder Prävention von:
1.
   - Glucoseverwertungsstörungen und Störungen des Fettsäurestoffwechsels
   - Störungen, bei denen Insulinresistenz eine Rolle spielt

   Diabetes mellitus, insbesondere Typ-2-Diabetes, einschließlich der Prävention der damit verbundenen Folgeerkrankungen.
   Besondere Aspekte in diesem Zusammenhang sind
   - Hyperglykämia,
   - Verbesserung der Insulinresistenz,
   - Verbesserung der Glucosetoleranz,
   - Schutz der β-Zellen der Bauchspeicheldrüse
   - Prävention makro- und mikrovaskulärer Erkrankungen
2. Übergewicht und dessen Folgen wie beispielsweise Dyslipidemien, Atherosklerose, koronare Herzkrankheit, zerebrovaskuläre Erkrankungen usw., insbesondere (jedoch nicht darauf beschränkt) die, die durch einen oder mehrere der folgenden Faktoren gekennzeichnet sind:
   - hohe Plasmatriglyceridkonzentrationen, hohe postprandiale Plasmatriglyceridkonzentrationen,
   - niedrige HDL-Cholesterinkonzentration
   - niedrige ApoA-Lipoproteinkonzentrationen
   - hohe LDL-Cholesterinkonzentrationen
   - kleine dichte LDL-Cholesterinpartikel hohe ApoB-Lipoproteinkonzentrationen
3. Verschiedene andere Leiden, die mit dem metabolischen Syndrom bzw. Syndrom X assoziert sein können, wie
   - Zunahme des Bauchumfangs
   - Dyslipidämie (z.B. Hypertriglyceridämie und/oder niedriges HDL)
   - Insulinresistenz
   - Hyperkoagulabilität
   - Hyperurikämie
   - Mikroalbuminämie
   - Thrombosen, hyperkoagulabile und prothrombotische Zustände (arteriell und venös)
   - Bluthochdruck
   - Herzinsuffizienz, beispielsweise (jedoch nicht darauf beschränkt), nach Herzinfarkt, hypertensiver Herzkrankheit oder Kardiomyopathie
4. Primäre Hypertriglyceridämie oder sekundäre Hypertriglyceridämien nach familiärer Retikulohistiozytose
   Lipoproteinlipasemangel
   Hyperlipoproteinämien
   Apolipoproteinmangel (z.B. ApoCII- oder ApoE-Mangel)
5. Genetisch bedingte verringerte Aktivität von Glucokinase, insbesondere dem so genannten MODY2
6. Krankheiten bzw. Leiden, die mit neurologischen, psychiatrischen oder Immunerkrankungen bzw. -leiden in Zusammenhang stehen

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel 1. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel 1 abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus® (siehe www.lantus.com) oder HMR 1964 oder Levemir® (insulin detemir) oder solche, wie sie in W02005005477 (Novo Nordisk) beschrieben sind, schnell wirkende Insuline (siehe US 6,221,633), inhalierbare Insuline, wie z. B. Exubera ® oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe, Biguanidine,
Meglitinide,
Oxadiazolidindione,
Thiazolidindione,
Glukosidase-Inhibitoren,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden oder die, die in W02006045799 beschrieben sind (Solvay),
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11β-HSD1,
Inhibitoren der Protein-Tyrosin-Phosphatase-1B (PTP1B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699
verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, W02005042692, W02005005453), MD-0727 (Microbia Inc., WO2005021497, WO2005021495) oder mit Verbindungen, wie in W02002066464 (Kotobuki Pharmaceutical Co. Ltd.) oder WO2005044256 oder W02005062824 (Merck & Co.) oder WO2005061451 und W02005061452 (AstraZeneca AB) und W02006017257 (Phenomix) oder WO2005033100 (Lipideon Biotechnology AG) beschrieben, verabreicht.

Bei einer Ausf¨Uhrungsform der Erfindung wird die Verbindung der Formel I in Kombination mit Vytorin™, einer festen Kombination von Ezetimibe mit Simvastatin, verabreicht.
Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon) verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit duetactTM, einer festen Kombination von Pioglitazon Hydrochlorid mit Glimepirid, verabreicht.

Bei einer Ausführungsform der Erfindung wird die Verbindung der Formel I in Kombination mit Avandamet®, einer festen Kombination von Rosiglitazon Maleat mit Metformin Hydrochlorid, verabreicht.

Bei einer Ausführungsform der Erfindung wird die Verbindung der Formel I in Kombination mit PPAR alpha Agonisten, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht
Bei einer weiteren Ausführungsform der Erfindung wird die Verbindung der Formel I in Kombination mit einem Aktivator der AMP-aktivierten Proteinkinase (AMPK), wie z. B. A-769662 oder solchen Verbindungen wie sie in US20050038068 beschrieben sind, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide , BMS-201038, R-103757 oder solchen wie in WO2005085226, WO2005121091, W02006010423 beschrieben, verabreicht.

Bei einer Ausführungsform der Erfindung wird die Verbindung der Formel I in Kombination mit einem CETP-Inhibitor, wie z.B. Torcetrapib oder JTT-705 oder solchen wie sie in WO2006002342, W02006010422, WO2006012093 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in W02005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, β-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in W02005077907 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Aqonisten des GPR109A (HM74A Rezeptor Agonisten), wie z.B. Nicotinsäure oder "extended release niacin" in Verbindung mit MK-0524A oder solchen Verbindungen, wie sie in WO2006045565, W02006045564, W02006069242 beschrieben sind, verabreicht.

Bei einer anderen Ausführungsform der Erfindung wird die Verbindung der Formel I in Kombination mit einem Aqonisten des GPR116, wie sie z.B. in WO2006067531, W02006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht.

Bei einer Ausführungsform wird die Verbindung der Formel I in Kombination mit einer die Insulinsekretion verstärkende Substanz, wie z. B. KCP-265 (W02003097064), verabreicht.

Bei einer Ausführungsform wird die Verbindung der Formel I in Kombination mit Agonisten des glucose-abhängigen insulinotropischen Rezeptors (GDIR) wie z. B. APD-668 verabreicht

Bei einer Ausführungsform wird die Verbindung der Formel I in Kombination mit einem Biguanid, wie z.B. Metformin, verabreicht.

Bei wieder einer anderen Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, W02004007455, W02005073229-31, W02005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, W02005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, W02005044801, W02006016194, W02006058923 beschrieben sind, verabreicht.

Bei einer Ausführungsform wird die Verbindung der Formel I in Kombination mit einem Inhibitor der Glukoneogenese, wie z. B. FR-225654, verabreicht.
Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in W02006023515 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in W02004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X, KRP-104, DP-893 oder wie sie in W02003074500, WO2003106456, W0200450658, WO2005058901, WO2005012312, WO2005/012308, W02006039325, W02006058064, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Januvia™, einer festen Kombination von Sitagliptin Phosphat mit Metformin hydrochlorid, verabreicht.

Bei einer Ausführungsform wird die Verbindung der Formel I in Kombination mit Hemmstoffen der 11-beta-Hydroxysteroid-Dehydrogenase-1 (11β-HSD1), wie z. B. BVT-2733, JNJ-25918646, INCB-13739 oder solche, wie sie z. B. in WO200190090-94, WO200343999, WO2004112782, W0200344000, W0200344009, WO2004112779, WO2004113310, WO2004103980, WO2004112784, WO2003065983, WO2003104207, WO2003104208, WO2004106294, WO2004011410, W02004033427, WO2004041264, WO2004037251, WO2004056744, WO2004058730, WO2004065351, W02004089367, W02004089380, W02004089470-71, W02004089896, WO2005016877, W02005097759, W02006010546, WO2006012227, WO2006012173, W02006017542, W02006034804, W02006040329, WO2006051662, W02006048750, W02006049952, WO2006048331, W02006050908, W02006024627, W02006040329, W02006066109 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, W0200117516, WO2004506446, W02005012295, WO2005116003, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, W0200452902, PCT/EP2005/005959, WO2005085237, JP2004359630, WO2005121161, WO2006018150, WO2006035796, WO2006062224, WO2006058597 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Modulatoren des GPR119b, wie sie z. B. in WO2004041274 beschrieben sind, verabreicht.

Bei einer Ausführungsform wird die Verbindung der Formel I in Kombination mit Modulatoren des GPR119, wie sie z. B. in WO2005061489 (PSN-632408) beschrieben sind, verabreicht. Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262, WO200372197, WO2003072197, WO2005044814, W02005108370, JP2006131559 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in W02004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, W02005085230, PCT/EP2005/005346, W02003078403, W02004022544, WO2003106410, W02005058908, US2005038023, WO2005009997, US2005026984, W02005000836, WO2004106343, EP1460075, WO2004014910, W02003076442, WO2005087727, WO2004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in W02001000610, W02001030774, W02004022553, W02005097129 beschrieben sind, verabreicht.

Bei einer Ausführungsform wird die Verbindung der Formel I in Kombination mit Modulatoren des Glucocorticoidrezeptors, wie sie z. B. in W02005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804, S-2367 oder wie sie z. B. in W02006001318 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in W02005080424 beschrieben sind;
CB1R (Cannabinoid Rezeptor 1) Antagonisten (wie z.B. Rimonabant, SR147778 oder solche wie sie in z. B. EP 0656354, WO 00/15609, WO 02/076949, WO2005080345, W02005080328, W02005080343, W02005075450, W02005080357, W0200170700, W02003026647-48, W0200302776, WO2003040107, WO2003007887, W02003027069, US6,509,367, WO200132663, W02003086288, WO2003087037, WO2004048317, WO2004058145, W02003084930, WO2003084943, W02004058744, WO2004013120, WO2004029204, W02004035566, W02004058249, W02004058255, WO2004058727, W02004069838, US20040214837, US20040214855, US20040214856, WO2004096209, WO2004096763, W02004096794, WO2005000809, WO2004099157, US20040266845, WO2004110453, WO2004108728, WO2004000817, W02005000820, US20050009870, W0200500974, W02004111033-34, W0200411038-39, WO2005016286, WO2005007111, W02005007628, US20050054679, WO2005027837, W02005028456, W02005063761-62, W02005061509, W02005077897, WO2006047516, WO2006060461, W02006067428, WO2006067443 beschrieben sind);
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxoethyl]-amid; (WO 01/91752)) oder LB53280, LB53279, LB53278 oder THIQ, MB243, RY764, CHIR-785, PT-141 oder solche wie sie in W02005060985, W02005009950, W02004087159, WO2004078717, WO2004078716, W02004024720, US20050124652, WO2005051391, WO2004112793, WOUS20050222014, US20050176728, US20050164914, US20050124636, US20050130988, US20040167201, W02004005324, WO2004037797, WO2005042516, WO2005040109, WO2005030797, US20040224901, WO200501921, WO200509184, W02005000339, EP1460069, WO2005047253, WO2005047251, EP1538159, W02004072076, WO2004072077, WO2006021655-57 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in W0200196302, WO200185693, W02004085403, WO2005075458, WO2006067224 beschrieben sind); Histamin H3 Rezeptor Agonisten (z. B. 3-Cyclohexyl-1-(4,4-dimethyl-1,4,6,7-tetrahydroimidazo[4,5-c]pyridin-5-yl)-propan-1-on Oxalsäuresalz (WO 00/63208) oder solche, wie sie in W0200064884, W02005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, W02004012648, WO2003015769, WO2004072025, WO2005070898, WO2005070925, WO2004039780, W02003033476, WO2002006245, W02002089729, WO2002002744, W02003004027, FR2868780, WO2006010446, W02006038680, W02006044293, W02006044174 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034 beschrieben sind;
Serotonin-Wiederaufnahme-Inhibitoren (z.B. Dexfenfluramine);
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. Lorcaserin Hydrochlorid (APD-356) oder BVT-933 oder solche, wie sie in WO200077010, WO2007700-1-02, WO2005019180, W02003064423, WO200242304, W02005082859 beschrieben sind);
5-HT6 Rezeptor Antagonisten, wie sie z.B. in W02005058858 beschrieben sind; Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylaminoethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, W02005044250, W02005072740, JP2005206492, W02005013907, W02006004200, W02006019020, W02006064189 beschrieben;
Inhibitoren der Fettsäuresynthase (FAS) wie z.B. C75 oder solchen, wie in W02004005277 beschrieben;
Oxyntomodulin;
Oleoyl-Estron oder Agonisten des Schilddrüsenhormonrezeptors (thyroid hormone receptor agonists) wie z. B: KB-2115 oder solche, wie in W020058279, WO200172692, WO200194293, W02003084915, W02004018421, WO2005092316 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1, eines Mitglieds der humanen Sirtuinenzymfamilie.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform wird die Verbindung der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

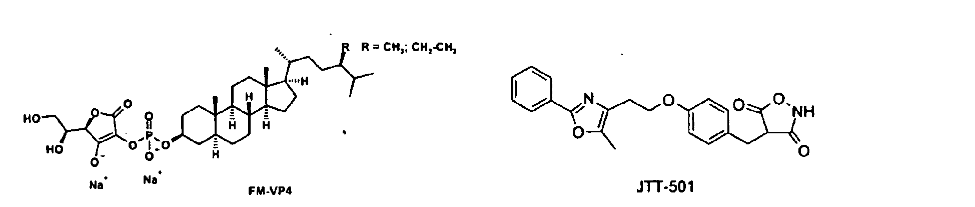

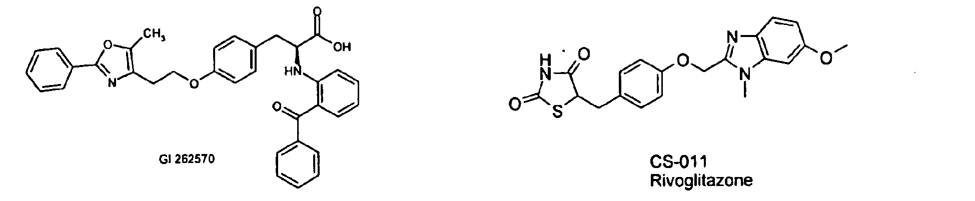

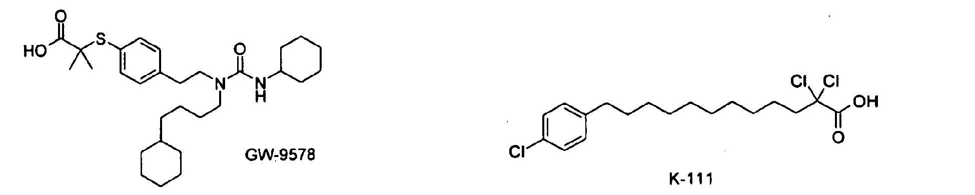

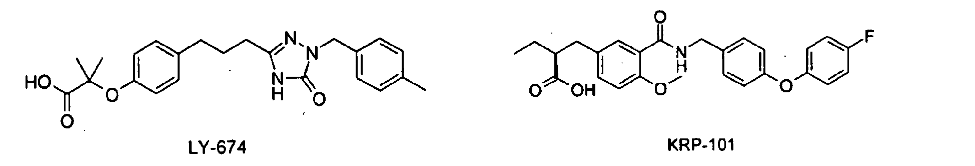

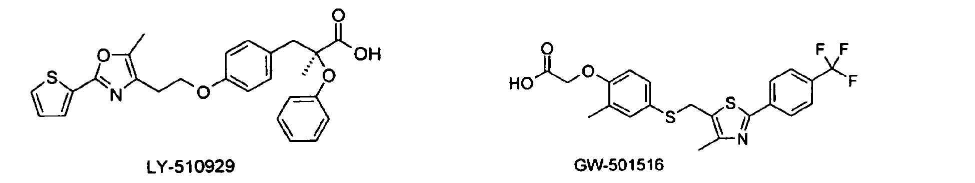

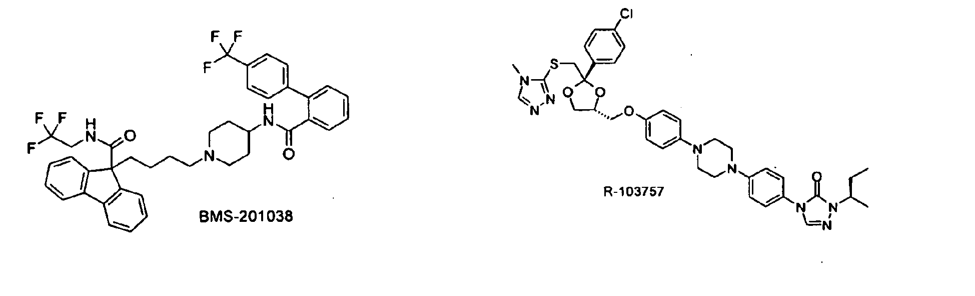

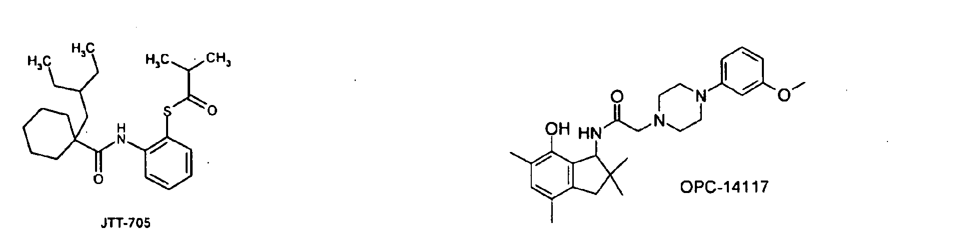

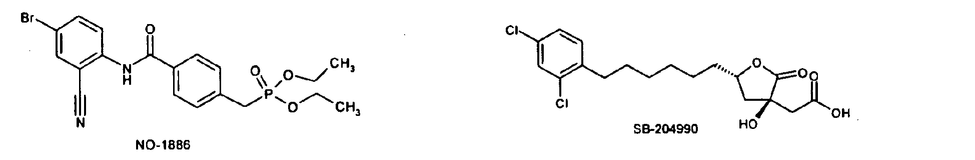

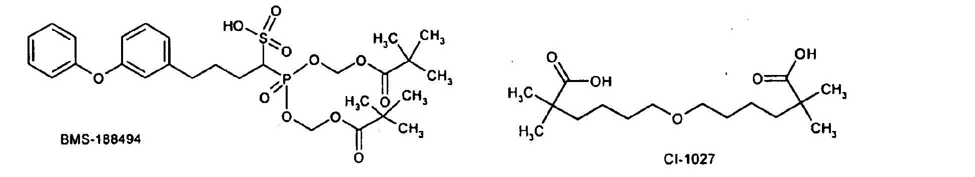

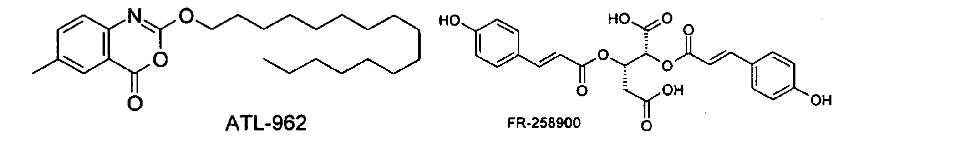

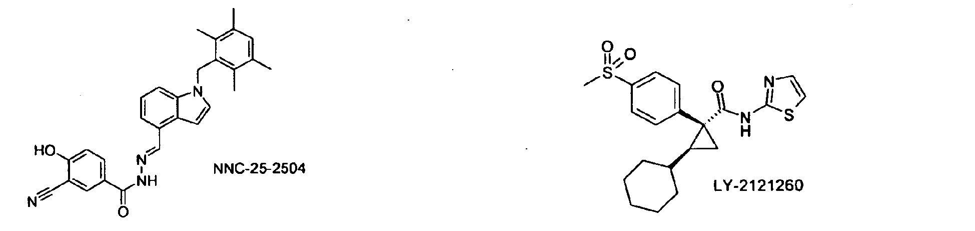

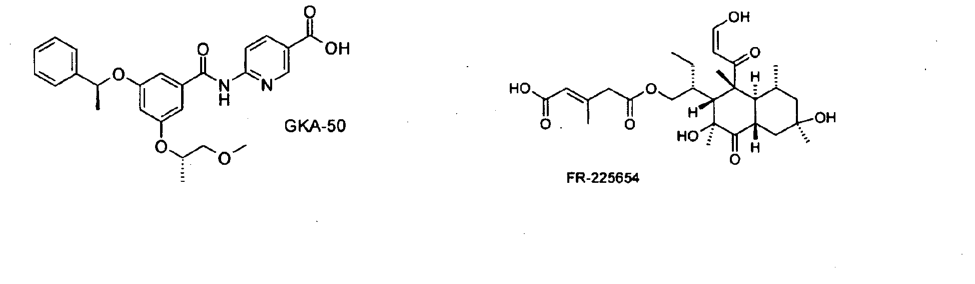

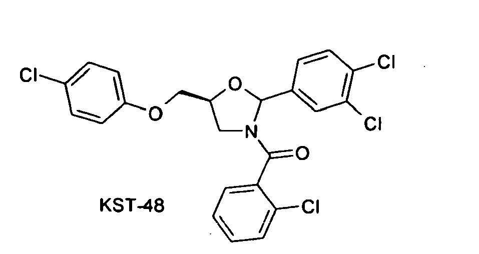

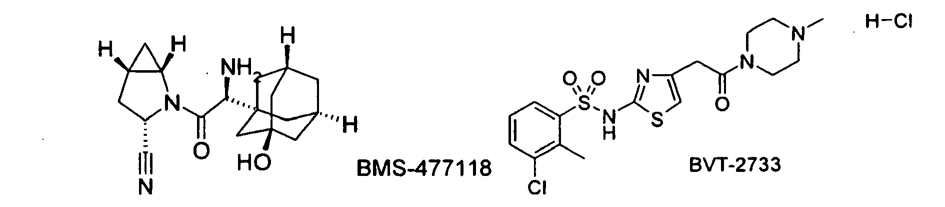

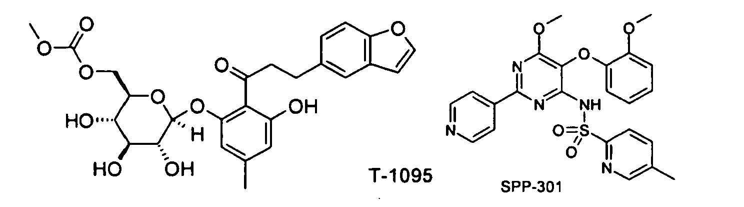

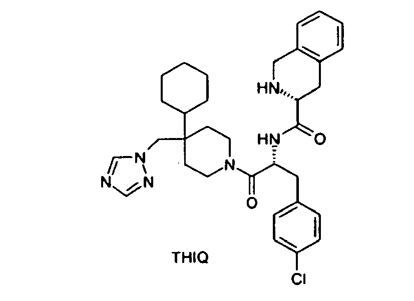

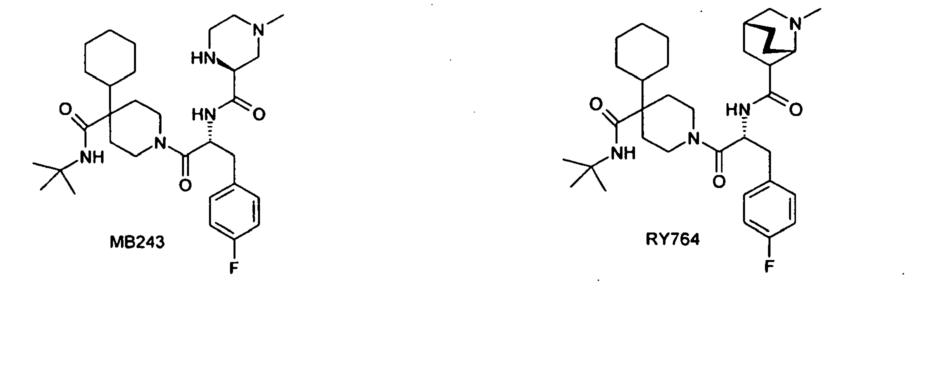

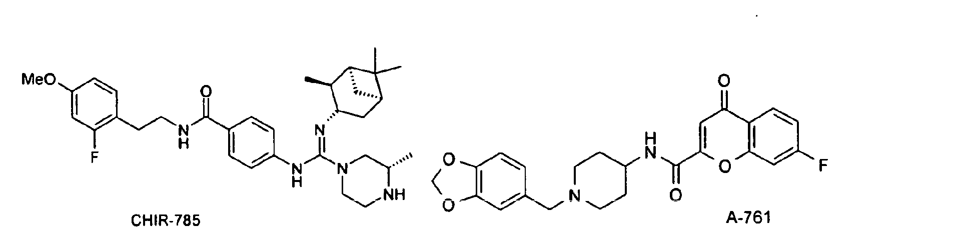

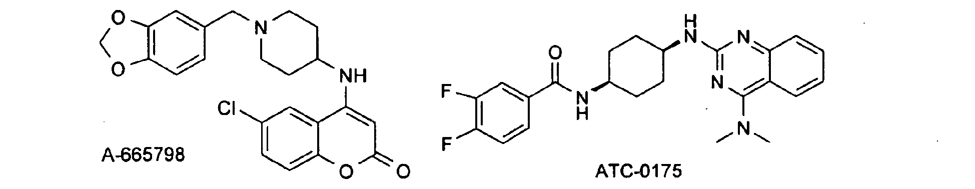

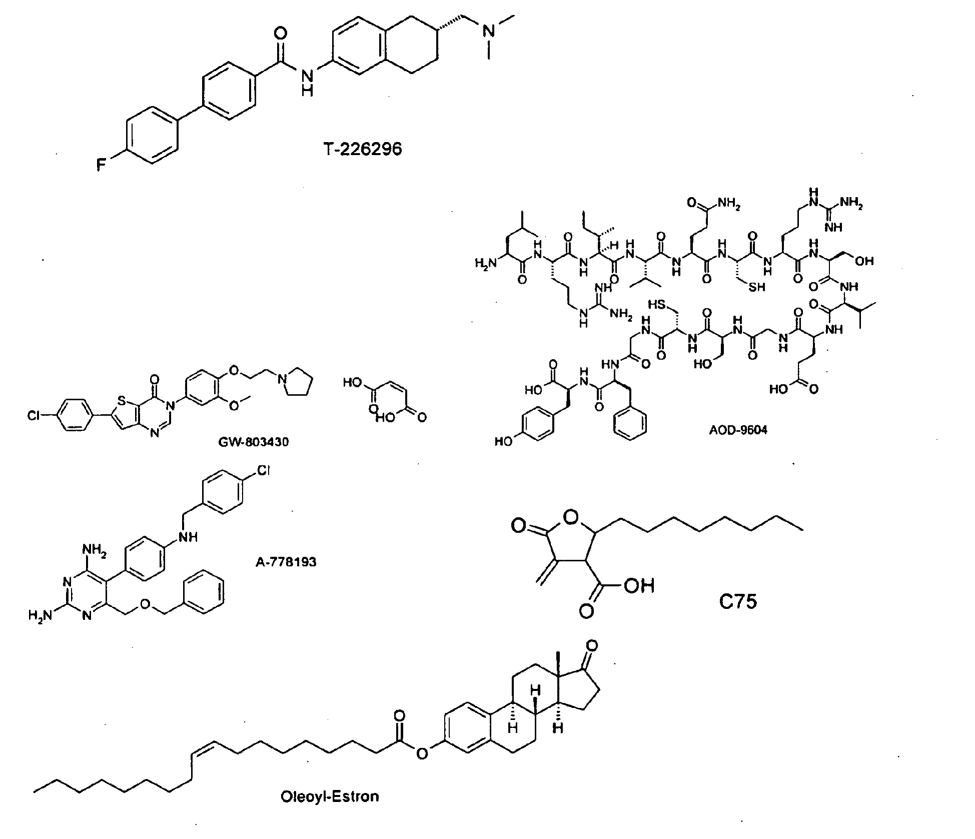

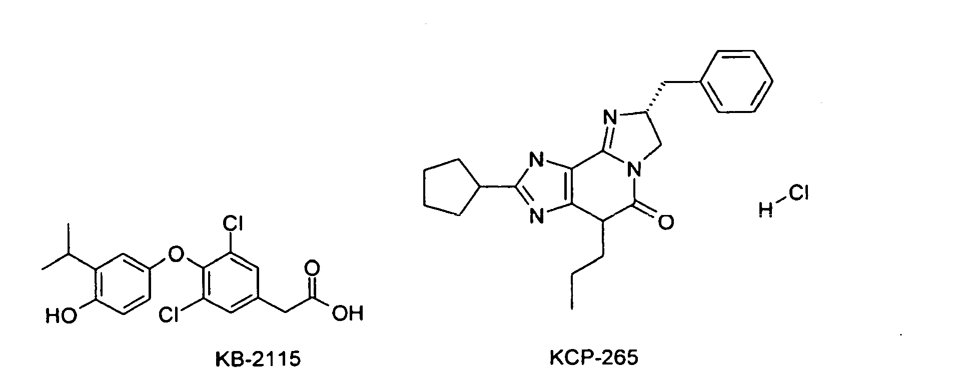

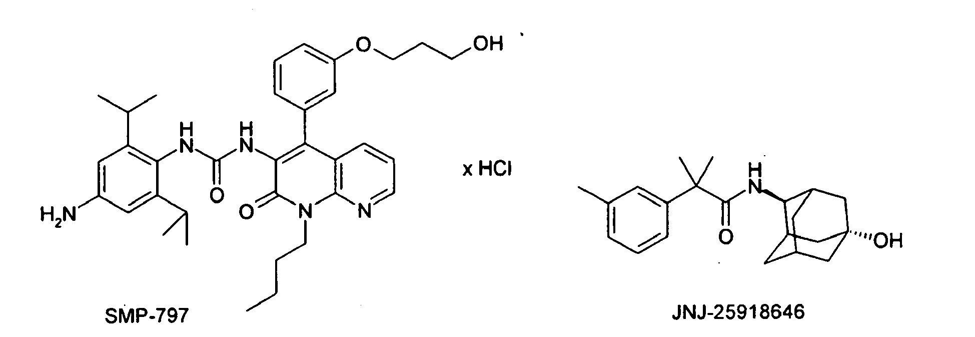

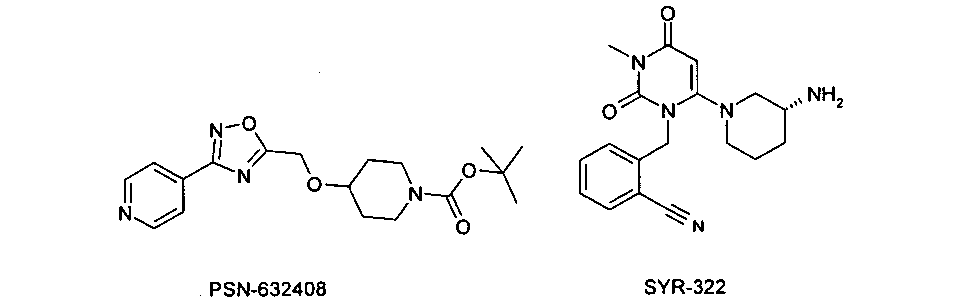

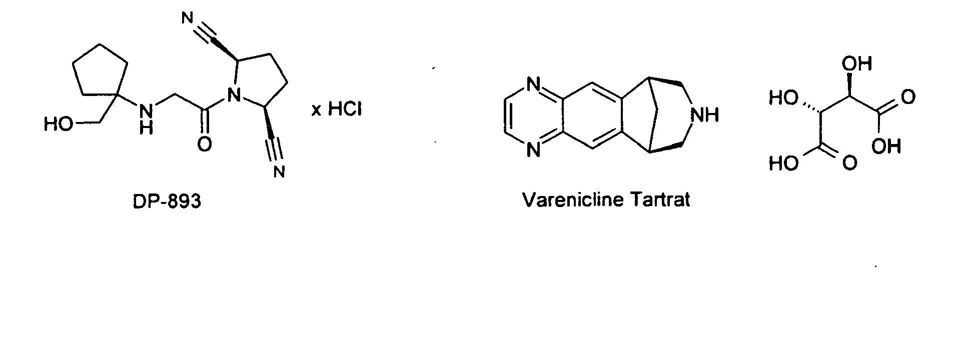

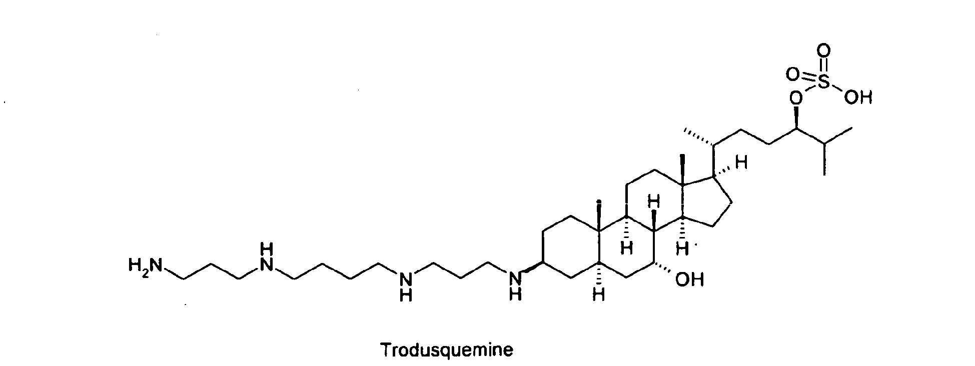

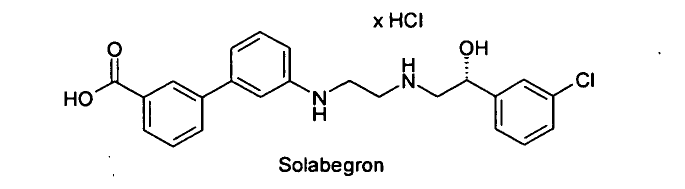

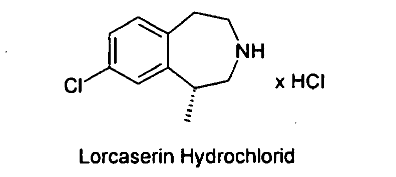

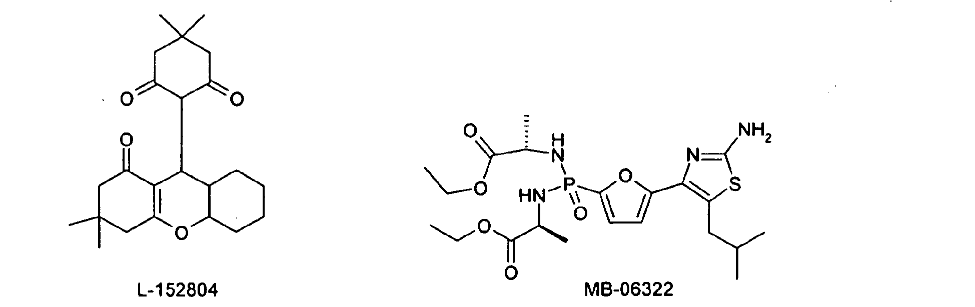

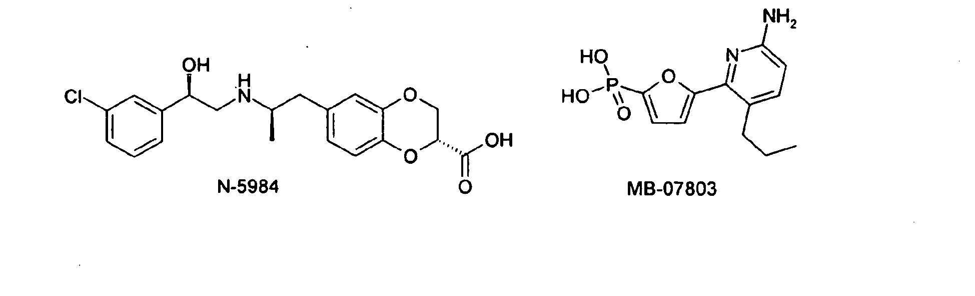

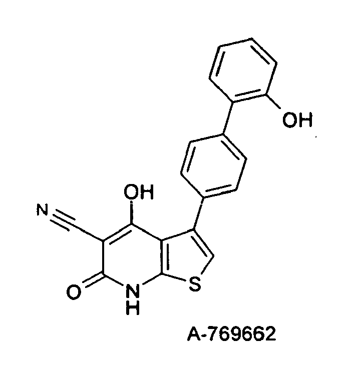

Die nachfolgend aufgeführten Beispiele dienen zur Erläuterung der Erfindung, ohne diese jedoch einzuschränken.

**Tabelle 1:**

| | | | | | | | | | |
|---|---|---|---|---|---|---|---|---|---|
| 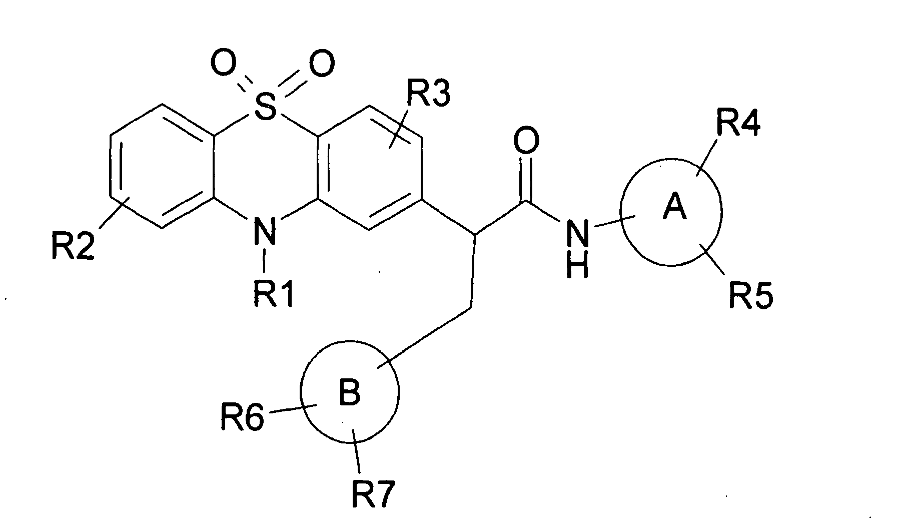 | | | | | | | | | |

| **Beispiel** | **R1** | **R2** | **R3** | **A** | **R4** | **R5** | **B** | **R6** | **R7** |
|---|---|---|---|---|---|---|---|---|---|
| 1 | CH₃ | H | H | 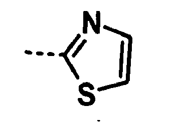 | H | H | 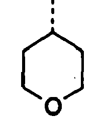 | H | H |
| 2 | CH₃ | H | H | 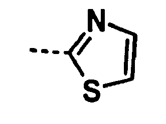 | 5-CH₃ | H | 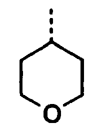 | H | H |
| 3 | CH₃ | H | H | 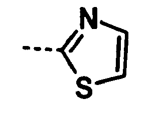 | 5-Cl | H | 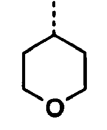 | H | H |
| 4 | CH₃ | H | H | 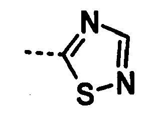 | 3-CH₃ | H | 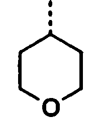 | H | H |
| 5 | CH₃ | H | H | 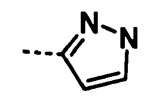 | 1- CH₃ | H | 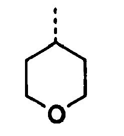 | H | H |
| 6 | CH₃ | H | H | 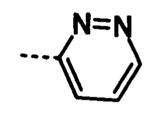 | 6-CH₃ | H | 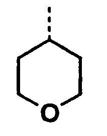 | H | H |
| 7 | CH₃ | H | H | 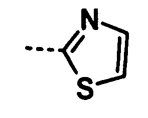 | 4-CO₂Et | H | 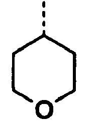 | H | H |
| 8 | CH₃ | H | H | 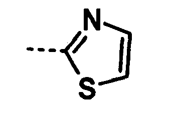 | 4-CH₂-CO₂Et | H | 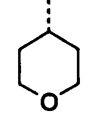 | H | H |
| 9 | CH₃ | H | H | 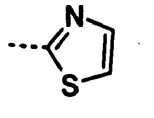 | H | H | 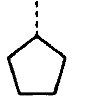 | H | H |
| 10 | CH₃ | H | H | 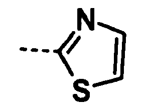 | 4-CH₂-CO₂H | H | 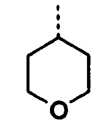 | H | H |
| 11 | CH₃ | H | H | 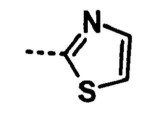 | 4-CO₂H | H | 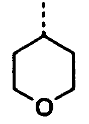 | H | H |
| 12 | CH₃ | H | H | 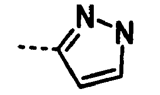 | 1- CH₃ | H | 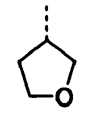 | H | H |
| 13 | CH₃ | H | H | 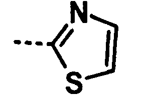 | H | H | 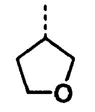 | H | H |
| 14 | CH₃ | H | H | 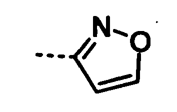 | H | H | 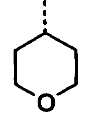 | H | H |
| 15 | CH₃ | H | H | 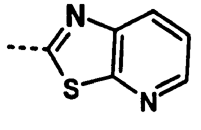 | 5-OCH₃ | H | 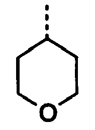 | H | H |
| 16 | CH₃ | H | H | 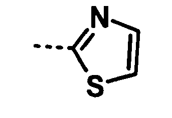 | 4-CH₃ | H | 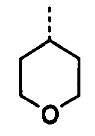 | H | H |
| 17 | CH₃ | H | H | 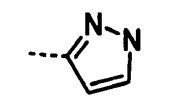 | 1- CH₂CO₂CH₂CH₃ | H | 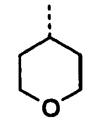 | H | H |
| 18 | CH₃ | H | H | 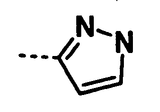 | 1-CH₂CO₂H | H | 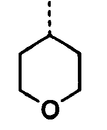 | H | H |
| 19 | CH₃ | H | H | 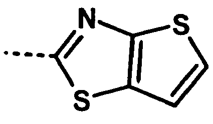 | 6-CO₂CH₃ | H | 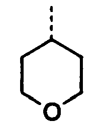 | H | H |
| 20 | CH₃ | H | H | 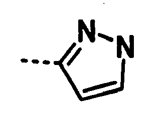 | 1-CH₂Ph | H | 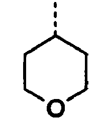 | H | H |
| 21 | CH₃ | H | H | 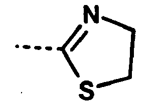 | 4- '=O' | H | 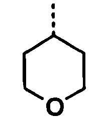 | H | H |
| 22 | CH₃ | H | H | 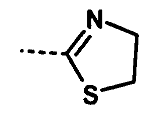 | H | H | 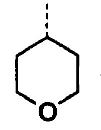 | H | H |
| 23 | CH₃ | H | H | 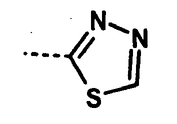 | H | H | 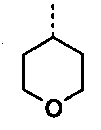 | H | H |
| 24 | CH₃ | H | H | 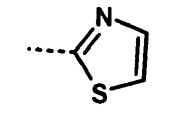 | H | H | 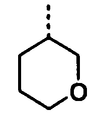 | H | H |
| 25 | CH₃ | H | H | 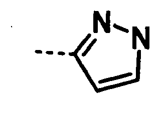 | 1-CH₃ | H | 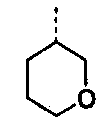 | H | H |
| 26 | CH₃ | H | H | 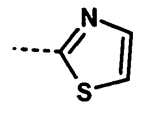 | H | H | 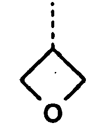 | 3-CH₃ | H |
| 27 | CH₃ | H | H | 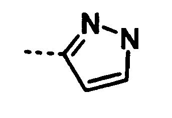 | 1- CH₃ | H | 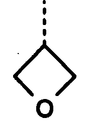 | 3-CH₃ | H |
| 28 | CH₃ | H | H | 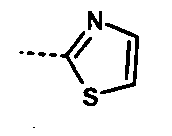 | H | H | 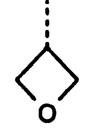 | 3-CH₃CH₂ | H |
| 29 | CH₃ | H | H | 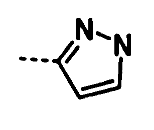 | 1- CH₃ | H | 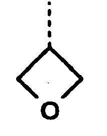 | 3-CH₃CH₂ | H |
| 30 | CH₃ | H | H | 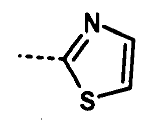 | H | H | 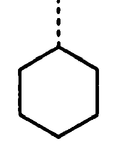 | 4-CH₃ | H |
| 31 | CH₃ | H | H | 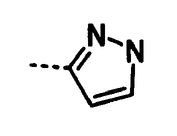 | 1- CH₃ | H | 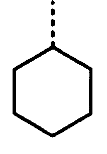 | 4-CH₃ | H |
| 32 | CH₃ | H | H | 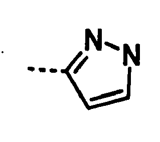 | 1- CH₃ | H | 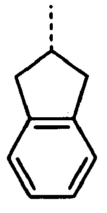 | H | H |
| 33 | CH₃ | H | H | 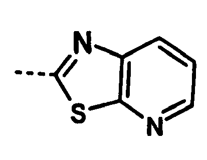 | 5-OCH₃ | H | 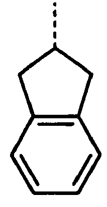 | H | H |
| 34 | CH₃ | H | H | 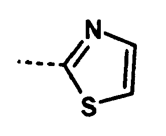 | H | H | 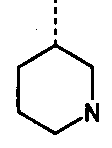 | 1-CH₃ | H |
| 35 | CH₃ | H | H | 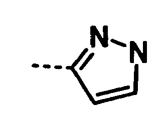 | 1- CH₃ | H | 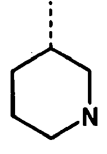 | 1-CH₃ | H |
| 36 | CH₃ | H | H | 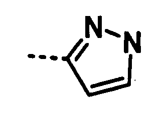 | 1-CH₃ | H | 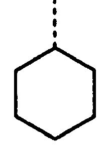 | 4-'=O' | H |
| 37 | CH₃ | H | H | 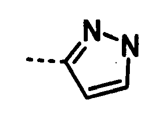 | 1- CH₃ | H | 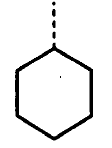 | 4-'-OH' | H |
| 38 | CH₃ | H | H | 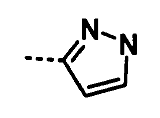 | 1-CH₃ | H | 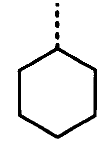 | 4-=NOH' | H |
| 39 | CH₃ | H | H | 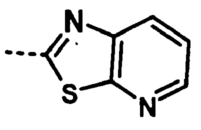 | 5-OCH₃ | H | 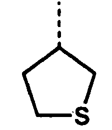 | 1-'=O' | 1-'=O' |
| 40 | CH₃ | H | H | 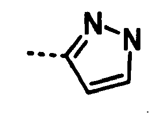 | 1- CH₃ | H | 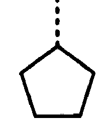 | 3-'=O' | H |
| 41 | CH₃ | H | H | 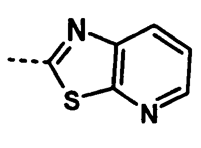 | 5-OCH₃ | H | 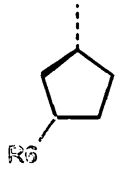 | 3-'=O' | H |
| 42 | CH₃ | 3-CO₂CH₂CH₃ | H | 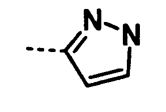 | 1-CH₃ | H | 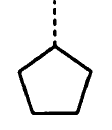 | 3-'=O' | H |
| 43 | CH₃ | 3-CO₂H | H | 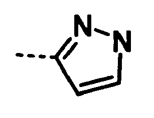 | 1-CH3 | H | 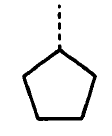 | 3-'=O' | H |
| 44 | H | H | H | 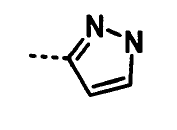 | 1- CH₃ | H | 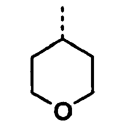 | H | H |
| 45 | H | H | H | 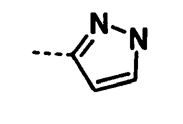 | 1- CH₃ | H | 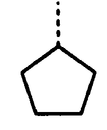 | 3-'=O' | H |
| 46 | H | H | H | 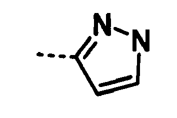 | 1- CH₃ | H | 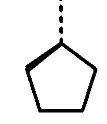 | 3-'=O' | H |
| 47 | H | H | H | 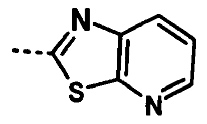 | 5-OCH₃ | H | 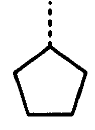 | 3-'=O' | H |
| 48 | H | H | H | 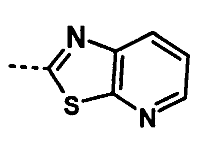 | 5-OCH₃ | H | 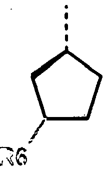 | 3-'=O' | H |
| 49 | H | H | H | 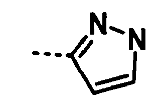 | 1-CH₂Ph | H | 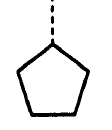 | 3-'=O' | H |
| 50 | H | H | H | 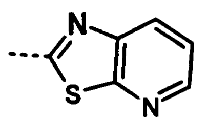 | 5-OCH₃ | H | 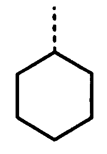 | 4-F | 4-F |
| 51 | H | H | H | 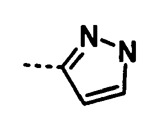 | 1-CH₂Ph | H | 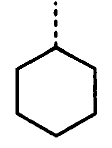 | 4-F | 4-F |
| 52 | H | H | H | 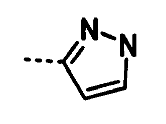 | 1-CH₃ | H | 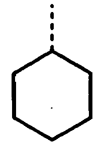 | 4-F | 4-F |
| 53 | H | H | H | 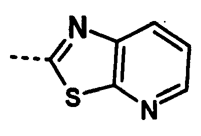 | 5-OCH₃ | H | 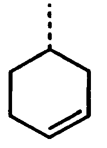 | 4-F | H |
| 54 | H | H | H | 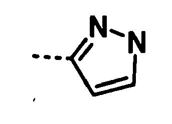 | 1-CH₃ | H | 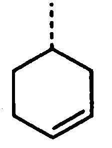 | 4-F | H |
| 55 | H | H | H | 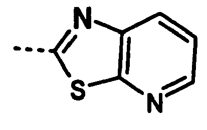 | 5-OCH₃ | H | 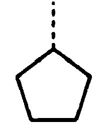 | 3-F | 3-F |
| 56 | H | H | H | 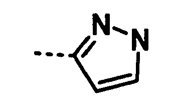 | 1-CH₃ | H | 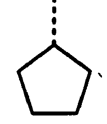 | 3-F | 3-F |

Die gestrichelte Linie in den Resten A und B zeigt den Anknüpfungspunkt der Bindung am Ring.

Die Wirksamkeit der Verbindungen wurde wie folgt getestet:
Enzymatischer Test von Glucokinase-Aktivatoren
Humane Glucokinase

Humane Glucokinase wird als Fusionsprotein mit Glutathion-S-Transferase (GST) in E. coli B121 exprimiert und durch eine Affinitätschromatographie aufgereinigt. Durch Verdauung mit Faktor Xa wird GSH abgespalten und das Glucokinase-Polypeptid beginnend mit Ser-6 erhalten. Letzteres wird chromatographisch aufgereinigt. Eine typische Präparation der Glucokinase besitzt bei Raumtemperatur eine spezifische Aktivität von 30 U/mg Protein.

### Enyzymatischer Test

Die Aktivität von Glucokinase und der Einfluss von Verbindungen auf diese Aktivität werden durch einen gekoppelten optischen Test bei 25 °C bestimmt. Das Testvolumen beträgt 100 µl. Die Testzusammensetzung ist: 25 mM HEPES/NaOH (Merck; #110110) pH 7, 25 mM KCl (Merck; #04933), 2 mM MgCl₂ (Merck; #05833), 1 mM Dithiothreitol (Merck; #112013), 1 mM NAD (Sigma; #N1511), 5 mM Glucose (Merck; #108337), 1 mM ATP (Sigma; #A2383), 0,1 % (w/v) Rinderserumalbumin (Merck; #112018), 0,002 U Glucokinase-Präparation und 3,2 U Glucose-6-Phosphat Dehydrogenase (Sigma; #G8529). Ferner enthält der Ansatz eine Testverbindung. Die Testverbindungen sind jeweils in 10 mM DMSO gelöst und werden bei Endkonzentrationen von 0 µM, 0,1 µM, 0,3 µM, 1 µM, 3 µM, 10 µM, 30 µM und 100 µM getestet. Die Endkonzentration an DMSO im Test beträgt 1 % (v/v). Die Reaktion wird durch die Zugabe von ATP gestartet. Die Absorption des Ansatzes bei 340 nm wird unmittelbar nach der Zugabe von ATP und dann 25 min später mit einem Multiwellplattenphotometer (Firma Labsystems, Multiskan Ascent) bestimmt. Die Änderung der Absorption in diesem Zeitraum wird berechnet.

### Auswertung:

Die Rohdaten der Extinktionsänderungen werden in ein Microsoft Excel-File transferiert. Der Wert für 0 µM Testverbindung wird als 100 % gesetzt. Dosis-Wirkungskurven werden mit dem Programm XL.Fit nach Vorgabe des Herstellers (Firma IDBS) berechnet. Als EC150 wird die Konzentration einer Testverbindung definiert, die eine Steigerung der enzymatischen Aktivität um 50 % hervorruft. Die maximal fache Stimulation entspricht dem Verhältnis der größten Extinktionsänderung im Konzentrationsbereich einer Testverbindung zu der Änderung der Absorption ohne Testsubstanz.

**Tabelle 2: Biologische Aktivität**

| **Beispiel Nr** | **EC₁₅₀ [µM]** | **Fold induction** |
|---|---|---|
| 1 | 4.2 | 2.0 |
| 5 | 2.1 | 4.4 |
| 6 | 15.5 | 2.1 |
| 9 | 2.2 | 2.8 |
| 15 | 0.9 | 5.0 |
| 36 | 0.5 | 4.0 |
| 41 | 0.7 | 3.9 |
| 42 | 1.2 | 3.6 |
| 46 | 0.6 | 5.0 |
| 54 | 1.0 | 4.5 |

Aus den Messdaten der Tabelle ist abzulesen, dass die erfindungsgemäßen Verbindungen eine Aktivierung von Glukokinase bewirken. Diese Verbindungen eignen sich damit insbesondere zur Senkung des Blutzuckerspiegel und zur Behandlung von Diabetes.

### Verfahren

Die erfindungsgemäßen Verbindungen der Formel I können entsprechend dem folgenden Reaktionsschema hergestellt werden:

### Verfahren A:

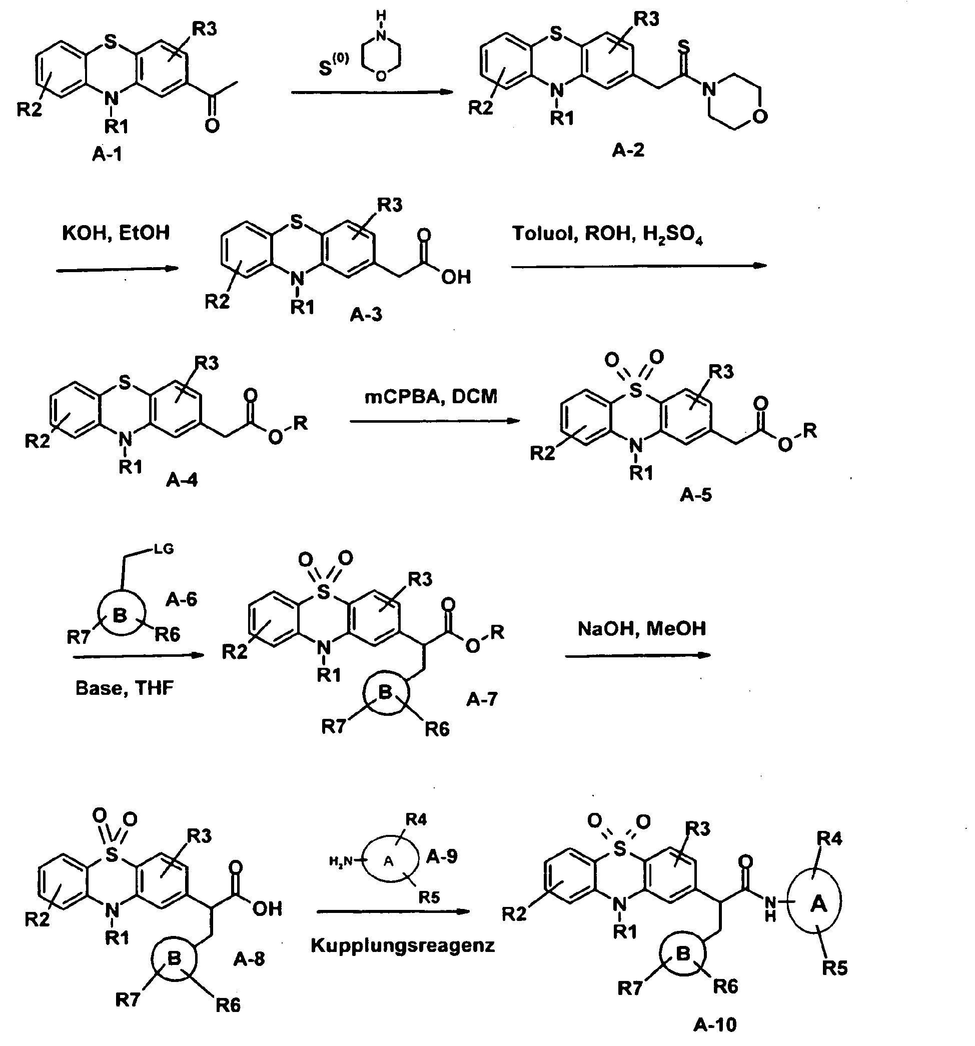

Ein 1-(Phenothiazin-2-yl)-ethanon der allgemeinen Formel A-1, wobei R1, R2 und R3 die oben genannten Bedeutungen haben, wird mit Schwefel und Morpholin bei erhöhter Temperatur (120-180°C) zum Morpholin-4-yl-ethanthion der allgemeinen Formel A-2 umgesetzt. Dieses wird mit einer Base wie zum Beispiel Kaliumhydroxid in einem polaren Lösungsmittel wie zum Beispiel Wasser und Ethanol zur Carbonsäure der allgemeinen Formel A-3 hydrolisiert. Die Carbonsäure wird zum Ester der allgemeinen Formel A-4 umgesetzt, indem die Carbonsäure in einem Alkohol, wie zum Beispiel Ethanol, in Gegenwart eines Säurekatalysators, wie zum Beispiel Schwefelsäure, unter wasserentziehenden Bedingungen, wie zum Beispiel durch Kochen an einem Wasserabscheider in einem Lösungsmittel wie beispielsweise Toluol, azeotrop erhitzt wird. Die Verbindung der allgemeinen Formel A-4 wird mit einem Oxidationsmittel wie zum Beispiel meta-Chlorperbenzoesäure in einem inerten Lösungsmittel wie zum Beispiel Dichlormethan zum Phenothiazin der allgemeinen Formel A-5 umgesetzt. Die Verbindung der allgemeinen Formel A-5 wird mit einer Base wie zum Beispiel Kaliumhexamethyldisilazid in einem polar aprotischen Lösungsmittel wie Tetrahydrofuran, deprotoniert und mit einer Verbindung der allgemeinen Formel A-6 alkyliert, wobei B, R6 und R7 die oben genannten Bedeutungen haben und LG eine Abgangsgruppe wie beispielsweise ein Iodid, Bromid, Mesylat oder Tosylat bedeutet. Die dadurch erhaltene Verbindung der allgemeinen Formel A-7 wird mittels einer Base wie zum Beispiel Natronlauge in einem polar protischen Lösungsmittelgemisch wie Methanol/Wasser zur Carbonsäure der allgemeinen Formel A-8 hydrolysiert. Unter Einwirkung eines Kupplungsreagenzes, wie zum Beispiel O-[Cyan(ethoxycarbonyl)methylenamino]-1,1,3,3-tetramethyluronium-tetrafluoroborat (TOTU) oder [Dimethylamino-([1,2,3]triazolo[4,5-b]pyridin-3-yloxy)-methylene]-dimethyl-ammonium hexafluoro phosphate ( HATU) / [1,2,3]Triazolo[4,5-b]pyridin-3-ol (HOAT) in Gegenwart einer Base, wie zum Beispiel Diisopropylethylamin in einem polar aprotischen Lösungsmittel wie N,N-Diemethylformamid wird die Carbonsäure der allgemeinen Formel A-8 mit dem Amin der allgemeinen Formel A-9, worin A, R4 und R5 die oben beschriebenen Bedeutungen haben, zum Amid der allgemeinen Formel A-10 umgesetzt. Die racemischen Verbindungen der allgemeinen Formel A-10 können durch Chromatographie an chiraler Phase in die Enantiomere getrennt werden.

### Die Beispiele 1-56 wurden nach Verfahren A hergestellt.

Die verwendeten Abkürzungen stehen für:

| | |
|---|---|
| Ac | Acetyl |
| Bn | Benzyl |
| iBu | Isobutyl |
| tBu | tert-Butyl |
| BuLi | n-Butyllithium |
| DC | Dünnschichtchromatographie |
| DCI | direkte chemische Ionisation (bei MS) |
| DCM | Dichlormethan |
| DMAP | 4-N,N-Dimethylaminopyridin |
| DMF | N,N-Dimethylformamid |
| DMSO | Dimethylsulfoxid |
| dppp | 1,3-Bis(diphenylphosphino)propan |
| EE | Ethylacetat |
| ent | Enantiomer / enantiomerenrein |
| EI | Elektronenstoß-Ionisation (bei MS) |
| eq | Äquivalent |
| ESI | Elektronenspray-Ionisation (bei MS) |
| Et | Ethyl |
| GC | Gaschromatographie |
| HATU | [Dimethylamino-([1,2,3]triazolo[4,5-b]pyridin-3-yloxy)-methylene]- |
| | dimethyl-ammonium hexafluoro phosphate |
| HOAT | [1,2,3]Triazolo[4,5-b]pyridin-3-ol |
| HPLC | Hochdruck-, Hochleistungsflüssigchromatographie |
| LiHMDS | Lithiumhexamethyldisilazid |
| LC-MS | Flüssigchromatographie-gekoppelte Massenspektroskopie |
| m | meta |
| M | molar |
| mCPBA | meta-Chlorperbenzoesäure |
| Me | Methyl |
| MeCN | Acetonitril |
| MS | Massenspektroskopie |
| NMR | Kernresonanzspektroskopie |
| o | ortho |
| p | para |
| Pd/C | Palladium auf Kohle |
| Ph | Phenyl |
| iPr | Isopropyl |
| nPr | n-Propyl |
| rac | racemisch / racemisches Gemisch |
| Rf | Retentionszeit (bei DC) |
| RP | Reversed Phase |
| tert | tertiär |
| | |
| THF | Tetrahydrofuran |
| TOTU | O-[Cyan(ethoxycarbonyl)methylenamino]-1,1,3,3-tetramethyluronium-tetrafluoroborat |

### Beispielsynthesen nach Verfahren A

### Beispiel 1

### 2-(10-Methyl-5,5-dioxo-5,10-dihydro-phenothiazin-2-yl)-3-(tetrahydro-pyran-4-yl)-N-thiazol-2-yl-propionamid

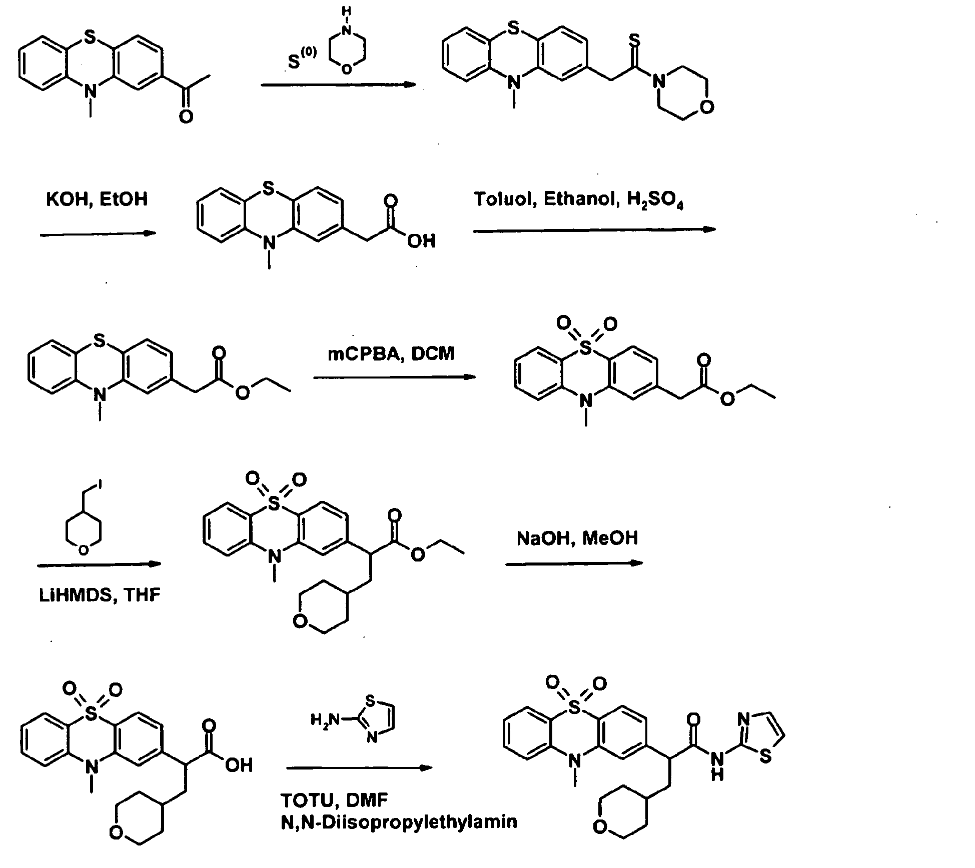

### 2-(10-Methyl-10H-phenothiazin-2-yl)-1-morpholin-4-yl-ethanthion

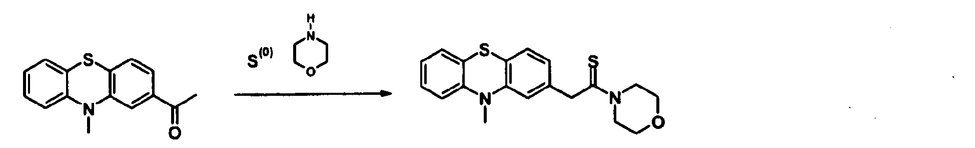

22.7 g 1-(10-Methyl-10H-phenothiazin-2-yl)-ethanon werden mit 7.08 g Schwefel und 15.57 ml Morpholin versetzt. Das Reaktionsgemisch wird zweieinhalb Stunden bei 150°C gerührt. Das Reaktionsgemisch wird in einem Eisbad abgekühlt und mit Ethylacetat und Ethanol verrührt. Der ausgefallene Niederschlag wird abgesaugt und das Filtrat im Vakuum eingeengt bis sich erneut ein Niederschlag abscheidet. Dieser wird ebenfalls abgesaugt. Die Niederschläge werden vereinigt und im Vakuum getrocknet. Man erhält 30.2 g 2-(10-Methyl-10H-phenothiazin-2-yl)-1-morpholin-4-yl-ethanthion als orangefarbenen Feststoff. C19H20N2OS2 (356.51), LCMS(ESI): 357.1 (M+H⁺), Rf(n-Heptan:Ethylacetat = 3:1) = 0.12.

### (10-Methyl-10H-phenothiazin-2-yl)-essigsäure

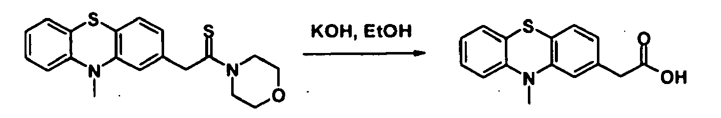

30.2 g 2-(10-Methyl-10H-phenothiazin-2-yl)-1-morpholin-4-yl-ethanthion werden in einer Lösung aus 50 ml 50%iger wässriger Kalilauge und 100 ml Ethanol zwölf Stunden unter Rückfluss zum Sieden erhitzt. Das Reaktionsgemisch wird im Eisbad abgekühlt und mit konzentrierter Salzsäure auf pH 3 angesäuert. Anschließend wird das Produkt dreimal mit je 250 ml Ethylacetat extrahiert. Die vereinigten organischen Phasen werden über MgSO₄ getrocknet und anschließend im Vakuum eingeengt. Man erhält 18.0 g (10-Methyl-10H-phenothiazin-2-yl)-essigsäure als gelbes Öl.
C15H13NO2S (271.34), LCMS(ESI): 271.95 (M+H⁺), Rf(n-Heptan:Ethylacetat = 1:1) = 0.26.

### (10-Methyl-10H-phenothiazin-2-yl)-essigsäureethylester

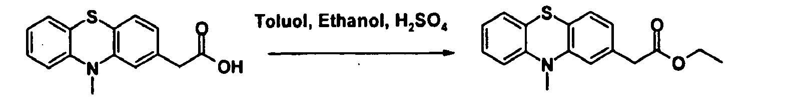

18.0 g (10-Methyl-10H-phenothiazin-2-yl)-essigsäure werden in einer Mischung aus 400 ml Toluol und 200 ml Ethanol gelöst und mit 5 ml konzentrierter Schwefelsäure versetzt. Das Reaktionsgemisch wird an einem mit Molsieb gefüllten Wasserabscheider zwölf Stunden unter Rückfluss zum Sieden erhitzt. Danach wird das Reaktionsgemisch im Eisbad abgekühlt und mit 200 ml Wasser versetzt. Die Mischung wird durch Zugabe von festem Natriumkarbonat neutralisiert. Anschließend wird das Produkt fünfmal mit je 250 ml Ethylacetat extrahiert. Die vereinigten organischen Phasen werden über MgSO₄ getrocknet und anschließend im Vakuum eingeengt. Der Rückstand wird an Kieselgel mit dem Eluens n-Heptan => n-Heptan : Ethylacetat = 10:1 1 gereinigt. Man erhält 17.0 g (10-Methyl-10H-phenothiazin-2-yl)-essigsäureethylester als gelbes Öl.
C17H17NO2S (299.39), LCMS(ESI): 300.1 (M+H⁺), Rf(n-Heptan:Ethylacetat = 3:1) = 0.34.

### (10-Methyl-5,5-dioxo-5,10-dihydro-phenothiazin-2-yl)-essigsäureethylester

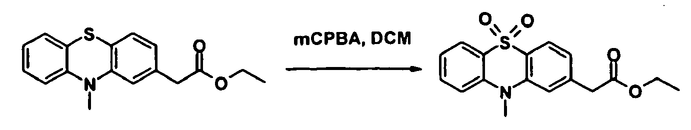

17.0 g (10-Methyl-10H-phenothiazin-2-yl)-essigsäureethylester werden in 300 ml Dichlormethan gelöst und portionsweise mit 35.0 g mCPBA versetzt. Das Reaktionsgemisch wird eine Stunde bei Raumtemperatur gerührt. Danach wird das Reaktionsgemisch mit 100 ml gesättigter NaHCO₃-Lösung, 5x mit je 100 ml 2 M NaOH und einmal mit 100 ml gesättigter NaCl-Lösung gewaschen, über MgSO₄ getrocknet und anschließend das Lösungsmittel im Vakuum entfernt. Man erhält 6.7 g (10-Methyl-5,5-dioxo-5,10-dihydrophenothiazin-2-yl)-essigsäureethylester als gelbes Öl, welches beim Stehenlassen langsam durchkristallisiert.
C17H17NO4S (331.39), LCMS(ESI): 332.1 (M+H⁺), Rf(n-Heptan:EE= 1:1) = 0.49.

### 2-(10-Methyl-5,5-dioxo-5,10-dihydro-phenothiazin-2-yl)-3-(tetrahydro-pyran-4-yl)-propionsäureethylester

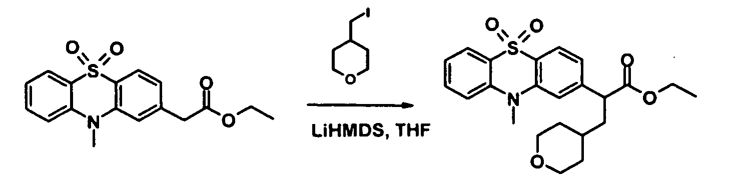

1.66 ml 1,1,1,3,3,3-Hexamethyldisilazan werden in 20 ml Tetrahydrofuran unter Argon gelöst. Unter Eiskühlung werden 2.90 ml n-Buthyllithium (2.5 M in n-Hexan) zugetropft und 30 Minuten bei 0°C nachgerührt. Anschließend wird diese Lösung zu einer gerührten Lösung von 2.0 g (10-Methyl-5,5-dioxo-5,10-dihydro-phenothiazin-2-yl)-essigsäureethylester in 100 ml Tetrahydrofuran bei -78°C zugetropft. Das Reaktionsgemisch wird 20 Minuten bei -78°C gerührt, dann werden 2.0 g 4-(Iodomethyl)tetrahydro-2H-pyran zugetropft. Das Kühlbad wird entfernt und man lässt langsam auf Raumtemperatur erwärmen. Der Reaktionsansatz wird über Nacht bei Raumtemperatur gerührt. Dann werden 10 ml Wasser zugegeben, das Tetrahydrofuran im Vakuum entfernt und der Rückstand dreimal mit je 100 ml Ethylacetat extrahiert. Die vereinigten organischen Phasen werden über MgSO₄ getrocknet und anschließend im Vakuum eingeengt. Der Rückstand wird an Kieselgel mit dem Eluens n-Heptan:Ethylacetat (100%:0%)=> n-Heptan:Ethylacetat (0%:100%) gereinigt. Man erhält 2.0 g 2-(10-Methyl-5,5-dioxo-5,10-dihydro-phenothiazin-2-yl)-3-(tetrahydro-pyran-4-yl)-propionsäureethylester als farblosen Feststoff.
C23H27NO5S (429.54), LCMS(ESI): 430.2 (M+H⁺).

### 2-(10-Methyl-5,5-dioxo-5,10-dihydro-phenothiazin-2-yl)-3-(tetrahydro-pyran-4-yl)-propionsäure

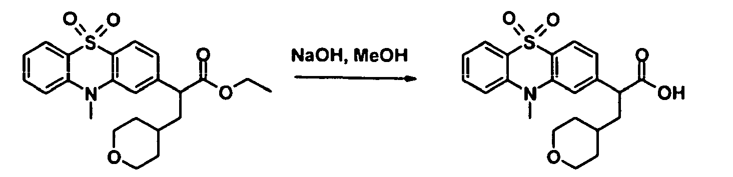

2.0 g 2-(10-Methyl-5,5-dioxo-5,10-dihydro-phenothiazin-2-yl)-3-(tetrahydro-pyran-4-yl)-propionsäureethylester werden in 100 ml Methanol suspendiert und mit 32.1 ml 2M NaOH Lösung versetzt. Das Reaktionsgemisch wird eine Stunde bei 80°C gerührt. Das Methanol wird im Vakuum entfernt und das Reaktionsgemisch durch Zugabe konzentrierter Salzsäure auf pH 4 eingestellt. Man extrahiert dreimal mit je 100 ml Ethylacetat. Die vereinigten organischen Phasen werden über MgSO₄ getrocknet und anschließend im Vakuum eingeengt. Man erhält 1.85 g 2-(10-Methyl-5,5-dioxo-5,10-dihydro-phenothiazin-2-yl)-3-(tetrahydro-pyran-4-yl)-propionsäure als Feststoff.
C21H23NO5S (401.49), LCMS(ESI): 402.2 (M+H⁺).

### 2-(10-Methyl-5,5-dioxo-5,10-dihydro-phenothiazin-2-yl)-3-(tetrahydro-pyran-4-yl)-N-thiazol-2-yl-propionamid

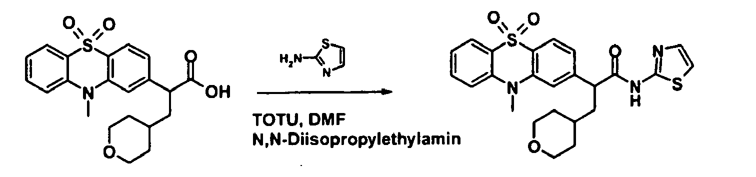

200 mg 2-(10-Methyl-5,5-dioxo-5,10-dihydro-phenothiazin-2-yl)-3-(tetrahydro-pyran-4-yl)-propionsäure, 74.8 mg kommerziell erhältliches 2-Amino-thiazol und 220 µl N,N-Diisopropylethylamin werden in 10 ml Dimethylformamid gelöst. Man gibt 212 mg TOTU hinzu und rührt bei Raumtemperatur über Nacht. Danach wird das Reaktionsgemisch durch Zugabe von 50 ml Ethylacetat verdünnt und fünfmal mit je 30 ml gesättigter Natriumhydrogenkarbonatlösung gewaschen. Die organische Phase wird über MgSO₄ getrocknet und anschließend das Lösungsmittel im Vakuum entfernt. Der Rückstand wird mittels RP-HPLC gereinigt. Man erhält 141 mg 2-(10-Methyl-5,5-dioxo-5,10-dihydrophenothiazin-2-yl)-3-(tetrahydro-pyran-4-yl)-N-thiazol-2-yl-propionamid als farbloses Lyophilisat.
C24H25N3O4S2 (483.61), LCMS(ESI): 484.1(M+H⁺).

### Beispiel 2

### 2-(10-Methyl-5,5-dioxo-5,10-dihydro-phenothiazin-2-yl)-N-(5-methyl-thiazol-2-yl)-3-(tetrahydro-pyran-4-yl)-propionamid

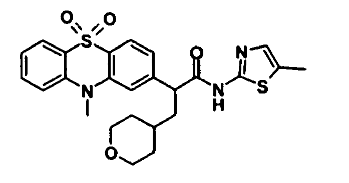

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-Amino-5-methylthiazol 2-(10-Methyl-5,5-dioxo-5,10-dihydro-phenothiazin-2-yl)-N-(5-methyl-thiazol-2-yl)-3-(tetrahydro-pyran-4-yl)-propionamid.
C25H27N3O4S2 (497.64), LCMS(ESI): 498.1 (M+H⁺).

### Beispiel 3

### N-(5-Chloro-thiazol-2-yl)-2-(10-methyl-5,5-dioxo-5,10-dihydro-phenothiazin-2-yl)-3-(tetrahydro-pyran-4-yl)-propionamid

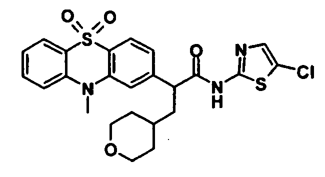

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-Amino-5-chlorothiazol Hydrochlorid N-(5-Chloro-thiazol-2-yl)-2-(10-methyl-5,5-dioxo-5,10-dihydrophenothiazin-2-yl)-3-(tetrahydro-pyran-4-yl)-propionamid.
C24H24ClN3O4S2 (518.06), LCMS(ESI): 518.1 (M+H⁺).

### Beispiel 4

### 2-(10-Methyl-5,5-dioxo-5,10-dihydro-phenothiazin-2-yl)-N-(3-methyl-[1,2,4]thiadiazol-5-yl)-3-(tetrahydro-pyran-4-yl)-propionamid

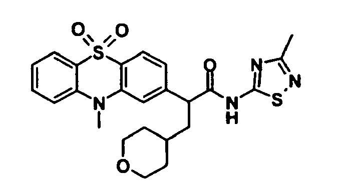

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 3-Methyl-[1,2,4]thiadiazol-5-ylamin 2-(10-Methyl-5,5-dioxo-5,10-dihydro-phenothiazin-2-yl)-N-(3-methyl-[1,2,4]thiadiazol-5-yl)-3-(tetrahydro-pyran-4-yl)-propionamid.
C24H26N4O4S2 (498.63), LCMS(ESI): 499.2 (M+H⁺).

### Beispiel 5

### 2-(10-Methyl-5,5-dioxo-5,10-dihydro-phenothiazin-2-yl)-N-(1-methyl-1H-pyrazol-3-yl)-3-(tetrahydro-pyran-4-yl)-propionamid

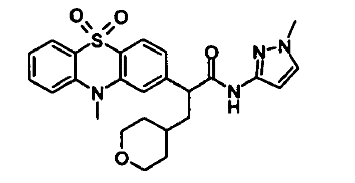

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 1-Methyl-1H-pyrazol-3-amin 2-(10-Methyl-5,5-dioxo-5,10-dihydro-phenothiazin-2-yl)-N-(1-methyl-1H-pyrazol-3-yl)-3-(tetrahydro-pyran-4-yl)-propionamid.
C25H28N4O4S (480.59), LCMS(ESI): 481.2(M+H⁺).

### Beispiel 6

### 2-(10-Methyl-5,5-dioxo-5,10-dihydro-phenothiazin-2-yl)-N-(6-methyl-pyridazin-3-yl)-3-(tetrahydro-pyran-4-yl)-propionamid

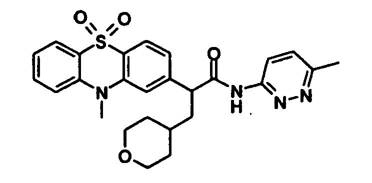

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 6-Methyl-3-pyridazinamin 2-(10-Methyl-5,5-dioxo-5,10-dihydro-phenothiazin-2-yl)-N-(6-methylpyridazin-3-yl)-3-(tetrahydro-pyran-4-yl)-propionamid.
C26H28N4O4S (492.60), LCMS(ESI): 493.2(M+H⁺).

### Beispiel 7

### 2-[2-(10-Methyl-5,5-dioxo-5,10-dihydro-phenothiazin-2-yl)-3-(tetrahydro-pyran-4-yl)-propionylamino]-thiazol-4-carbonsäureethylester

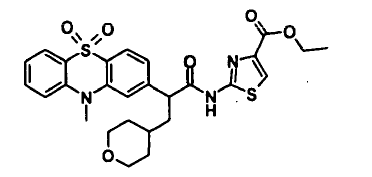

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-Aminothiazol-4-carbonsäureethylester 2-[2-(10-Methyl-5,5-dioxo-5,10-dihydro-phenothiazin-2-yl)-3-(tetrahydro-pyran-4-yl)-propionylamino]-thiazol-4-carbonsäureethylester. C27H29N3O6S2 (555.68), LCMS(ESI): 597.20(M+MeCN+H⁺).

### Beispiel 8

### {2-[2-(10-Methyl-5,5-dioxo-5,10-dihydro-phenothiazin-2-yl)-3-(tetrahydro-pyran-4-yl)-propionylamino]-thiazol-4-yl}-essigsäureethylester

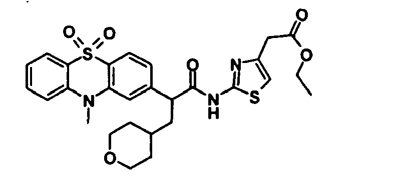

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-Aminothiazol-4-essigsäureethylester {2-[2-(10-Methyl-5,5-dioxo-5,10-dihydro-phenothiazin-2-yl)-3-(tetrahydro-pyran-4-yl)-propionylamino]-thiazol-4-yl -essigsäureethylester. C28H31N3O6S2 (569.70), LCMS(ESI): 570.20(M+H⁺).

### Beispiel 9

### 3-Cyclopentyl-2-(10-methyl-5,5-dioxo-5,10-dihydro-phenothiazin-2-yl)-N-thiazol-2-yl-propionamid

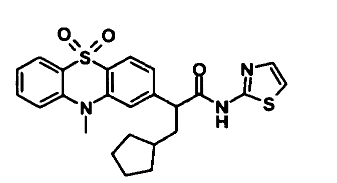

Analog zu Beispiel 1 erhält man aus (10-Methyl-5,5-dioxo-5,10-dihydro-phenothiazin-2-yl)-essigsäureethylester, kommerziell erhältlichem Iodmethyl-cyclopentan und, kommerziell erhältlichem 2-Amino-thiazol 3-Cyclopentyl-2-(10-methyl-5,5-dioxo-5,10-dihydrophenothiazin-2-yl)-N-thiazol-2-yl-propionamid.
C24H25N3O3S2 (467.61), LCMS(ESI): 468.2 (M+H⁺), 509.3 (M+MeCN+H⁺).
Das racemische Gemisch wird an chiraler Phase (Chiralpak AS-H 43) mit dem Eluens n-Heptan:Methanol:Ethanol = 10:1:1 (Säule vorkonditioniert mit Trifluoressigsäure) in die Enantiomere (Rt = 13.66 min und Rt= 16.58 min) getrennt.

### Beispiel 10

### {2-[2-(10-Methyl-5,5-dioxo-5,10-dihydro-phenothiazin-2-yl)-3-(tetrahydro-pyran-4-yl)-propionylamino]-thiazol-4-yl}-essigsäure

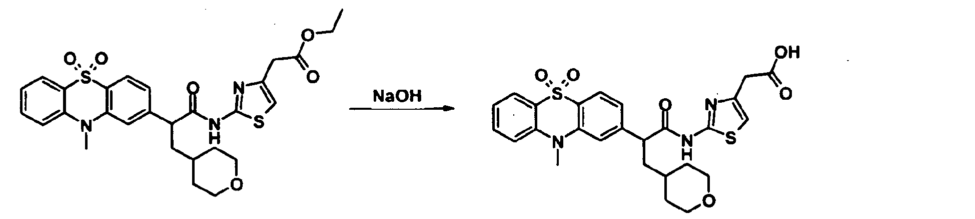

Zu einer Suspension aus 25.0 mg {2-[2-(10-Methyl-5,5-dioxo-5,10-dihydro-phenothiazin-2-yl)-3-(tetrahydropyran-4-yl)-propionylamino]-thiazol-4-yl}-essigsäureethylester in 5 ml Tetrahydrofuran und 2 ml Wasser wird bei Raumtemperatur unter Rühren 0.25 ml 2M NaOH gegeben und 12 Stunden bei Raumtemperatur gerührt. Dann wird das Reaktionsgemisch durch Zugabe von 1M HCl neutralisiert. Die Lösungsmittel werden im Vakuum entfernt, der Rückstand in 20 ml Ethylacetat aufgenommen und dreimal mit je 10 ml Wasser gewaschen. Die organische Phase wird mit MgSO₄ getrocknet und im Vakuum eingeengt. Der Rückstand wird mittels RP-HPLC gereinigt. Man erhält 22.5 mg {2-[2-(10-Methyl-5,5-dioxo-5,10-dihydro-phenothiazin-2-yl)-3-(tetrahydropyran-4-yl)-propionylamino]-thiazol-4-yl}-essigsäure als Lyophilisat.
C26H27N3O6S2 (541.65), LCMS(ESI): 542.2 (M+H⁺).

### Beispiel 11

### 2-[2-(10-Methyl-5,5-dioxo-5,10-dihydro-phenothiazin-2-yl)-3-(tetrahydro-pyran-4-yl)-propionylamino]-thiazol-4-carbonsäure

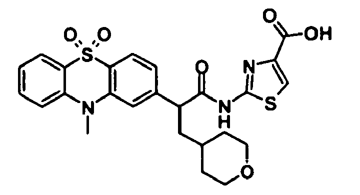

Analog zu Beispiel 10 erhält man aus 2-[2-(10-Methyl-5,5-dioxo-5,10-dihydro-phenothiazin-2-yl)-3-(tetrahydropyran-4-yl)-propionylamino]-thiazol-4-carbonsäureethylester 2-[2-(10-Methyl-5,5-dioxo-5,10-dihydro-phenothiazin-2-yl)-3-(tetrahydropyran-4-yl)-propionylamino]-thiazol-4-carbonsäure.
C25H25N3O6S2 (527.62), LCMS(ESI): 528.2 (M+H⁺), 569.2(M+MeCN+H⁺).

### Beispiel 12

### 2-(10-Methyl-5,5-dioxo-5,10-dihydro-phenothiazin-2-yl)-N-(1-methyl-1H-pyrazol-3-yl)-3-(tetrahydro-furan-3-yl)-propionamid

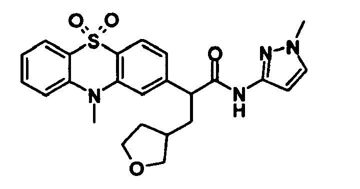

Analog zu Beispiel 1 erhält man aus (10-Methyl-5,5-dioxo-5,10-dihydro-phenothiazin-2-yl)-essigsäureethylester, kommerziell erhältlichem 3-Iodmethyl-tetrahydrofuran und kommerziell erhältlichem 1-Methyl-1H-pyrazol-3-amin 2-(10-Methyl-5,5-dioxo-5,10-dihydrophenothiazin-2-yl)-N-(1-methyl-1H-py razol-3-yl)-3-(tetrahydro-furan-3-yl)-propionamid. C24H26N4O4S (466.56), LCMS(ESI): 467.2 (M+H⁺).

### Beispiel 13

### 2-(10-Methyl-5,5-dioxo-5,10-dihydro-phenothiazin-2-yl)-3-(tetrahydro-furan-3-yl)-N-thiazol-2-yl-propionamid

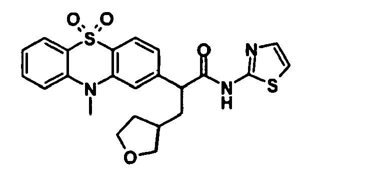

Analog zu Beispiel 1 erhält man aus (10-Methyl-5,5-dioxo-5,10-dihydro-phenothiazin-2-yl)-essigsäureethylester, kommerziell erhältlichem 3-Iodmethyl-tetrahydrofuran und kommerziell erhältlichem 2-Amino-thiazol 2-(10-Methyl-5,5-dioxo-5,10-dihydro-phenothiazin-2-yl)-3-(tetrahydro-furan-3-yl)-N-thiazol-2-yl-propionamid.
C23H23N3O4S2 (469.59), LCMS(ESI): 470.1 (M+H⁺).

### Beispiel 14

### N-Isoxazol-3-yl-2-(10-methyl-5,5-dioxo-5,10-dihydro-phenothiazin-2-yl)-3-(tetrahydro-pyran-4-yl)-propionamid

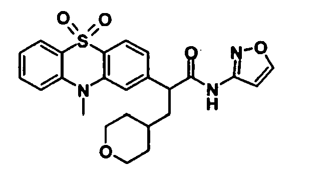

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 3-Aminoisoxazol N-Isoxazol-3-yl-2-(10-methyl-5,5-dioxo-5,10-dihydro-phenothiazin-2-yl)-3-(tetrahydro-pyran-4-yl)-propionamid.
C24H25N3O5S (467.55), LCMS(ESI): 468.3 (M+H⁺).

### Beispiel 15

### N-(5-Methoxy-thiazolo[5,4-b]pyridin-2-yl)-2-(10-methyl-5,5-dioxo-5,10-dihydrophenothiazin-2-yl)-3-(tetrahydro-pyran-4-yl)-propionamide

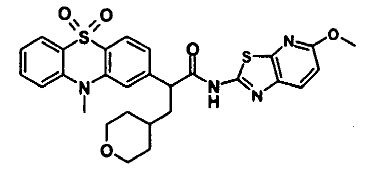

Analog zu Beispiel erhält man aus 2-(10-Methyl-5,5-dioxo-5,10-dihydro-phenothiazin-2-yl)-3-(tetrahydro-pyran-4-yl)-propionsäure und 5-Methoxy-thiazolo[5,4-b]pyridin-2-ylamin N-(5-Methoxy-thiazolo[5,4-b]pyridin-2-yl)-2-(10-methyl-5,5-dioxo-5,10-dihydrophenothiazin-2-yl)-3-(tetrahydro-pyran-4-yl)-propionamid.
C28H28N4O5S2 (564.69), LCMS(ESI): 565.2 (M+H⁺).

### Beispiel 16

### 2-(10-Methyl-5,5-dioxo-5,10-dihydro-phenothiazin-2-yl)-N-(4-methyl-thiazol-2-yl)-3-(tetrahydro-pyran-4-yl)-propionamid

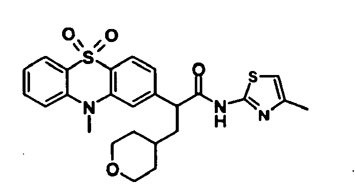

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-Amino-4-methylthiazolhydrochlorid 2-(10-Methyl-5,5-dioxo-5,10-dihydro-phenothiazin-2-yl)-N-(4-methyl-thiazol-2-yl)-3-(tetrahydro-pyran-4-yl)-propionamid.
C25H27N3O4S2 (497.64), LCMS(ESI): 498.1 (M+H⁺).

### Beispiel 17

### {3-[2-(10-Methyl-5,5-dioxo-5,10-dihydro-phenothiazin-2-yl)-3-(tetrahydro-pyran-4-yl)-propionylamino]-pyrazol-1-yl}-essigsäureethylester

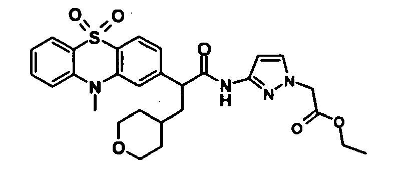

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem (3-Amino-pyrazol-1-yl)-essigsäureethylesterhydrochlorid {3-[2-(10-Methyl-5,5-dioxo-5,10-dihydrophenothiazin-2-yl)-3-(tetrahydro-pyran-4-yl)-propionylamino]-pyrazol-1-yl }-essigsäureethylester.
C28H32N4O6S (552.65), LCMS(ESI): 553.3 (M+H⁺).

### Beispiel 18

### {3-[2-(10-Methyl-5,5-dioxo-5,10-dihydro-phenothiazin-2-yl)-3-(tetrahydro-pyran-4-yl)-propionylamino]-pyrazol-1-yl-essigsäure

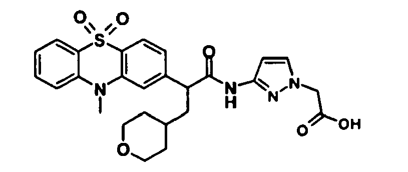

Analog zu Beispiel 10 erhält man aus {3-[2-(10-Methyl-5,5-dioxo-5,10-dihydrophenothiazin-2-yl)-3-(tetrahydro-pyran-4-yl)-propionylamino]-pyrazol-1-yl}-essigsäureethylester {3-[2-(10-Methyl-5,5-dioxo-5,10-dihydro-phenothiazin-2-yl)-3-(tetrahydro-pyran-4-yl)-propionylamino]-pyrazol-1-yl}-essigsäure.
C26H28N4O6S (524.60), LCMS(ESI): 525.2 (M+H⁺).

### Beispiel 19

### 2-[2-(10-Methyl-5,5-dioxo-5,10-dihydro-phenothiazin-2-yl)-3-(tetrahydro-pyran-4-yl)-propionylamino]-thieno[2,3-d]thiazol-6-carbonsäuremethylester

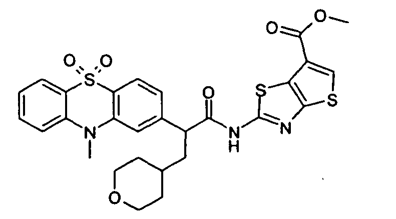

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-aminothieno[2,3-d]thiazol-6-carbonsäuremethylester 2-[2-(10-Methyl-5,5-dioxo-5,10-dihydrophenothiazin-2-yl)-3-(tetrahydro-pyran-4-yl)-propionylamino]-thieno[2,3-d]thiazol-6-carbonsäuremethylester.
C28H27N3O6S3 (597.74), LCMS(ESI): 598.3 (M+H⁺).

### Beispiel 20

### N-(1-Benzyl-1H-pyrazol-3-yl)-2-(10-methyl-5,5-dioxo-5,10-dihydro-phenothiazin-2-yl)-3-(tetrahydro-pyran-4-yl)-propionamid

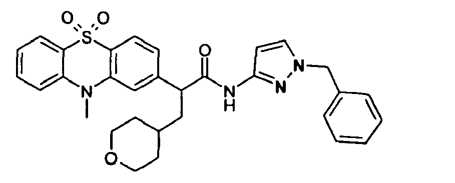

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 1-Benzyl-1H-pyrazol-3-ylamin N-(1-Benzyl-1H-pyrazol-3-yl)-2-(10-methyl-5,5-dioxo-5,10-dihydrophenothiazin-2-yl)-3-(tetrahydro-pyran-4-yl)-propionamid.
C31H32N4O4S (556.69), LCMS(ESI): 557.4 (M+H⁺).

### Beispiel 21

### 2-(10-Methyl-5,5-dioxo-5,10-dihydro-phenothiazin-2-yl)-N-(4-oxo-4,5-dihydro-thiazol-2-yl)-3-(tetrahydro-pyran-4-yl)-propionamid

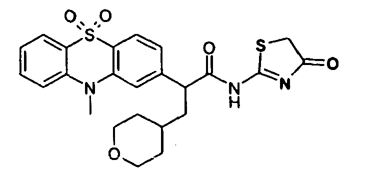

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem Pseudothiohydantoin 2-(10-Methyl-5,5-dioxo-5,10-dihydro-phenothiazin-2-yl)-N-(4-oxo-4,5-dihydro-thiazol-2-yl)-3-(tetrahydro-pyran-4-yl)-propionamid.
C24H25N3O5 (499.61), LCMS(ESI): 500.2 (M+H⁺), 541.2 (M+MeCN+H⁺).

### Beispiel 22

### N-(4,5-Dihydro-thiazol-2-yl)-2-(10-methyl-5,5-dioxo-5,10-dihydro-phenothiazin-2-yl)-3-(tetrahydro-pyran-4-yl)-propionamid

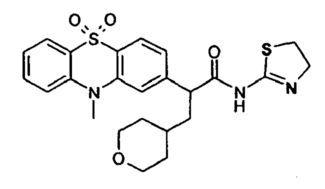

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-Amino-2-thiazolin N-(4,5-Dihydro-thiazol-2-yl)-2-(10-methyl-5,5-dioxo-5,10-dihydro-phenothiazin-2-yl)-3-(tetrahydro-pyran-4-yl)-propionamid.
C24H27N3O4S2 (485.62), LCMS(ESI): 486.2 (M+H⁺).

### Beispiel 23

### 2-(10-Methyl-5,5-dioxo-5,10-dihydro-phenothiazin-2-yl)-3-(tetrahydro-pyran-4-yl)-N-[1,3,4]thiadiazol-2-yl-propionamid

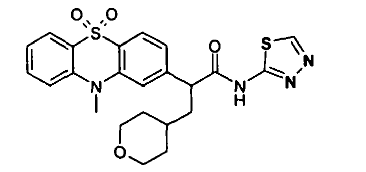

Analog zu Beispiel 1 erhält man aus 2-(10-Methyl-5,5-dioxo-5,10-dihydro-phenothiazin-2-yl)-3-(tetrahydro-pyran-4-yl)-propionsäure und kommerziell erhältlichem 2-Amino-1,3,4-thiadiazol 2-(10-Methyl-5,5-dioxo-5,10-dihydro-phenothiazin-2-yl)-3-(tetrahydro-pyran-4-yl)-N-[1,3,4]thiadiazol-2-yl-propionamid.
C23H24N4O4 (484.60), LCMS(ESI): 485.2 (M+H⁺), 526.2 (M+MeCN+H⁺).

### Beispiel 24

### 2-(10-Methyl-5,5-dioxo-5,10-dihydro-phenothiazin-2-yl)-3-(tetrahydro-pyran-3-yl)-N-thiazol-2-yl-propionamid

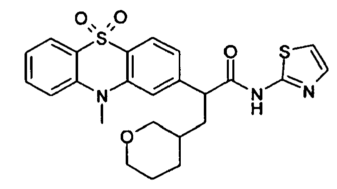

Analog zu Beispiel 1 erhält man aus (10-Methyl-5,5-dioxo-5,10-dihydro-phenothiazin-2-yl)-essigsäureethylester, 3-Iodomethyl-tetrahydro-pyran und kommerziell erhältlichem 2-Amino-thiazol 2-(10-Methyl-5,5-dioxo-5,10-dihydro-phenothiazin-2-yl)-3-(tetrahydro-pyran-3-yl)-N-thiazol-2-yl-propionamid.
C24H25N3O4S2 (483.61), LCMS(ESI): 484.1 (M+H⁺).

### Beispiel 25

### 2-(10-Methyl-5,5-dioxo-5,10-dihydro-phenothiazin-2-yl)-N-(1-methyl-1H-pyrazol-3-yl)-3-(tetrahydro-pyran-3-yl)-propionamid

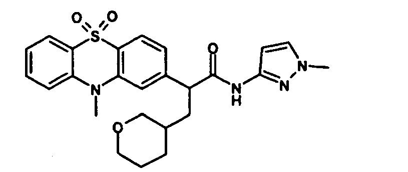

Analog zu Beispiel 1 erhält man aus (10-Methyl-5,5-dioxo-5,10-dihydro-phenothiazin-2-yl)-essigsäureethylester, 3-Iodomethyl-tetrahydro-pyran und kommerziell erhältlichem 1-Methyl-1H-pyrazol-3-amin 2-(10-Methyl-5,5-dioxo-5,10-dihydro-phenothiazin-2-yl)-N-(1-methyl-1H-pyrazol-3-yl)-3-(tetrahydro-pyran-3-yl)-propionamid.
C25H28N4O4S (480.59), LCMS(ESI): 481.2 (M+H⁺).

### Beispiel 26

### 2-(10-Methyl-5,5-dioxo-5,10-dihydro-phenothiazin-2-yl)-3-(3-methyl-oxetan-3-yl)-N-thiazol-2-yl-propionamid

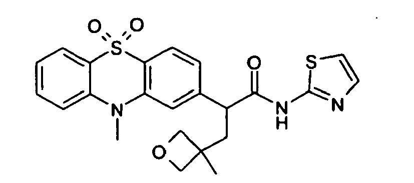

Analog zu Beispiel 1 erhält man aus (10-Methyl-5,5-dioxo-5,10-dihydro-phenothiazin-2-yl)-essigsäureethylester, 3-Iodomethyl-3-methyl-oxetan und kommerziell erhältlichem 2-Amino-thiazol 2-(10-Methyl-5,5-dioxo-5,10-dihydro-phenothiazin-2-yl)-3-(3-methyl-oxetan-3-yl)-N-thiazol-2-yl-propionamid.
C23H23N3O4S2 (469.59), LCMS(ESI): 470.10 (M+H⁺).

### Beispiel 27

### 2-(10-Methyl-5,5-dioxo-5,10-dihydro-phenothiazin-2-yl)-3-(3-methyl-oxetan-3-yl)-N-(1 - methyl-1H-pyrazol-3-yl)-propionamid

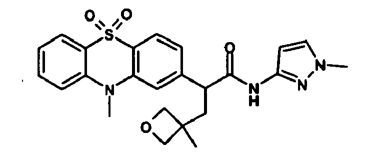

Analog zu Beispiel 1 erhält man aus (10-Methyl-5,5-dioxo-5,10-dihydro-phenothiazin-2-yl)-essigsäureethylester, 3-Iodomethyl-3-methyl-oxetan und kommerziell erhältlichem 1-Methyl-1H-pyrazol-3-amin 2-(10-Methyl-5,5-dioxo-5,10-dihydro-phenothiazin-2-yl)-3-(3-methyloxetan-3-yl)-N-(1-methyl-1H-pyrazol-3-yl)-propionamid.
C24H26N4O4S (466.56), LCMS(ESI): 467.2 (M+H⁺).

### Beispiel 28

### 3-(3-Ethyl-oxetan-3-yl)-2-(10-methyl-5,5-dioxo-5,10-dihydro-phenothiazin-2-yl)-N-thiazol-2-yl-propionamid

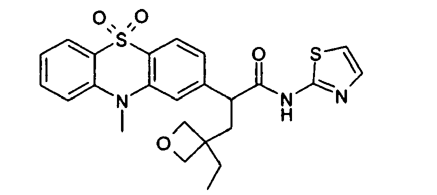

Analog zu Beispiel 1 erhält man aus (10-Methyl-5,5-dioxo-5,10-dihydro-phenothiazin-2-yl)-essigsäureethylester, 3-Ethyl-3-iodomethyl-oxetan und kommerziell erhältlichem 2-Amino-thiazol 3-(3-Ethyl-oxetan-3-yl)-2-(10-methyl-5,5-dioxo-5,10-dihydro-phenothiazin-2-yl)-N-thiazol-2-yl-propionamid.
C24H25N3O4S2 (483.61), LCMS(ESI): 484.1 (M+H⁺).

### Beispiel 29

### 3-(3-Ethyl-oxetan-3-yl)-2-(10-methyl-5,5-dioxo-5,10-dihydro-phenothiazin-2-yl)-N-(1-methyl-1H-pyrazol-3-yl)-propionamid

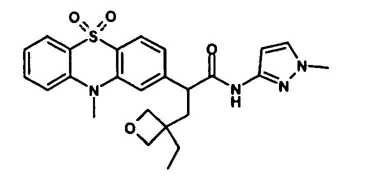

Analog zu Beispiel 1 erhält man aus (10-Methyl-5,5-dioxo-5,10-dihydro-phenothiazin-2-yl)-essigsäureethylester, 3-Ethyl-3-iodomethyl-oxetan und kommerziell erhältlichem 1-Methyl-H-pyrazol-3-amin 3-(3-Ethyl-oxetan-3-yl)-2-(10-methyl-5,5-dioxo-5,10-dihydrophenothiazin-2-yl)-N-(1-methyl-1H-pyrazol-3-yl)-propionamid.
C25H28N4O4S (480.59), LCMS(ESI): 481.2 (M+H⁺).

### Beispiel 30

### 3-(4-Methyl-cyclohexyl)-2-(10-methyl-5,5-dioxo-5,10-dihydro-phenothiazin-2-yl)-N-thiazol-2-yl-propionamid

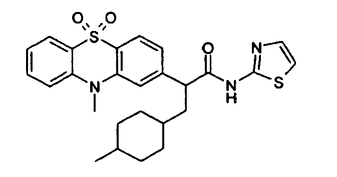

Analog zu Beispiel 1 erhält man aus (10-Methyl-5,5-dioxo-5,10-dihydro-phenothiazin-2-yl)-essigsäureethylester, 1-Iodomethyl-4-methyl-cyclohexan und kommerziell erhältlichem 2-Amino-thiazol 3-(4-Methyl-cyclohexyl)-2-(1-methyl-5,5-dioxo-5,10-dihydro-phenothiazin-2-yl)-N-thiazol-2-yl-propionamid.
C26H29N3O3S2 (495.67), LCMS(ESI): 496.2 (M+H⁺), 537.2 (M+MeCN+H⁺).

### Beispiel 31

### 3-(4-Methyl-cyclohexyl)-2-(10-methyl-5,5-dioxo-5,10-dihydro-phenothiazin-2-yl)-N-(1-methyl-1H-pyrazol-3-yl)-propionamid

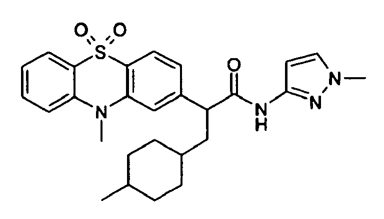

Analog zu Beispiel 1 erhält man aus (10-Methyl-5,5-dioxo-5,10-dihydro-phenothiazin-2-yl)-essigsäureethylester, 1-Iodomethyl-4-methyl-cyclohexan und kommerziell erhältlichem 1-Methyl-1H-pyrazol-3-amin 3-(4-Methyl-cyclohexyl)-2-(10-methyl-5,5-dioxo-5,10-dihydrophenothiazin-2-yl)-N-(1-methyl-1H-pyrazol-3-yl)-propionamid.
C27H32N4O3S(492.65), LCMS(ESI): 493.2 (M+H⁺).

### Beispiel 32

### 3-Indan-2-yl-2-(10-methyl-5,5-dioxo-5,10-dihydro-phenothiazin-2-yl)-N-(1-methyl-1H-pyrazol-3-yl)-propionamid

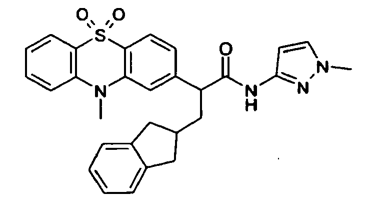

Analog zu Beispiel 1 erhält man aus (10-Methyl-5,5-dioxo-5,10-dihydro-phenothiazin-2-yl)-essigsäureethylester, 2-Iodomethyl-indan und kommerziell erhältlichem 1-Methyl-1H-pyrazol-3-amin 3-Indan-2-yl-2-(10-methyl-5,5-dioxo-5,10-dihydro-phenothiazin-2-yl)-N-(1-methyl-1H-pyrazol-3-yl)-propionamid.
C29H28N4O3S (512.63), LCMS(ESI): 513.2 (M+H⁺).

### Beispiel 33

### 3-Indan-2-yl-N-(5-methoxy-thiazolo[5,4-b]pyridin-2-yl)-2-(10-methyl-5,5-dioxo-5,10-dihydro-phenothiazin-2-yl)-propionamid

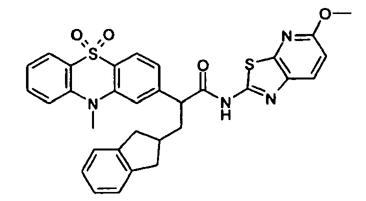

Analog zu Beispiel 1 erhält man aus (10-Methyl-5,5-dioxo-5,10-dihydro-phenothiazin-2-yl)-essigsäureethylester, 2-Iodomethyl-indan und 5-Methoxy-thiazolo[5,4-b]pyridin-2-ylamine 3-Indan-2-yl-N-(5-methoxy-thiazolo[5,4-b]pyridin-2-yl)-2-(10-methyl-5,5-dioxo-5,10-dihydro-phenothiazin-2-yl)-propionamid.
C32H28N4O4S2 (596.73), LCMS(ESI): 597.3 (M+H⁺).

### Beispiel 34

### 2-(10-Methyl-5,5-dioxo-5,10-dihydro-phenothiazin-2-yl)-3-(1-methyl-piperidin-3-yl)-N-thiazol-2-yl-propionamid

### 3-Iodomethyl-1-methyl-piperidin

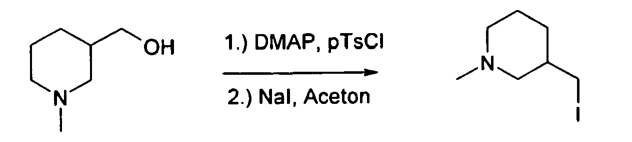

10.0g 1-Methyl-3-piperidinmethanol werden in 100 ml Dichlormethan gelöst und unter Eiskühlung mit 9.46 g 4-Dimethylaminopyridin und 15.5 g p-Toluolsulfonylchlorid versetzt. Die Reaktionslösung wird zwölf Stunden bei Raumtemperatur gerührt, anschließend mit gesättigter NaHCO₃ Lösung gewaschen, über MgSO₄ getrocknet und das Lösungsmittel im Vakuum entfernt. Der resultierende Rückstand wird in 150 ml Aceton gelöst und mit 39.5 g Natriumiodid versetzt. Das Reaktionsgemisch wird zwei Stunden unter Rückfluss zum Sieden erhitzt. Anschließend wird das Lösungsmittel im Vakuum entfernt und der Rückstand in 300 ml Ethylacetat aufgenommen und zweimal mit je 100 ml wasser gewaschen. Die organische Phase wird über MgSO₄ getrocknet und anschließend im Vakuum eingeengt. Man erhält 9.4 g 3-Iodomethyl-1-methyl-piperidin.
C7H14IN (239.10), LCMS(ESI): 240.0 (M+H⁺).

### 2-(10-Methyl-5,5-dioxo-5,10-dihydro-phenothiazin-2-yl)-3-(1-methyl-piperidin-3-yl)-N-thiazol-2-yl-propionamid

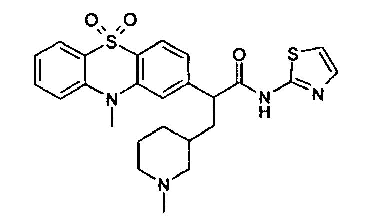

Analog zu Beispiel 1 erhält man aus (10-Methyl-5,5-dioxo-5,10-dihydro-phenothiazin-2-yl)-essigsäureethylester, 3-Iodomethyl-1-methyl-piperidin und und kommerziell erhältlichem 2-Amino-thiazol 2-(10-Methyl-5,5-dioxo-5,10-dihydro-phenothiazin-2-yl)-3-(-methylpiperidin-3-yl)-N-thiazol-2-yl-propionamid.
C25H28N4O3S2 (496.65), LCMS(ESI): 497.2 (M+H⁺).

### Beispiel 35

### 2-(10-Methyl-5,5-dioxo-5,10-dihydro-phenothiazin-2-yl)-3-(1-methyl-piperidin-3-yl)-N-(1-methyl-1H-pyrazol-3-yl)-propionamid

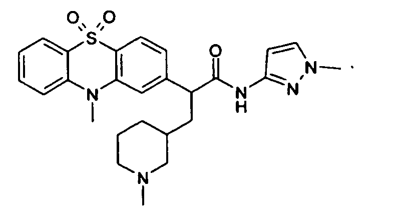

Analog zu Beispiel 1 erhält man aus (10-Methyl-5,5-dioxo-5,10-dihydro-phenothiazin-2-yl)-essigsäureethylester, 3-Iodomethyl-1-methyl-piperidin und und kommerziell erhältlichem 1-Methyl-1H-pyrazol-3-amin 2-(10-Methyl-5,5-dioxo-5,10-dihydro-phenothiazin-2-yl)-3-(1-methyl-piperidin-3-yl)-N-(1-methyl-1H-pyrazol-3-yl)-propionamid.
C26H31N5O3S (493.63), LCMS(ESI): 494.2 (M+H⁺).

### Beispiel 36

### 2-(10-Methyl-5,5-dioxo-5,10-dihydro-phenothiazin-2-yl)-N-(1-methyl-1H-pyrazol-3-yl)-3-(4-oxo-cyclohexyl)-propionamid

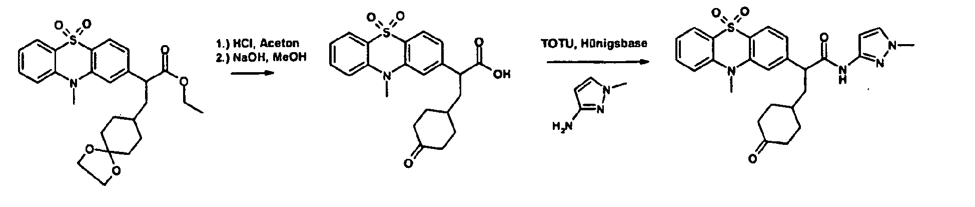

### 2-(10-Methyl-5,5-dioxo-5,10-dihydro-phenothiazin-2-yl)-3-(4-oxo-cyclohexyl)-propionsäure

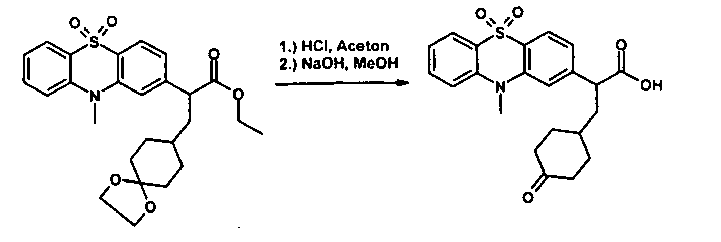

1.1g 3-(1,4-Dioxa-spiro[4.5]dec-8-yl)-2-(10-methyl-5,5-dioxo-5,10-dihydro-phenothiazin-2-yl)-propiosäureethylester (hergestellt analog zu Beispiel 1 aus (10-Methyl-5,5-dioxo-5,10-dihydro-phenothiazin-2-yl)-essigsäureethylester und 8-Iodomethyl-1,4-dioxa-spiro[4.5]decan) werden in 20 ml Aceton gelöst und mit 4 ml 10%iger Salzsäure versetzt. Das Reaktionsgemisch wird bei Raumtemperatur drei Stunden gerührt. Anschließend wird das Reaktionsgemisch mit gesättigter NaHCO₃ Lösung gewaschen, über MgSO₄ getrocknet und anschließend im Vakuum eingeengt. Der Rückstand wird in 20 ml Methanol gelöst und mit 7.9 ml 2M Natronlauge versetzt und eine Stunde unter Rückfluss zum Sieden erhitzt. Das erkaltete Reaktionsgemisch wird durch Zugabe von 200 ml Ethylacetat verdünnt und mit 2N HCl angesäuert. Anschließend wird die organische Phase zweimal mit je 80 ml Wasser und gesättigter Kochsasalzlösung gewaschen, über MgSO₄ getrocknet und danach im Vakuum eingeengt. Man erhält 600 mg 2-(10-Methyl-5,5-dioxo-5,10-dihydro-phenothiazin-2-yl)-3-(4-oxo-cyclohexyl)-propionsäure.
C22H23NO5S (413.50), LCMS(ESI): 414.1 (M+H⁺).

### 2-(10-Methyl-5,5-dioxo-5,10-dihydro-phenothiazin-2-yl)-N-(1-methyl-1H-pyrazol-3-yl)-3-(4-oxo-cyclohexyl)-propionamid

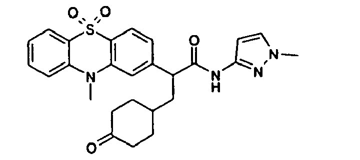

Analog zu Beispiel 1 erhält man aus 2-(10-Methyl-5,5-dioxo-5,10-dihydro-phenothiazin-2-yl)-3-(4-oxo-cyclohexyl)-propionsäure und kommerziell erhältlichem 1-Methyl-1H-pyrazol-3-amin 2-(10-Methyl-5,5-dioxo-5,10-dihydro-phenothiazin-2-yl)-N-(1-methyl-1H-pyrazol-3-yl)-3-(4-oxo-cyclohexyl)-propionamid.
C26H28N4O4S (492.60), LCMS(ESI): 493.2 (M+H⁺).
Das racemische Gemisch wird an chiraler Phase (S,S-Whelk 51) mit dem Eluens n-Heptan:Methanol:Ethanol =4:1:1+0.1% Diethylamin in die Enantiomere (Rt = 10.68 min und Rt= 12.54 min) getrennt.

### Beispiel 37

### 3-(4-Hydroxy-cyclohexyl)-2-(10-methyl-5,5-dioxo-5,10-dihydro-phenothiazin-2-yl)-N-(1-methyl-1H-pyrazol-3-yl)-propionamid

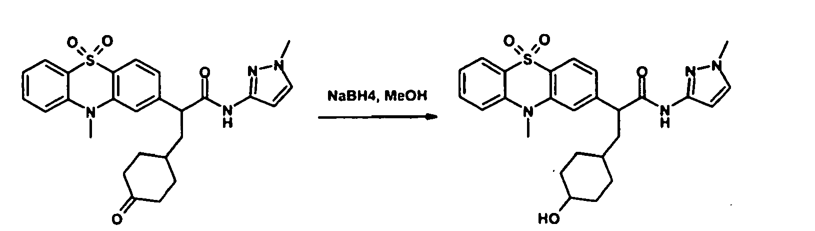

50 mg 2-(10-Methyl-5,5-dioxo-5,10-dihydro-phenothiazin-2-yl)-N-(1-methyl-1H-pyrazol-3-yl)-3-(4-oxo-cyclohexyl)-propionamid wird in 20 ml Methanol suspendiert und bei Raumtemperatur mit 3.8 mg Natriumborhydrid versetzt. Man rührt eine Stunde bei Raumtemperatur nach. Im Reaktionszeitraum entsteht langsam eine klare Lösung. Das Methanol wird im Vakuum entfernt und der Rückstand mit Ethylacetat und Wasser extrahiert. Die organische Phase wird zweimal mit Wasser gewaschen, anschließend über MgSO₄ getrocknet und im Vakuum eingeengt. Der Rückstand wird lyophilisiert. Man erhält 42 mg 3-(4-Hydroxy-cyclohexyl)-2-(10-methyl-5,5-dioxo-5,10-dihydro-phenothiazin-2-yl)-N-(1-methyl-1H-pyrazol-3-yl)-propionamid als weißen amorphen Feststoff.
C26H30N4O4S (494.62), LCMS(ESI): 495.3 (M+H⁺).

### Beispiel 38

### 3-(4-Hydroxyimino-cyclohexyl)-2-(10-methyl-5,5-dioxo-5,10-dihydro-phenothiazin-2-yl)-N-(1-methyl-1H-pyrazol-3-yl)-propionamid

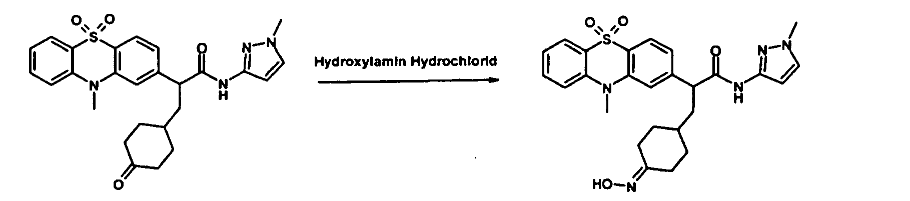

67 mg 2-(10-Methyl-5,5-dioxo-5,10-dihydro-phenothiazin-2-yl)-N-(1-methyl-1H-pyrazol-3-yl)-3-(4-oxo-cyclohexyl)-propionamid und 14 mg Hydroxylaminhydrochlorid werden in 13 ml Methanol gelöst und drei Stunden unter Rückfluss zum Sieden erhitzt. Anschhließend wird das Methanol im Vakuum entfernt und der resultierende Rückstand in einem Gemisch aus 20 ml Ethylacetat und 10 ml Wasser aufgenommen. Die organische. Phase wird zweimal mit je 10 ml Wasser gewaschen, über MgSO₄ getrocknet, im Vakuum eingeengt und anschließend lyophilisiert. Man erhält 49 mg 3-(4-Hydroxyimino-cyclohexyl)-2-(10-methyl-5,5-dioxo-5,10-dihydro-phenothiazin-2-yl)-N-(1-methyl-1H-pyrazol-3-yl)-propionamid. C26H29N5O4S (507.62), LCMS(ESI): 508.2 (M+H⁺).

### Beispiel 39

### 3-(1,1-Dioxo-tetrahydro-thiophen-3-yl)-N-(5-methoxy-thiazolo[5,4-b]pyridin-2-yl)-2-(10-methyl-5,5-dioxo-5,10-dihydro-phenothiazin-2-yl)-propionamid

### 3-Iodomethyl-tetrahydro-thiophen-1,1-dioxide

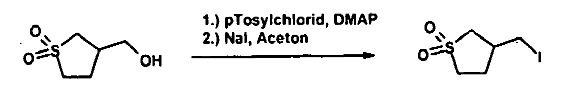

1.0g (1,1-Dioxo-tetrahydro-thiophen-3-yl)-methanol werden in 30 ml Dichlormethan gelöst und unter Eiskühlung mit 902 mg 4-Dimethylaminopyridin und 1.33 g p-Toluolsulfonylchlorid versetzt. Die Reaktionslösung wird zwölf Stunden bei Raumtemperatur gerührt, anschließend mit gesättigter NaHCO₃ Lösung gewaschen, über MgSO₄ getrocknet und das Lösungsmittel im Vakuum entfernt. Rf des Zwischenproduktes (Tosylat) in n-Heptan:Ethylacetat = 1:1:Rf(Tosylat) = 0,18. Der resultierende Rückstand wird in 30 ml Aceton gelöst und mit 3.5 g Natriumiodid versetzt. Das Reaktionsgemisch wird sechs Stunden unter Rückfluss zum Sieden erhitzt. Anschließend wird das Lösungsmittel im Vakuum entfernt und der Rückstand in 150 ml Ethylacetat aufgenommen und zweimal mit je 50 ml Wasser gewaschen. Die organische Phase wird über MgSO₄ getrocknet und anschließend im Vakuum eingeengt. Man erhält 1.44 g 3-Iodomethyl-tetrahydro-thiophen-1,1-dioxide.
CSH9IO2S (260.09), LCMS(ESI): 260.9 (M+H⁺), Rf(n-Heptan:Ethylacetat = 4:1) =0.33 .

### 3-(1,1-Dioxo-tetrahydro-thiophen-3-yl)-N-(5-methoxy-thiazolo[5,4-b]pyridin-2-yl)-2-(10-methyl-5,5-dioxo-5,10-dihydro-phenothiazin-2-yl)-propionamid

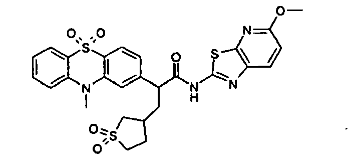

Analog zu Beispiel 1 erhält man aus (10-Methyl-5,5-dioxo-5,10-dihydro-phenothiazin-2-yl)-essigsäureethylester, 3-Iodomethyl-tetrahydro-thiophen-1,1-dioxide und 5-Methoxy-thiazolo[5,4-b]pyridin-2-ylamin 3-(1,1-Dioxo-tetrahydro-thiophen-3-yl)-N-(5-methoxy-thiazolo [5,4-b]pyridin-2-yl)-2-(10-methyl-5,5-dioxo-5,10-dihydro-phenothiazin-2-yl)-propionamid.
C27H26N4O6S3 (598.72), LCMS(ESI): 599.1 (M+H⁺).

### Beispiel 40

### 2-(10-Methyl-5,5-dioxo-5,10-dihydro-phenothiazin-2-yl)-N-(1-methyl-1H-pyrazol-3-yl)-3-(3-oxo-cyclopentyl)-propionamid

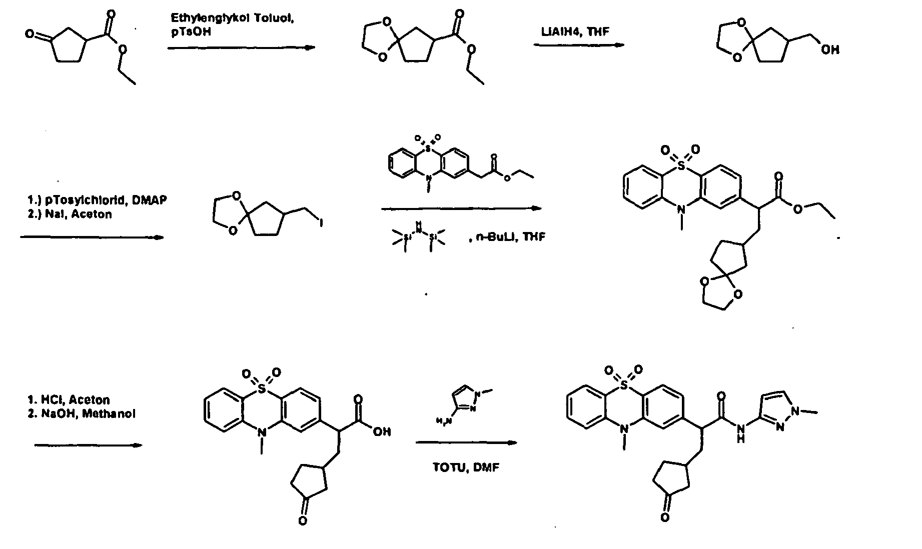

### 1,4-Dioxa-spiro[4.4]nonane-7-carbonsäureethylester

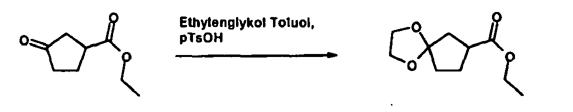

Eine Lösung von 9.9 g 3-Oxo-cyclopentancarbonsäureethylester, 600 mg p-Toluolsulfonsäuremonohydrat und 3.5 ml Ethylenglykol in 100 ml Toluol werden vier Stunden an einem Wasserabscheider unter Rückfluss zum Sieden erhitzt. Das Lösungsmittel wird im Vakuum entfernt und der Rückstand in 250 ml Ethylacetat aufgenommen und mit 150 ml Wasser gewaschen. Die organische Phase wird über MgSO₄ getrocknet und im Vakuum eingeengt. Der Rückstand wird an Kieselgel mit dem Eluens n-Heptan:Ethylacetat als linearer Gradient von n-Heptan zu n-Heptan:Ethylacetat =7:10 gereinigt. Man erhält 8.65 g 1,4-Dioxa-spiro[4.4]nonan-7-carbonsäureethylester als Öl.
C1OH16O4 (200.24), Rf(n-Heptan:Ethylacetat = 2:1) = 0.38.

### (1,4-Dioxa-spiro[4.4]non-7-yl)-methanol

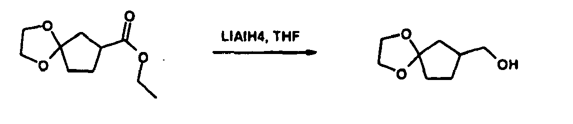

1.22 g Lithiumaluminiumhydrid werden in 200 ml trockenem Tetrahydrofuran vorgelegt und auf-20°C abgekühlt. Bei diesder Temperatur werden 8.65 g 1,4-Dioxa-spiro[4.4]nonan-7-carbonsäureethylester gelöst in 50 ml Tetrahydrofuran unter Argon zugetropft. Nach einer Stunde wird das Kältbad entfernt und die Reaktionsmischung drei Stunden nachgerührt. Durch Zugabe von 10 ml Wasser wird das Reaktionsgemisch abgelöscht und zwei Stunden bei Raumtemperatur nachgerührt. Das Reaktionsgemsich wird filtriert und das Filtrat im Vakuum eingeengt. Man erhält 6.7 g (1,4-Dioxa-spiro[4.4]non-7-yl)-methanol als ÖI.
C8H1403 (158.20), Rf(n-Heptan:Ethylacetat = 2:1) = 0.09 .

### 7-Iodomethyl-1,4-dioxa-spiro[4.4]nonan

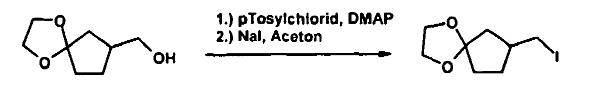

16.7 g (1,4-Dioxa-spiro[4.4]non-7-yl)-methanol werden in 160 ml Dichlormethan gelöst und unter Eiskühlung mit 5.74 g 4-Dimethylaminopyridin und 8.48 g p-Toluolsulfonylchlorid versetzt. Die Reaktionslösung wird zwölf Stunden bei Raumtemperatur gerührt, anschließend mit gesättigter NaHCO₃ Lösung gewaschen, über MgSO₄ getrocknet und das Lösungsmittel im Vakuum entfernt. Rf des Zwischenproduktes (Tosylat) in n-Heptan:Ethylacetat = 4:1: Rf (Tosylat) = 0,09. Der resultierende Rückstand wird in 160 ml Aceton gelöst und mit 21.58 g Natriumiodid versetzt. Das Reaktionsgemisch wird sechs Stunden unter Rückfluss zum Sieden erhitzt. Anschließend wird das Lösungsmittel im Vakuum entfernt und der Rückstand in 150 ml Ethylacetat aufgenommen und zweimal mit je 50 ml Wasser gewaschen. Die organische Phase wird über MgSO₄ getrocknet und anschließend im Vakuum eingeengt. Man erhält 9.1 g 7-Iodomethyl-1,4-dioxa-spiro[4.4]nonan.
C8H13IO2 (268.10), LCMS(ESI): 269.1 (M+H⁺), Rf(n-Heptan:Ethylacetat =4:1) =0.34.

### 2-(10-Methyl-5,5-dioxo-5,10-dihydro-phenothiazin-2-yl)-N-(1-methyl-1H-pyrazol-3-yl)-3-(3-oxo-cyclopentyl)-propionamid

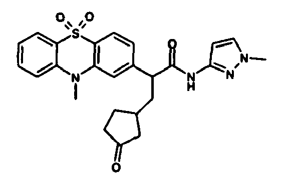

Analog zu Beispiel 1 und Beispiel 36 erhält man aus (10-Methyl-5,5-dioxo-5,10-dihydrophenothiazin-2-yl)-essigsäureethylester, 7-Iodomethyl-1,4-dioxa-spiro[4.4]nonan und kommerziell erhältlichem 1-Methyl-1H-pyrazol-3-amin 2-(10-Methyl-5,5-dioxo-5,10-dihydro-phenothiazin-2-yl)-N-(1-methyl-1H-pyrazol-3-yl)-3-(3-oxo-cyclopentyl)-propionamid.
C25H26N4O4S (478.57), LCMS(ESI): 479.2 (M+H⁺).
Das racemische Diastereomerengemisch wird an chiraler Phase (IA-103) mit dem Eluens Methanol:Ethanol = 1:1 in die Isomere (Rt = 8.276 min, Rt = 9.897 min, Rt = 10.539 min und Rt= 12.117 min) getrennt.

### Beispiel 41

### N-(5-Methoxy-thiazolo[5,4-b]pyridin-2-yl)-2-(10-methyl-5,5-dioxo-5,10-dihydrophenothiazin-2-yl)-3-((R)-3-oxo-cyclopentyl)-propionamid

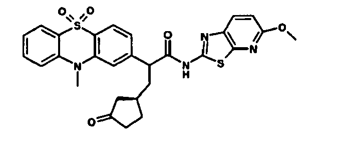

Analog zu Beispiel 1 und Beispiel 36 erhält man aus (10-Methyl-5,5-dioxo-5,10-dihydrophenothiazin-2-yl)-essigsäureethylester, (S)-7-Iodomethyl-1,4-dioxa-spiro[4.4]nonan (hergestellt analog zu Beispiel 40 aus kommerziell erhältlichem (S)-3-Oxo-cyclopentanecarbonsäuremethylester) und 5-Methoxy-thiazolo[5,4-b]pyridin-2-ylamin N-(5-Methoxy-thiazolo[5,4-b]pyridin-2-yl)-2-(10-methyl-5,5-dioxo-5,10-dihydro-phenothiazin-2-yl)-3-((R)-3-oxo-cyclopentyl)-propionamid.
C28H26N4O5S2 (562.67), LCMS(ESI): 563.1 (M+H⁺).

### Beispiel 42

### 10-Methyl-8-[1-(1-methyl-1H-pyrazol-3-ylcarbamoyl)-2-(3-oxo-cyclopentyl)-ethyl]-5,5-dioxo-5,10-dihydro-phenothiazin-3-carbonsäureethylester

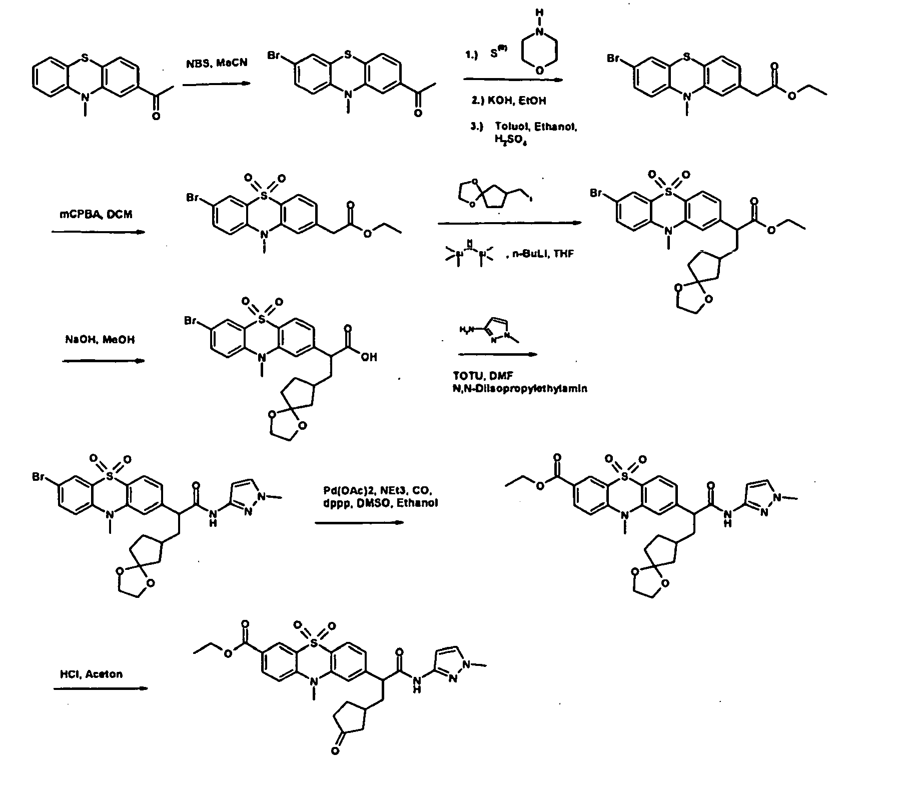

### 1-(7-Bromo-10-methyl-10H-phenothiazin-2-yl)-ethanon

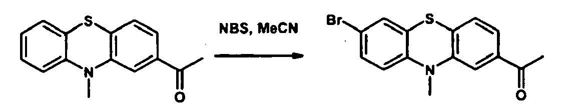

9.93 g 1-(10-Methyl-10H-phenothiazin-2-yl-ethanon wird in 300 ml Acetonitril gelöst, und mit 8.31 g N-Bromsuccinimid versetzt. Das Reaktionsgemisch färbt sich schwarz.
Das Reaktionsgemisch wird durch Zugabe von 500 ml Ethylacetat verdünnt und dreimal mit je 200 ml gesättigter Natriumthiosulfatlösung und gesättigter NaHCO₃ Lösung gewaschen, über MgSO₄ getrocknet und anschließend die Lösungsmittel im Vakuum entfernt. Der resultierende Rückstand wird an Kieselgel mit dem Eluens n-Heptan => n-Heptan:Ethylacetat => Ethylacetat als linearer Gradient gereinigt. Man erhält 10.8 g 1-(7-Bromo-10-methyl-10H-phenothiazin-2-yl)-ethanon als gelben Feststoff.
C15H12BrNOS (334.24), LCMS(ESI): 333.95, 334.95, 335.95 (M+H⁺), Rf(n-Heptan:Ethylacetat=4:1)=0.21.

### 10-Methyl-8-[1-(1-methyl-1H-pyrazol-3-ylcarbamoyl)-2-(3-oxo-cyclopentyl)-ethyl]-5,5-dioxo-5,10-dihydro-phenothiazin-3-carbonsäureethylester

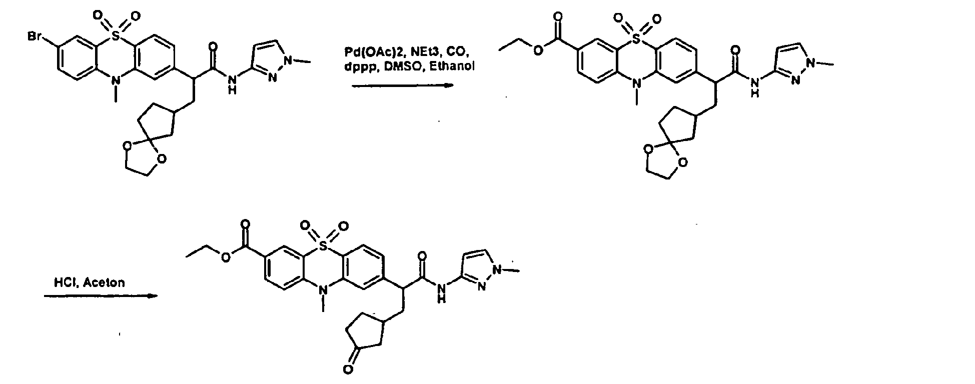

Eine Lösung von 445 mg 2-(7-Bromo-10-methyl-5,5-dioxo-5,10-dihydro-phenothiazin-2-yl)-3-(1,4-dioxa-spiro[4.4]non-7-yl)-N-(1-methyl-1H-pyrazol-3-yl)-propionamid (hergestellt analog zu Beispiel 1 aus (10-Methyl-5,5-dioxo-5,10-dihydro-phenothiazin-2-yl)-essigsäureethylester, 7-Iodomethyl-1,4-dioxa-spiro[4.4]nonan und kommerziell erhältlichem 1-Methyl-1H-pyrazol-3-amin) werden in 8 ml wasserfreiem Ethanol und 8 ml DMSO gelöst und mit 0.74 ml Triethylamin und 76 mg 1,3-Bis(diphenylphosphino)propan unter Argon versetzt. Die Reaktionslösung wird mit Argon gespült und dann mit 35 mg Palladiumacetat versetzt. Das Reaktionsgemisch wird drei Stunden bei 70°C und 1 bar CO gerührt. Das abgekühlte Reaktionsgemisch wird durch Zugabe von 200 ml Ethylacetat verdünnt und mit je 100 ml Wasser und gesättigter Kochsalzlösung gewaschen, über MgSO₄ getrocknet und anschließend im Vakuum eingeengt. Der Rückstand wird in 25 ml Aceton gelöst und mit 3 ml 10%iger Salzsäure versetzt. Das Reaktionsgemisch wird bei Raumtemperatur drei Stunden gerührt. Anschließend wird das Reaktionsgemisch mit gesättigter NaHCO₃ Lösung gewaschen, über MgSO₄ getrocknet und anschließend im Vakuum eingeengt. Der Rückstand wird an Kieselgel mit dem Eluens n-Heptan:Ethylacetat = 1:1 => Ethylacetat => Ethylacetat:Methanol = 99:1 gereinigt. Man erhält 844 mg 10-Methyl-8-[1-(1-methyl-1H-pyrazol-3-ylcarbamoyl)-2-(3-oxo-cyclopentyl)-ethyl]-5,5-dioxo-5,10-dihydro-phenothiazin-3-carbonsäureethylester.
C28H30N4O6S (550.64), LCMS(ESI): 551.2 .

### Beispiel 43

### 10-Methyl-8-[1-(1-methyl-1H-pyrazol-3-ylcarbamoyl)-2-(3-oxo-cyclopentyl)-ethyl]-5,5-dioxo-5,10-dihydro-phenothiazin-3-carbonsäure

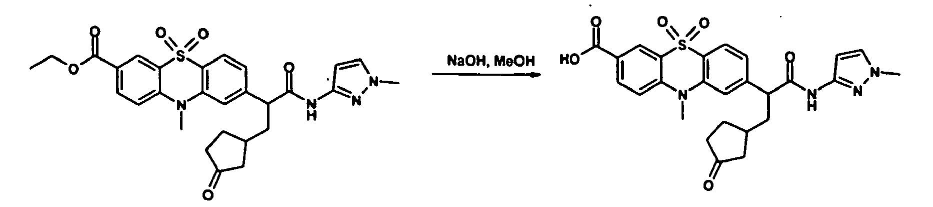

260 mg 10-Methyl-8-[1-(1-methyl-1H-pyrazol-3-ylcarbamoyl)-2-(3-oxo-cyclopentyl)-ethyl]-5,5-dioxo-5,10-dihydro-phenothiazin-3-carbonsäureethylester werden in 10 ml Methanol suspendiert und mit 1.64 ml 2M NaOH Lösung versetzt. Das Reaktionsgemisch wird eine Stunde unter Rückfluss zum Sieden erhitzt. Das Methanol wird im Vakuum entfernt und das Reaktionsgemisch durch Zugabe konzentrierter Salzsäure auf pH 4 eingestellt. Man extrahiert dreimal mit je 100 ml Ethylacetat. Die vereinigten organischen Phasen werden über MgSO₄ getrocknet und anschließend im Vakuum eingeengt. Man erhält 155 mg 10-Methyl-8-[1-(1-methyl-1H-pyrazol-3-ylcarbamoyl)-2-(3-oxo-cyclopentyl)-ethyl]-5,5-dioxo-5,10-dihydrophenothiazin-3-carbonsäure.
C26H26N4O6S (522.58), LCMS(ESI): 523.20 (M+H⁺).

### Beispiel 44

### 2-(5,5-Dioxo-5,10-dihydro-phenothiazin-2-yl)-N-(1-methyl-1H-pyrazol-3-yl)-3-(tetrahydro-pyran-4-yl)-propionamid

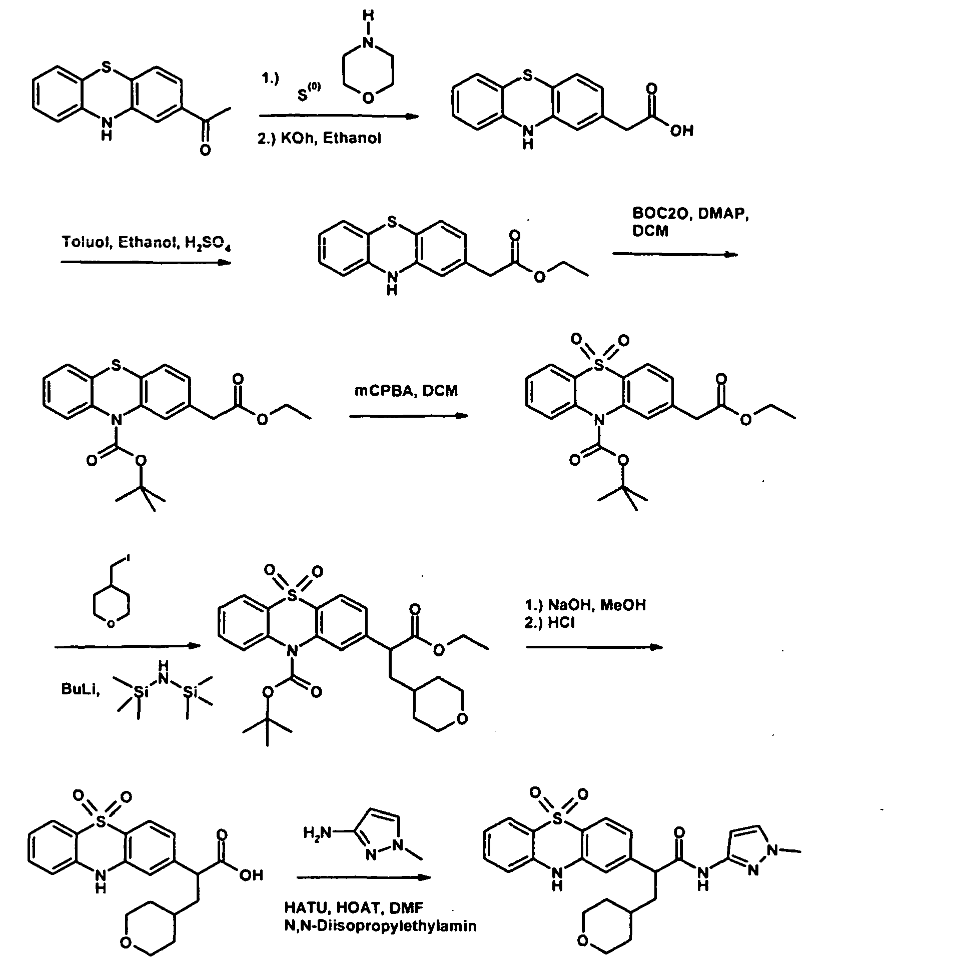

### 2-Ethoxycarbonylmethyl-5,5-dioxo-5H-phenothiazin-10-carbonsäure-tert-butylester

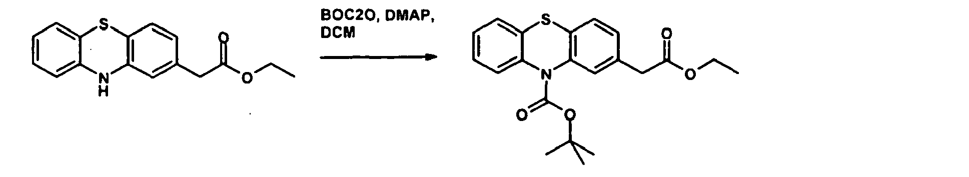

18.5 g (10H-Phenothiazin-2-yl)-essigsäureethylester (hergestellt analog zu Beispiel 1 aus 2-Acetylphenothiazin) werden in 300 ml Acetonitril gelöst und mit 21.2 g Di-tert-butyldicarbonat und 1.58 g 4-Dimethylaminopyridin verstezt. Das Reakionsgemisch wird drei Stunden unter Rückfluss zum Sieden erhitzt. Das abgekühlte Reaktionsgemisch wird durch Zugabe von 500 ml Ethylacetat verdünnt und dreimal mit je 200 ml 0.5 %iger Zitronensäurelösung gewaschen. Die organische Phase wird über MgSO₄ getrocknet und anschließend im Vakuum eingeengt. Man erhält 25.0 g 2-Ethoxycarbonylmethyl-5,5-dioxo-5H-phenothiazin-10-carbonsäure-tert-butylester als farblosen amorphen Schaum. C21H23NO4S (385.49), LCMS(ESI): 330.1 (M-tert Butyl+H⁺), Rf(n-Heptan:Ethylacetat = 3:1) =0.49.

### 2-Ethoxycarbonylmethyl-5,5-dioxo-5H-phenothiazin-10-carbonsäure-tert-butylester

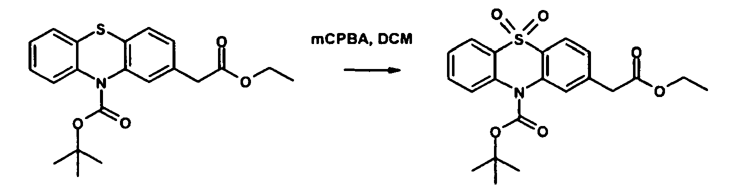

25.0 g 2-Ethoxycarbonylmethyl-5,5-dioxo-5H-phenothiazin-10-carbonsäure-tert-butylester werden in 500 ml Dichlormethan gelöst und portionsweise mit 50.4 g mCPBA versetzt. Das Reaktionsgemisch wird eine Stunde bei Raumtemperatur gerührt. Danach wird das Reaktionsgemisch mit 200 ml gesättigter NaHCO₃-Lösung , 5x mit je 200 ml 2 M NaOH und einmal mit 100 ml gesättigter NaCl-Lösung gewaschen, über MgSO₄ getrocknet und anschließend das Lösungsmittel im Vakuum entfernt. Man erhält 14.4 g 2-Ethoxycarbonylmethyl-5,5-dioxo-5H-phenothiazin-10-carbonsäure-tert-butylester als amorphen farblosen Schaum.
C21H23NO6S (417.48), LCMS(ESI):435.1 (M+NH4⁺), 362.1 (M-tert.Butyl+H⁺), Rf(n-Heptan: EE=1:1)=0.31 .

### 2-[1-Ethoxycarbonyl-2-(tetrahydro-pyran-4-yl)-ethyl]-5,5-dioxo-5H-phenothiazin-10-carbonsäure-tert-butylester

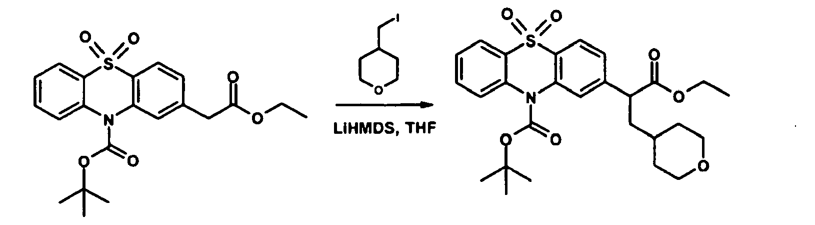

1.31 ml 1,1,1,3,3,3-Hexamethyldisilazan werden in 20 ml Tetrahydrofuran unter Argon gelöst. Unter Eiskühlung werden 2.3 ml n-Buthyllithium (2.5 M in n-Hexan) zugetropft und 30 Minuten bei 0°C nachgerührt. Anschließend wird diese Lösung zu einer gerührten Lösung von 2.0 g 2-Ethoxycarbonylmethyl-5,5-dioxo-5H-phenothiazin-10-carbonsäure-tert-butylester in 100 ml Tetrahydrofuran bei -78°C zugetropft. Das Reaktionsgemisch wird 20 Minuten bei - 78°C gerührt, dann werden 1.1 g 4-(Iodomethyl)tetrahydro-2H-pyran zugetropft. Das Kühlbad wird entfernt und man lässt langsam auf Raumtemperatur erwärmen. Der Reaktionsansatz wird über Nacht bei Raumtemperatur gerührt. Dann werden 10 ml Wasser zugegeben, das Tetrahydrofuran im Vakuum entfernt und der Rückstand dreimal mit je 100 ml Ethylacetat extrahiert. Die vereinigten organischen Phasen werden über MgSO₄ getrocknet und anschließend im Vakuum eingeengt. Der Rückstand wird an Kieselgel mit dem Eluens n-Heptan:Ethylacetat (100%:0%)=> n-Heptan:Ethylacetat (50%:50%) gereinigt. Man erhält 700 mg 2-[1-Ethoxycarbonyl-2-(tetrahydro-pyran-4-yl)-ethyl]-5,5-dioxo-5H-phenothiazin-10-carbonsäure-tert-butylester.
C27H33NO7S (515.63), LCMS(ESI): 533.2 (M+NH4⁺), 460.1 (M-tert.Butyl+H⁺), Rf(n-Heptan:EE= 1:1) = 0.28.

### 2-(5,5-Dioxo-5,10-dihydro-phenothiazin-2-yl)-3-(tetrahydro-pyran-4-yl)-propionsäure

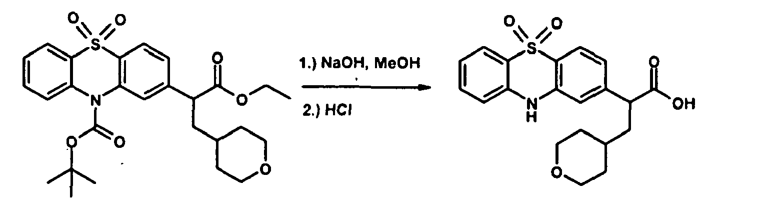

700 mg 2-[1-Ethoxycarbonyl-2-(tetrahydro-pyran-4-yl)-ethyl]-5,5-dioxo-5H-phenothiazin-10-carbonsäure-tert-butylester werden in 50 ml Methanol suspendiert und mit 10 ml 2 M NaOH Lösung versetzt. Das Reaktionsgemisch wird eine Stunde bei 60°C gerührt. Das Methanol wird im Vakuum entfernt und das Reaktionsgemisch durch Zugabe konzentrierter Salzsäure auf pH 4 eingestellt. Man extrahiert dreimal mit je 100 ml Ethylacetat. Die vereinigten organischen Phasen werden über MgSO₄ getrocknet und anschließend im Vakuum eingeengt. Man erhält 600 mg 2-(5,5-Dioxo-5,10-dihydro-phenothiazin-2-yl)-3-(tetrahydro-pyran-4-yl)-propionsäure.
C20H21NO5S (387.46), LCMS(ESI): 388.1 (M+H⁺).

### 2-(5,5-Dioxo-5,10-dihydro-phenothiazin-2-yl)-N-(1-methyl-1H-pyrazol-3-yl)-3-(tetrahydro-pyran-4-yl)-propionamid

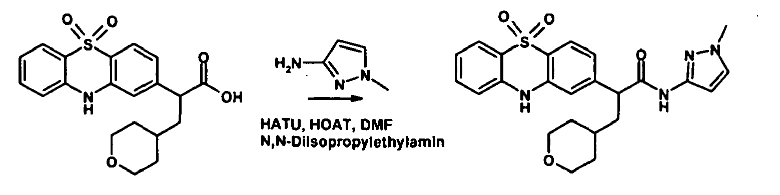

600 mg 2-(5,5-Dioxo-5,10-dihydro-phenothiazin-2-yl)-3-(tetrahydro-pyran-4-yl)-propionsäure, 120 mg kommerziell erhältliches 1-Methyl-1H-pyrazol-3-amin und 729 µl N,N-Diisopropylethylamin werden in 5 ml Dimethylformamid gelöst. Man gibt 561 mg HATU und 200 mg HOAT hinzu und rührt zwei Stunden bei Raumtemperatur. Danach wird das Reaktionsgemisch durch Zugabe von 200 ml Ethylacetat verdünnt und dreimal mit je 50 m! Wasser und gesättigter Kochsalzlösung gewaschen. Die organische Phase wird über MgSO₄ getrocknet und anschließend das Lösungsmittel im Vakuum entfernt. Der Rückstand wird mittels RP-HPLC gereinigt. Man erhält 200 mg 2-(5,5-Dioxo-5,10-dihydrophenothiazin-2-yl)-N-(1-methyl-1H-pyrazol-3-yl)-3-(tetrahydro-pyran-4-yl)-propionamid als farbloses Lyophilisat.
C24H26N4O4S (466.17), LCMS(ESI): 467.2(M+H⁺).

### Beispiel 45

### 2-(5,5-Dioxo-5,10-dihydro-phenothiazin-2-yl)-N-(1-methyl-1H-pyrazol-3-yl)-3-(3-oxocyclopentyl)-propionamid

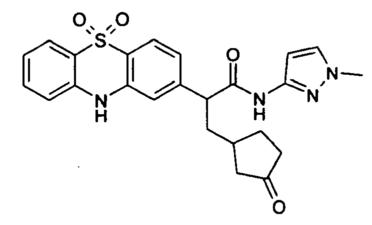

Analog zu Beispiel 36 und Beispiel 44 erhält man aus 2-Ethoxycarbonylmethyl-5,5-dioxo-5H-phenothiazin-10-carbonsäure-tert-butylester, 7-Iodomethyl-1,4-dioxa-spiro[4.4]nonan und 1-Methyl-1H-pyrazol-3-amin 2-(5,5-Dioxo-5,10-dihydro-phenothiazin-2-yl)-N-(1-methyl-1H-pyrazol-3-yl)-3-(3-oxo-cyclopentyl)-propionamid.
C24H24N4O4S (464.55), LCMS(ESI): 465.4 (M+H⁺).

### Beispiel 46

### 2-(5,5-Dioxo-5,10-dihydro-phenothiazin-2-yl)-N-(1-methyl-1H-pyrazol-3-yl)-3-((R)-3-oxo-cyclopentyl)-propionamid

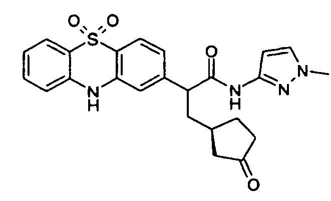

Analog zu Beispiel 36 und Beispiel 44 erhält man aus 2-Ethoxycarbonylmethyl-5,5-dioxo-5H-phenothiazin-10-carbonsäure-tert-butylester, (2R,3R,7S)-7-Iodomethyl-2,3-diphenyl-1,4-dioxa-spiro[4.4]nonan und 1-Methyl-1H-pyrazol-3-amin 2-(5,5-Dioxo-5,10-dihydrophenothiazin-2-yl)-N-(1-methyl-1H-pyrazol-3-yl)-3-((R)-3-oxo-cyclopentyl)-propionamid. C24H24N4O4S (464.55), LCMS(ESI): 465.4 (M+H⁺).

### Beispiel 47

### 2-(5,5-Dioxo-5,10-dihydro-phenothiazin-2-yl)-N-(5-methoxy-thiazolo[5,4-b]pyridin-2-yl)-3-(3-oxo-cyclopentyl)-propionamid

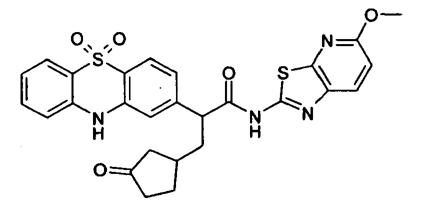

Analog zu Beispiel 36 und Beispiel 44 erhält man aus 2-Ethoxycarbonylmethyl-5,5-dioxo-5H-phenothiazin-10-carbonsäure-tert-butylester, 7-Iodomethyl-1,4-dioxa-spiro[4.4]nonan und 5-Methoxy-thiazolo[5,4-b]pyridin-2-ylamin 2-(5,5-Dioxo-5,10-dihydro-phenothiazin-2-yl)-N-(5-methoxy-thiazolo[5,4-b]pyridin-2-yl)-3-(3-oxo-cyclopentyl)-propionamid. C27H24N4O5 (548.64), LCMS(ESI): 549.4 (M+H⁺).

### Beispiel 48

### 2-(5,5-Dioxo-5,10-dihydro-phenothiazin-2-yl)-N-(5-methoxy-thiazolo[5,4-b]pyridin-2-yl)-3-((R)-3-oxo-cyclopentyl)-propionamid

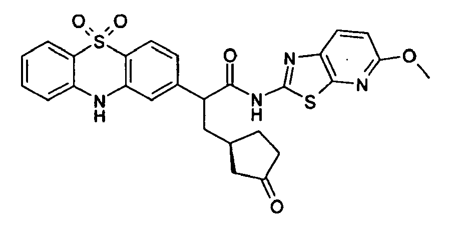

Analog zu Beispiel 36 und Beispiel 44 erhält man aus 2-Ethoxycarbonylmethyl-5,5-dioxo-5H-phenothiazin-10-carbonsäure-tert-butylester, 7(2R,3R,7S)-7-Iodomethyl-2,3-diphenyl-1,4-dioxa-spiro[4.4]nonan und 5-Methoxy-thiazolo[5,4-b]pyridin-2-ylamin 2-(5,5-Dioxo-5,10-dihydro-phenothiazin-2-yl)-N-(5-methoxy-thiazolo[5,4-b]pyridin-2-yl)-3-((R)-3-oxo-cyclopentyl)-propionamid.
C27H24N4O5 (548.64), LCMS(ESI): 549.4 (M+H⁺).

Das Diastereomerengemisch wird an chiraler Phase (Chiracel OD-H/74) mit dem Eluens n-Hptan:Ethanol:Methanol =4:1.1 in die Diastereomeren (Rt = 7.745 min und Rt = 9.064 min) getrennt.

### Beispiel 49

### N-(1-Benzyl-1H-pyrazol-3-yl)-2-(5,5-dioxo-5,10-dihydro-phenothiazin-2-yl)-3-(3-oxo-cyclopentyl)-propionamid

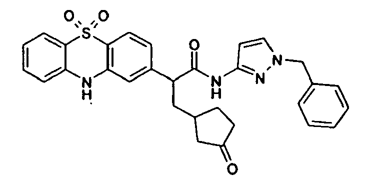

Analog zu Beispiel 36 und Beispiel 44 erhält man aus 2-Ethoxycarbonylmethyl-5,5-dioxo-5H-phenothiazin-10-carbonsäure-tert-butylester, 7-Iodomethyl-1,4-dioxa-spiro[4.4]nonan und 1-Benzyl-1H-pyrazol-3-ylamin N-(1-Benzyl-1H-pyrazol-3-yl)-2-(5,5-dioxo-5,10-dihydro-phenothiazin-2-yl)-3-(3-oxo-cyclopentyl)-propionamid.
C30H28N4O4S (540.65), LCMS(ESI): 541.44 (M+H⁺).

### Beispiel 50

### 3-(4,4-Difluoro-cyclohexyl)-2-(5,5-dioxo-5,10-dihydro-phenothiazin-2-yl)-N-(5-methoxy-thiazolo[5,4-b]pyridin-2-yl)-propionamid

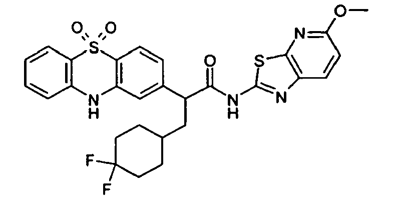

Analog zu Beispiel 36 und Beispiel 44 erhält man aus 2-Ethoxycarbonylmethyl-5,5-dioxo-5H-phenothiazin-10-carbonsäure-tert-butylester, 1,1-Difluoro-4-iodomethyl-cyclohexan (hergestellt aus kommerziell erhältlichem 4,4-Difluoro-cyclohexancarbonsäureethylester analog zur Iodidsynthese beschrieben in Beispiel 40) und 5-Methoxy-thiazolo[5,4-b]pyridin-2-ylamin 3-(4,4-Difluoro-cyclohexyl)-2-(5,5-dioxo-5,10-dihydro-phenothiazin-2-yl)-N-(5-methoxy-thiazolo[5,4-b]pyridin-2-yl)-propionamid.
C28H26F2N4O4S2 (584.67), LCMS(ESI): 585.1 (M+H⁺), 626.1 (M+MeCN+H⁺).

### Beispiel 51

### N-(1-Benzyl-1H-pyrazol-3-yl)-3-(4,4-difluoro-cyclohexyl)-2-(5,5-dioxo-5,10-dihydrophenothiazin-2-yl)-propionamid

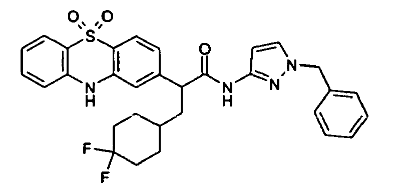

Analog zu Beispiel 36 und Beispiel 44 erhält man aus 2-Ethoxycarbonylmethyl-5,5-dioxo-5H-phenothiazin-10-carbonsäure-tert-butylester, 1,1-Difluoro-4-iodomethyl-cyclohexan (hergestellt aus kommerziell erhältlichem 4,4-Difluoro-cyclohexancarbonsäureethylester analog zur Iodidsynthese beschrieben in Beispiel 40) und 1-Benzyl-1H-pyrazol-3-ylamin N-(1-Benzyl-1H-pyrazol-3-yl)-3-(4,4-difluoro-cyclohexyl)-2-(5,5-dioxo-5,10-dihydrophenothiazin-2-yl)-propionamid.
C31H30F2N4O3S (576.67), LCMS(ESI): 577.3 (M+H⁺).

### Beispiel 52

### 3-(4,4-Difluoro-cyclohexyl)-2-(5,5-dioxo-5,10-dihydro-phenothiazin-2-yl)-N-(1-methyl-1H-pyrazol-3-yl)-propionamid

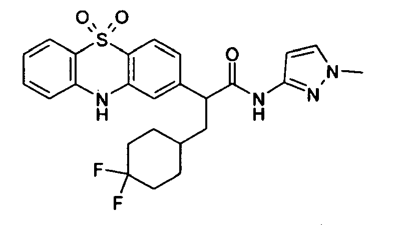

Analog zu Beispiel 36 und Beispiel 44 erhält man aus 2-Ethoxycarbonylmethyl-5,5-dioxo-5H-phenothiazin-10-carbonsäure-tert-butylester, 1,1-Difluoro-4-iodomethyl-cyclohexan (hergestellt aus kommerziell erhältlichem 4,4-Difluoro-cyclohexancarbonsäureethylester analog zur Iodidsynthese beschrieben in Beispiel 40) und 1-Methyl-1H-pyrazol-3-amin 3-(4,4-Difluoro-cyclohexyl)-2-(5,5-dioxo-5,10-dihydro-phenothiazin-2-yl)-N-(1-methyl-1H-pyrazol-3-yl)-propionamid.
C25H26F2N4O3S (500.57), LCMS(ESI): 501.2 (M+H⁺).

### Beispiel 53

### 2-(5,5-Dioxo-5,10-dihydro-phenothiazin-2-yl)-3-(4-fluoro-cyclohex-3-enyl)-N-(5-methoxy-thiazolo[5,4-b]pyridin-2-yl)-propionamid

### 1-Fluoro-4-iodomethyl-cyclohexen

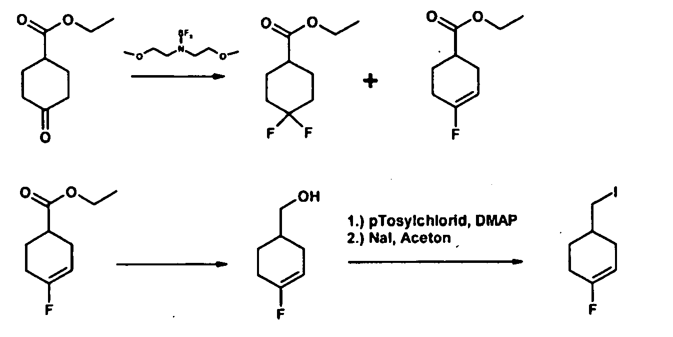

### 4-Fluoro-cyclohex-3-encarbonsäureethylester

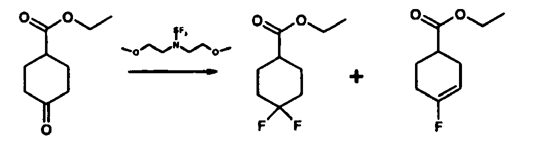

4.0 g 4-Oxo-cyclohexanearbonsäureethylester werden in einem Teflonkolben unter Argon in 50 ml Dichlormethan gelöst. Unter Eiskühlung werden 13 ml [Bis(2-methoxyethyl)amino]schwefeltrifluorid (50%ige Lösung in Toluol) zugegeben. Das Reaktionsgemisch wird zwölf Stunden bei Raumtemperatur gerührt, dann auf Eis gegossen und fünfmal mit je 100 ml Dichlormethan extrahiert. Die vereinigten organischen Phasen werden zweimal mit je 100 ml Wasser gewaschen, über MgSO₄ getrocknet und im Vakuum eingeengt. Der resultierende Rückstand wird an Kieselgel mit dem Eluens n-Heptan => n-Heptan:Ethylacetat = 10:3 gereinigt und das gewünschte Produkt vom mitentstandenem 4,4-Difluoro-cyclohexancarbonsäureethylester abgetrennt. Man erhält 726 mg 4-Fluoro-cyclohex-3-encarbonsäureethylester.
C9H13F02 (172.20), GC-MS: 172 (M⁺), Rf(n-Heptan:Ethylacetat = 4:1) = 0.51 .

### (4-Fluoro-cyclohex-3-enyl)-methanol

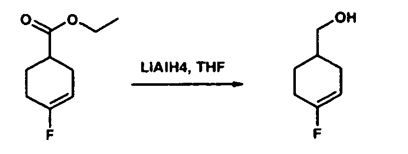

119 mg Lithiumaluminiumhydrid werden in 20 ml trockenem Tetrahydrofuran vorgelegt und auf -20°C abgekühlt. Bei dieser Temperatur werden 720 mg 4-Fluoro-cyclohex-3-encarbonsäureethylester gelöst in 10 ml Tetrahydrofuran unter Argon zugetropft. Nach einer Stunde wird das Kältbad entfernt und die Reaktionsmischung drei Stunden nachgerührt. Duch Zugabe von 10 ml Wasser wird das Reaktionsgemisch abgelöscht und zwei Stunden bei Raumtemperatur nachgerührt. Das Reaktionsgemsich wird filtriert und das Filtrat im Vakuum eingeengt. Man erhält 584 mg (4-Fluoro-cyclohex-3-enyl)-methanol als farbloses ÖI. C7H11FO (130.16), Rf(n-Heptan:Ethylacetat = 2:1) = 0.22 .

### 1-Fluoro-4-iodomethyl-cyclohexen

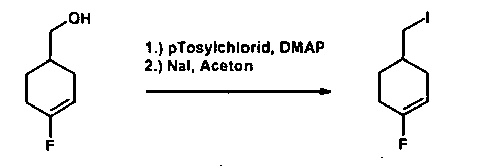

584 mg (4-Fluoro-cyclohex-3-enyl)-methanol werden in 10ml Dichlormethan gelöst und unter Eiskühlung mit 610 mg 4-Dimethylaminopyridin und 900 mg p-Toluolsulfonylchlorid versetzt. Die Reaktionslösung wird zwölf Stunden bei Raumtemperatur gerührt, anschließend mit gesättigter NaHCO₃ Lösung gewaschen, über MgSO₄ getrocknet und das Lösungsmittel im Vakuum entfernt. Der resultierende Rückstand wird in 15 ml Aceton gelöst und mit 2.29 g Natriumiodid versetzt. Das Reaktionsgemisch wird drei Stunden unter Rückfluss zum Sieden erhitzt. Anschließend wird das Lösungsmittel im Vakuum entfernt und der Rückstand in 100 ml Ethylacetat aufgenommen und zweimal mit je 30 ml Wasser gewaschen. Die organische Phase wird über MgSOp₄ getrocknet und anschließend im Vakuum eingeengt. Man erhält 696 mg 1-Fluoro-4-iodomethyl-cyclohexen.
C7H10FI (240.06), Rf(n-Heptan:Ethylacetat = 4:1) = 0.61.

### 2-(5,5-Dioxo-5,10-dihydro-phenothiazin-2-yl)-3-(4-fluoro-cyclohex-3-enyl)-N-(5-methoxy-thiazolo[5,4-b]pyridin-2-yl)-propionamid

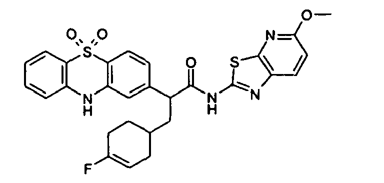

Analog zu Beispiel 36 und Beispiel 44 erhält man aus 2-Ethoxycarbonylmethyl-5,5-dioxo-5H-phenothiazin-10-carbonsäure-tert-butylester, 1-Fluoro-4-iodomethyl-cyclohexen und 5-Methoxy-thiazolo[5,4-b]pyridin-2-ylamin 2-(5,5-Dioxo-5,10-dihydro-phenothiazin-2-yl)-3-(4-fluoro-cyclohex-3-enyl)-N-(5-methoxy-thiazolo[5,4-b]pyridin-2-yl)-propionamid. C28H25FN4O4S2 (564.66), LCMS(ESI): 565.2

### Beispiel 54

### 2-(5,5-Dioxo-5,10-dihydro-phenothiazin-2-yl)-3-(4-fluoro-cyclohex-3-enyl)-N-(1-methyl-1H-pyraol-3-yl)-propionamid

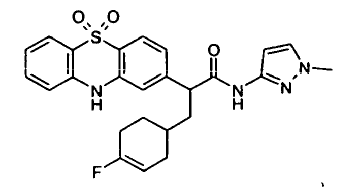

Analog zu Beispiel 36 und Beispiel 44 erhält man aus 2-Ethoxycarbonylmethyl-5,5-dioxo-5H-phenothiazin-10-carbonsäure-tert-butylester, 1-Fluoro-4-iodomethyl-cyclohexen und 1-Methyl-1H-pyrazol-3-amin 2-(5,5-Dioxo-5,10-dihydro-phenothiazin-2-yl)-3-(4-fluoro-cyclohex-3-enyl)-N-(1-methyl-1H-pyrazol-3-yl)-propionamid.
C25H25FN4O3S (480.57), LCMS(ESI): 481.2

### Beispiel 55

### 3-(3,3-Difluoro-cyclopentyl)-2-(5,5-dioxo-5,10-dihydro-phenothiazin-2-yl)-N-(5-methoxy-thiazolo[5,4-b]pyridin-2-yl)-propionamid

### 1,1-Difluoro-3-iodomethyl-cyclopentan

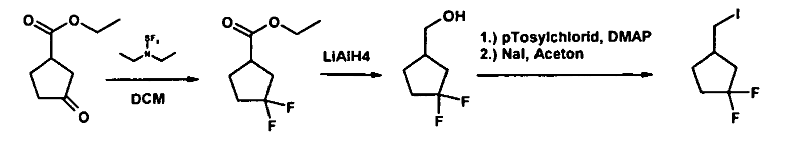

### 3,3-Difluoro-cyclopentancarbonsäureethylester

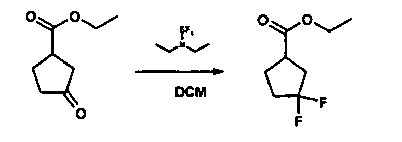

1.0 g 3-Oxo-cyclopentancarbonsäureethylester werden in einem Teflonkolben unter Argon in 1 ml Dichlormethan gelöst. Unter Eiskühlung werden 0.84 ml Diethylaminoschwefeltrifluorid zugegeben. Man läßt das Reaktionsgemisch innerhalb von zwei Stunden langsam auf Raumtemperatur erwärmen und rührt zwei Tage bei Raumtemperatur nach. Anschließend wird das Reaktionsgemisch durch Zugabe von 100 ml Dichlormethan verdünnt zweimal mit je 50 ml Wasser gewaschen, über MgSO₄ getrocknet und im Vakuum eingeengt. Man erhält 1.12 g 3,3-Difluoro-cyclopentancarbonsäureethylester.
C8H12F2O2 (178.20), GC-MS: 178.0 (M⁺).

### (3,3-Difluoro-cyclopentyl)-methanol

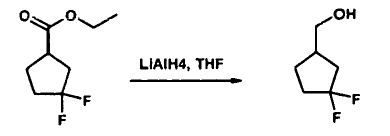

6.4 ml einer 1M Lösung von Lithiumaluminiumhydrid in THF werden in 40 ml Diethylether vorgelegt. Unter Eiskühlung 1.12 g 3,3-Difluoro-cyclopentancarbonsäureethylester gelöst in 10 ml Diethylether unter Argon zugetropft. Nach einer Stunde wird das Kältbad entfernt und die Reaktionsmischung drei Stunden nachgerührt. Dann wird das Reaktionsgemisch auf eiskalte gesättigte Ammoniumchloridlösung gegossen und über eine Filtrierhilfe abgesaugt. Das Filtrat wird mit gesättigter NaHCO₃ Lösung gewaschen, über MgSO₄ getrocknet und im Vakuum eingeengt. Man erhält 625 mg (3,3-Difluoro-cyclopentyl)-methanol.
C6H10F20 (136.14), GC-MS: 99.0 (M⁺-F-H2O).

### 1,1-Difluoro-3-iodomethyl-cyclopentan

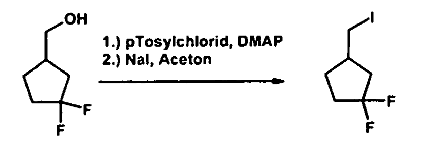

620 mg (3,3-Difluoro-cyclopentyl)-methanol werden in 17 ml Dichlormethan gelöst und unter Eiskühlung mit 618 mg 4-Dimethylaminopyridin und 911 mg p-Toluolsulfonylchlorid versetzt. Die Reaktionslösung wird zwölf Stunden bei Raumtemperatur gerührt, anschließend mit gesättigter NaHCO₃ Lösung gewaschen, über MgSO₄ getrocknet und das Lösungsmittel im Vakuum entfernt. Der resultierende Rückstand wird in 20 ml Aceton gelöst und mit 2.32 g Natriumiodid versetzt. Das Reaktionsgemisch wird sechs Stunden unter Rückfluss zum Sieden erhitzt. Anschließend wird das Lösungsmittel im Vakuum entfernt und der Rückstand in 100 ml Ethylacetat aufgenommen und zweimal mit je 30 ml Wasser gewaschen. Die organische Phase wird über MgSO₄ getrocknet und anschließend im Vakuum eingeengt. Man erhält 570 mg 1,1-Difluoro-3-iodomethyl-cyclopentan.
C6H9F2I (246.04).

### 3-(3,3-Difluoro-cyclopentyl)-2-(5,5-dioxo-5,10-dihydro-phenothiazin-2-yl)-N-(5-methoxy-thiazolo[5,4-b]pyridin-2-yl)-propionamid

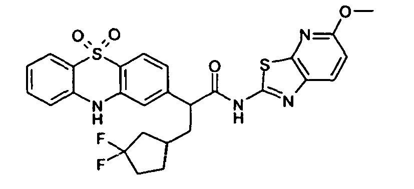

Analog zu Beispiel 36 und Beispiel 44 erhält man aus 2-Ethoxycarbonylmethyl-5,5-dioxo-5H-phenothiazin-10-carbonsäure-tert-butylester, 1,1-Difluoro-3-iodomethyl-cyclopentan und 5-Methoxy-thiazolo[5,4-b]pyridin-2-ylamin 3-(3,3-Difluoro-cyclopentyl)-2-(5,5-dioxo-5,10-dihydro-phenothiazin-2-yl)-N-(5-methoxy-thiazolo[5,4-b]pyridin-2-yl)-propionamid. C27H24F2N4O4S2 (570.64), LCMS(ESI): 571.2

### Beispiel 56

### 3-(3,3-Difluoro-cydopentyl)-2-(5,5-dioxo-5,10-dihydro-phenothiazin-2-yl)-N-(1-methyl-1H-pyrazol-3-yl)-propionamid

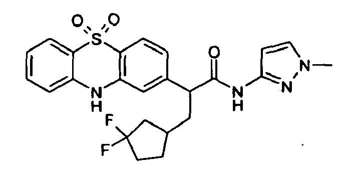

Analog zu Beispiel 36 und Beispiel 44 erhält man aus 2-Ethoxycarbonylmethyl-5,5-dioxo-5H-phenothiazin-10-carbonsäure-tert-butylester, 1,1-Difluoro-3-iodomethyl-cyclopentan und 1-Methyl-1H-pyrazol-3-amin 3-(3,3-Difluoro-cyclopentyl)-2-(5,5-dioxo-5,10-dihydrophenothiazin-2-yl)-N-(1-methyl-1H-pyrazol-3-yl)-propionamid.
C24H24F2N4O3S (486.54), LCMS(ESI): 487.2

## Patentansprüche

1. Verbindungen der Formel I, 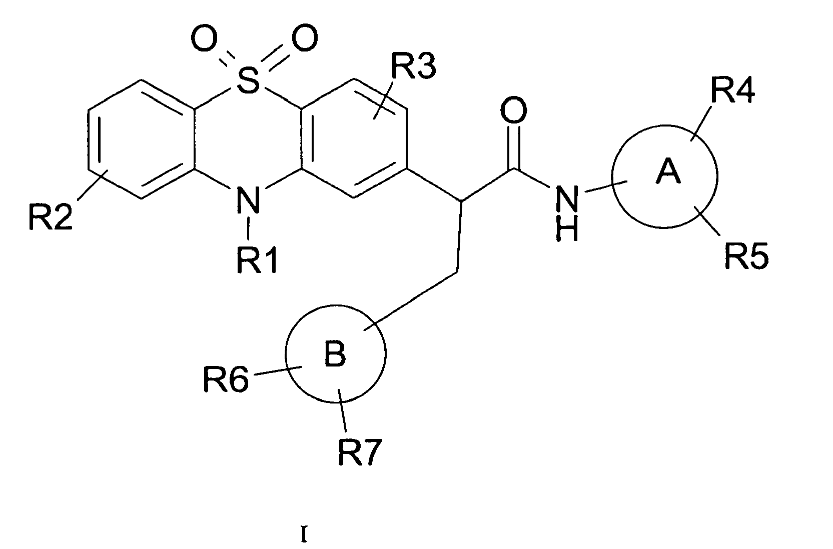 worin bedeuten
R1 H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
R2, R3 unabhängig voneinander H, F, Cl, Br, CN, NO₂ (C₀-C₆)-Alkylen-COO-(C₀- C₆)-Alkyl, (C₀-C₆)-Alkylen-O-(C₀-C₆)-Alkyl, (C₁-C₆)-Alkyl, (C₀-C₆)-Alkylen- CO-(C₁-C₆)-Alkyl, (C₀-C₆)-Alkylen-Phenyl, SCF₃, SF₅, SCH₃;
R4, R5 unabhängig voneinander H, F, Cl, Br, CN, SCN, NO₂ =O, (Co-C6)-Alkylen- COO-(C₀-C₆)-Alkyl, -CO-COO-(C₀-C₆)-Alkyl, (C₀-C₆)-Alkylen-O-(C₀-C₆)- Alkyl, (C₀-C₆)-Alkyl, (C₀-C₆)-Alkylen-CO-(C₁-C₆)-Alkyl, (C₀-C₆)-Alkylen- CONH(C₀-C₆)-Alkyl, (C₀-C₆)-Alkylen-CON[(C₀-C₆)-Alkyl]₂, (C₀-C6)-Alkylen- NH(C₀-C₆)-Alkyl, (C₀-C₆)-Alkylen-NH-COO-(C₀-C₆)-Alkyl, (C₀-C₆)-Alkylen- CON[(C₀-C₆)-Alkyl]-O-(C₀-C₆)-Alkyl, (C₀-C₆)-Alkylen-N[(C₀-C₆)-Alkyl]₂, (C₀-C₆)-Alkylen-Aryl, SF₅, (C₀-C₆)-Alkyl-S(O)ₓ(C₁-C₆)-Alkyl, S(O)ₓ(C₁-C₆)- Alkylen-COO-(C₀-C₆)-Alkyl, S(O)ₓ(C₂-C₆)-Alkylen-O-(C₀-C₆)-Alkyl, -SO₂- NH-(C₀-C₆)-Alkyl, -SO₂-N-[(C₀-C₆)-Alkyl]₂, S(O)ₓ(C₀-C₆)-Alkylen- Heterocyclus, S(O)ₓ(C₁-C₆)-Alkylen-CO-Heterocyclus, NH-SO₂-(C₁-C₆)- Alkyl, (C₀-C₆)-Alkylen-Cycloalkyl, (C₀-C₆)-Alkylen-Heterocyclus, (C₀-C₆)- Alkylen-Aryl;
x 0, 1, 2;
R6, R7 unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀- C₆)-Alkylen-O-CO-(C₁-C₆)-Alkyl, (C₁-C₆)-Alkyl, (C₀-C₆)-Alkylen-CO-(C₁- C₆)-Alkyl, (C₀-C₆)-Alkylen-Aryl, SF₅, S(O)ₓ-(C₁-C₆)-Alkyl;
A 5 bis 10 gliedriger Heterocyclus, wobei der Heterocyclus mit einem weiteren 5 bis 10 gliedrigen Ring anneliert sein kann;
B 4 bis 8 gliedriger Cycloalkylring, ein 4 bis 10 gliedriger Heterocyclus oder ein 6 bis 10 gliedriger Arylring;
sowie deren physiologisch verträgliche Salze,
wobei unter der Defintion (C₀-C₆)-Alkylen- verstanden wird, dass entweder eine Bindung oder eine (C₁-C₆)-Alkylen Gruppe vorhanden sein kann und wobei unter der Defintion -(C₀-C₆)-Alkyl verstanden wird , dass entweder ein Wasserstoff oder eine (C₁-C₆)-Alkyl Gruppe vorhanden sein kann,
wobei unter einem Cycloalkylrest ein einen oder mehrere Ringe enthaltendes Ringssystem, welches gesättigt oder partiell ungesättigt (mit einer oder zwei Doppelbindungen) vorliegt, verstanden wird, das ausschließlich aus Kohlenstoffatomen aufgebaut ist.

2. Verbindungen der Formel I, gemäß Anspruch 1, **dadurch gekennzeichnet, dass** darin bedeuten
R1 H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
R2, R3 unabhängig voneinander H, F, Cl, Br, CN, NO₂ (C₀-C₆)-Alkylen-COO-(C₀- C₆)-Alkyl, (C₀-C₆)-Alkylen-O-(C₀-C₆)-Alkyl, (C₁-C₆)-Alkyl, (C₀-C₆)-Alkylen- CO-(C₁-C₆)-Alkyl, (C₀-C₆)-Alkylen-Phenyl, SCF₃, SF₅, SCH₃;
R4, R5 unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁-C₆)-Alkyl, (C₀-C₆)-Alkylen-CO-(C₁-C₆)-Alkyl, (C₀-C₆)-Alkylen- CONH(C₀-C₆)-Alkyl, (C₀-C₆)-Alkylen-CON[(C₀-C₆)-Alkyl]₂, (C₀-C₆)-Alkylen- NH( C₀-C₆)-Alkyl, (C₀-C₆)-Alkylen-NH-COO-(C₀-C₆)-Alkyl, (C₀-C₆)-Alkylen- CON[(C₀-C₆)-Alkyl]-0-(C₀-C₆)-Alkyl, (C₀-C₆)-Alkylen-N[(C₀-C₆)-Alkyl]₂, (C₀-C₆)-Alkylen-Aryl, SF₅, (C₀-C₆)-Alkyl-S(O)ₓ(C₁-C₆)-Alkyl, S(O)ₓ(C₁-C₆)- Alkylen-COO-(C₁-C₆)-Alkyl, S(O)ₓ(C₂-C₆)-Alkylen-O-(C₀-C₆)-Alkyl, -SO₂- NH-(C₀-C₆)-Alkyl, -SO₂-N-[(C₀-C₆)-Alkyl]₂, S(O)ₓ(C₀-C₆)-Alkylen- Heterocyclus, S(O)ₓ(C₁-C₆)-Alkylen-CO-Heterocyclus, -NH-SO₂-(C₁-C₆)- Alkyl, (C₀-C₆)-Alkylen-Cycloalkyl, (C₀-C₆)-Alkylen-Heterocyclus, (C₀-C₆)- Alkylen-Aryl;
x 0, 1, 2;
R6, R7 unabhängig voneinander H, F, Cl, Br, CN, NO₂, =O, =S, =N-O-(C₀-C₆)-Alkyl, (C₀-C₆)-Alkylen-COO-(C₀-C₆)-Alkyl, (C0₋C₆)-Alkylen-O-(C₀-C₆)-Alkyl, (C₀- C₆)-Alkylen-O-CO-(C₁-C₆)-Alkyl, (C₁-C₆)-Alkyl, (C₀-C₆)-Alkylen-CO-(C₁- C₆)-Alkyl, (C₀-C₆)-Alkylen-Aryl, SF₅, S(O)ₓ-(C₁-C₆)-Alkyl;
A 5 bis 10 gliedriger Heterocyclus, wobei der Heterocyclus mit einem weiteren 5 bis 10 gliedrigen Ring anneliert sein kann;
B 4 bis 8 gliedriger Cycloalkylring, ein 4 bis 10 gliedriger Heterocyclus oder ein 6 bis 10 gliedriger Arylring;
sowie deren physiologisch verträgliche Salze.

3. Verbindungen der Formel I, gemäß Anspruch 1 oder 2, **dadurch gekennzeichnet, dass** darin bedeuten
R1 H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
R2, R3 unabhängig voneinander H, F, C1, Br, CN, NO₂, (C₀-C₆)-Alkylen-COO-(C₀- C₆)-Alkyl, (C₀-C₆)-Alkylen-O-(C₀-C₆)-Alkyl, (C₁-C₆)-Alkyl, (C₀-C₆)-Alkylen- CO-(C,-C₆)-Alkyl, (C₀-C₆)-Alkylen-Phenyl, SCF₃, SF₅, SCH₃;
R4, R5 unabhängig voneinander H, F, Cl, Br, CN, SCN, NO₂, (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁-C₆)-Alkyl, (C₀-C₆)-Alkylen-CO-(C₁-C₆)-Alkyl, (C₀-C₆)-Alkylen- CONH(C₀-C₆)-Alkyl, (C₀-C₆)-Alkylen-CON[(C₀-C₆)-Alkyl]₂, (C₀-C₆)-Alkylen- NH(C₀-C₆)-Alkyl, (C₀-C₆)-Alkylen-NH-COO-(C₀-C₆)-Alkyl, (C₀-C₆)-Alkylen- CON[(C₀-C₆)-Alkyl]-O-(C₀-C₆)-Alkyl, (C₀-C₆)-Alkylen-N[(C₀-C₆)-Alkyl]₂, (C₀-C₆)-Alkylen-Aryl, SF₅, (C₀-C₆)-Alkyl-S(O)ₓ(C₁-C₆)-Alkyl, S(O)ₓ(C₁-C₆)- Alkylen-COO-(C₀-C₆)-Alkyl, S(O)ₓ(C₂-C₆)-Alkylen-O-(C₀-C₆)-Alkyl, -SO₂- NH-(C₀-C₆)-Alkyl, -SO₂-N-[(C₀-C₆)-Alkyl]₂, S(O)ₓ(C₀-C₆)-Alkylen- Heterocyclus, S(O)ₓ(C₁-C₆)-Alkylen-CO-Heterocyclus, -NH-SO₂-(C₁-C₆)- Alkyl, (C₀-C₆)-Alkylen-Cycloalkyl, (C₀-C₆)-Alkylen-Heterocyclus, (C₀-C₆)- Alkylen-Aryl;
x 0, 1, 2;
R6, R7 unabhängig voneinander H, F, Cl, Br, CN, NO₂ =O =S, =N-O-(C₀-C₆)-Alkyl, (C₀-C₆)-Alkylen-COO-(C₀-C₆)-Alkyl, (C₀-C₆)-Alkylen-O-(C₀-C₆)-Alkyl, (C₀- C₆)-Alkylen-O-CO-(C₁-C₆)-Alkyl, (C₁-C₆)-Alkyl, (C₀-C₆)-Alkylen-CO-(C₁- C₆)-Alkyl, (C₀-C₆)-Alkylen-Aryl, SF₅, S(O)ₓ-(C₁-C₆)-Alkyl;
A 5 bis 10 gliedriger Heterocyclus, der in alpha Stellung eine -C=N- Bindung enthält, wobei der Heterocyclus mit einem weiteren 5 bis 10 gliedrigen Ring anneliert sein kann;
B 4 bis 8 gliedriger Cycloalkylring, ein 4 bis 10 gliedriger Heterocyclus oder ein 6 bis 10 gliedriger Arylring;
sowie deren physiologisch verträgliche Salze.

4. Verbindungen der Formel I, gemäß einem oder mehreren der Ansprüche 1 bis 3, **dadurch gekennzeichnet, dass** darin bedeuten
R1 H, (C₁-C₆)-Alkyl, (C₀-C₆)-Alkylen-Aryl, CO-(C₁-C₆)-Alkyl, (C₂-C₆)-Alkylen- COO-(C₀-C₆)-Alkyl, (C₂-C₆)-Alkylen-O-(C₁-C₆)-Alkyl;
R2, R3 H;
R4, R5 unabhängig voneinander H, F, Cl, Br, CN, SCN, NO₂ (C₀-C₆)-Alkylen-COO- (C₀-C₆)-Alkyl, -CO-COO-(C₀-C₆)-Alkyl, (C₀-C₆)-Alkylen-O-(C₀-C₆)-Alkyl, (C₁-C₆)-Alkyl, (C₀-C₆)-Alkylen-CO-(C₁-C₆)-Alkyl, (C₀-C₆)-Alkylen- CONH(C₀-C₆)-Alkyl, (C₀-C₆)-Alkylen-CON[(C₀-C₆)-Alkyl]₂, (C₀-C₆)-Alkylen- NH(C₀-C₆)-Alkyl, (C₀-C₆)-Alkylen-NH-COO-(C₀-C₆)-Alkyl, (C₀-C₆)-Alkylen- CON[(C₀-C₆)-Alkyl]-O-(C₀-C₆)-Alkyl, (C₀-C₆)-Alkylen-N[(C₀-C₆)-Alkyl]₂, (Co-C6)-Alkylen-Aryl, SF₅, (C₀-C₆)-Alkyl-S(O)ₓ(C₁-C₆)-Alkyl, S(O)ₓ(C₁-C₆)- Alkylen-COO-(C₀-C₆)-Alkyl, S(O)ₓ(C₂-C₆)-Alkylen-O-(C₀-C₆)-Alkyl, -SO₂- NH-(C₀-C₆)-Alkyl, -SO₂-N-[(C₀-C₆)-Alkyl]₂, S(O)ₓ(C₀-C₆)-Alkylen- Heterocyclus, S(O)ₓ(C₁-C₆)-Alkylen-CO-Heterocyclus, -NH-SO₂-(C₁-C₆)- Alkyl, (C₀-C₆)-Alkylen-Cycloalkyl, (C₀-C₆)-Alkylen-Heterocyclus, (C₀-C₆)- Alkylen-Aryl;
x 0, 1, 2;
R6, R7 unabhängig voneinander H, F, Cl, Br, CN, NO₂, =O =S, =N-O-(CO-C₆)-Alkyl, (C₀-C₆)-Alkylen-COO-(C₀-C₆)-Alkyl, (C₀-C₆)-Alkylen-O-(C₀-C₆)-Alkyl, (C₀- C₆)-Alkylen-O-CO-(C₁-C₆)-Alkyl, (C₁-C₆)-Alkyl, (C₀-C₆)-Alkylen-CO-(C₁- C₆)-Alkyl, (C₀-C₆)-Alkylen-Aryl, SF₅, S(O)ₓ-(C₁-C₆)-Alkyl;
A ein 5 gliedriger Heterocyclus, der in alpha Stellung eine -C=N- Bindung enthält, wobei der Heterocyclus mit einem weiteren 5 bis 10 gliedrigen Ring anneliert sein kann;
B 4 bis 8 gliedriger Cycloalkylring, ein 4 bis 10 gliedriger Heterocyclus oder ein 6 bis 10 gliedriger Arylring;
sowie deren physiologisch verträgliche Salze.

5. Verbindungen gemäß einem oder mehreren der Ansprüche 1 bis 4, zur Anwendung als Arzneimittel.

6. Arzneimittel enthaltend eine oder mehrere der Verbindungen gemäß einem oder mehreren der Ansprüche 1 bis 4 .

7. Arzneimittel enthaltend eine oder mehrere der Verbindungen gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

9. Verwendung der Verbindungen gemäß einem oder mehreren der Ansprüche 1 bis 4 zur Herstellung eines Medikamentes zur Behandlung des Diabetes.

10. Verwendung der Verbindungen gemäß einem oder mehreren der Ansprüche 1 bis 4 zur Herstellung eines Medikamentes zur Blutzuckersenkung.

11. Verwendung der Verbindungen gemäß einem oder mehreren der Ansprüche 1 bis 4 zur Herstellung eines Medikamentes zur Behandlung des metabolischen Syndroms.

12. Verwendung der Verbindungen gemäß einem oder mehreren der Ansprüche 1 bis 4 zur Herstellung eines Medikamentes zur Behandlung von Nikotinabhängigkeit.

13. Verwendung der Verbindungen gemäß einem oder mehreren der Ansprüche 1 bis 4 zur Herstellung eines Medikamentes zur Behandlung von Alkoholabhängigkeit.

14. Verwendung der Verbindungen gemäß einem oder mehreren der Ansprüche 1 bis 4 zur Herstellung eines Medikamentes zur Behandlung von ZNS-Erkrankungen.

15. Verwendung der Verbindungen gemäß einem oder mehreren der Ansprüche 1 bis 4 zur Herstellung eines Medikamentes zur Behandlung von Schizophrenie.

16. Verwendung der Verbindungen gemäß einem oder mehreren der Ansprüche 1 bis 4 zur Herstellung eines Medikamentes zur Behandlung von Alzheimer.

17. Verfahren zur Herstellung eines Arzneimittels enthaltend eine oder mehrere der Verbindungen gemäß einem oder mehreren der Ansprüche 1 bis 4, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 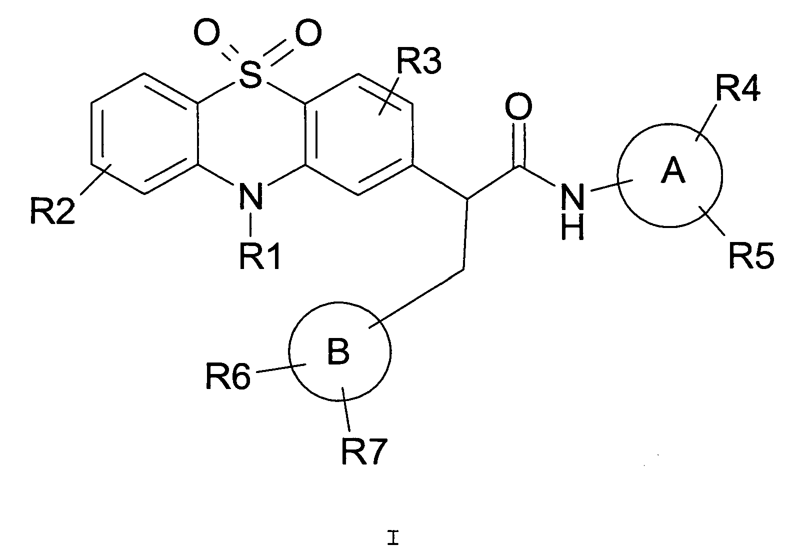 in which
R1 is H, (C₁-C₆)-alkyl, (C₀-C₆)-alkylene-aryl, CO- (C₁-C₆)-alkyl, (C₂-C₆)-alkylene-COO- (C₀-C₆)- alkyl, (C₂-C₆) -alkylene-O- (C₁-C₆)-alkyl;
R2, R3 independently of one another are H, F, Cl, Br, CN, NO₂, (C₀-C₆) -alkylene-COO- (C₀-C₆) -alkyl, (C₀-C₆) -alkylene-O- (C₀-C₆) -alkyl, (C₁-C₆)-alkyl, (C₀-C₆)-alkylene-CO- (C₁-C₆)-alkyl, (C₀-C₆)- alkylene-phenyl, SCF₃, SF₅, SCH₃;
R4, R5 independently of one another are H, F, Cl, Br, CN, SCN, NO₂, =O, (C₀-C₆) -alkylene-COO- (C₀-C₆)- alkyl, -CO-COO- (C₀-C₆)-alkyl, (C₀-C₆)-alkylene- O-(C₀-C₆)-alkyl, (C₁-C₆)-alkyl, (C₀-C₆)- alkylene-CO- (C₁-C₆)-alkyl, (C₀-C₆)-alkylene- CONH(C₀-C₆)-alkyl, (C₀-C₆)-alkylene-CON[(C₀-C₆)- alkyl]₂, (C₀-C₆)-alkylene-NH (C₀-C₆)-alkyl, (C₀- C₆)-alkylene-NH-COO-(C₀-C₆)-alkyl, (C₀-C₆)- alkylene-CON[(C₀-C₆)-alkyl]-O-(C₀-C₆)-alkyl, (C₀-C₆)-alkylene-N[(C₀-C₆)-alkyl]₂, (C₀-C₆)- alkylene-aryl, SF₅, (C₀-C₆)-alkyl-S(O)ₓ(C₁-C₆)- alkyl, S(O)ₓ(C₁-C₆)-alkylene-COO-(C₀-C₆)-alkyl, S(O)ₓ(C₂-C₆)-alkylene-O-(C₀-C₆)-alkyl, -SO₂-NH- (C₀-C₆)-alkyl, -SO₂-N-[(C₀-C₆)-alkyl]₂, S(O)ₓ(C₀-C₆)-alkylene-heterocycle, S(O)ₓ(C₁-C₆)- alkylene-CO-heterocycle, -NH-SO₂-(C₁-C₆)-alkyl, (C₀-C₆)-alkylene-cycloalkyl, (C₀-C₆)-alkylene- heterocycle, (C₀-C₆)-alkylene-aryl;
x is 0, 1, 2;
R6, R7 independently of one another are H, F, Cl, Br, CN, NO₂, =O, =S, =N-O-(C₀-C₆)-alkyl, (C₀-C₆)- alkylene-COO- (C₀-C₆)-alkyl, (C₀-C₆)-alkylene-O- (C₀-C₆)-alkyl, (C₀-C₆)-alkylene-O-CO- (C₁-C₆)- alkyl, (C₁-C₆)-alkyl, (C₀-C₆)-alkylene-CO-(C₁- C₆)-alkyl, (C₀-C₆)-alkylene-aryl, SF₅, S(O)ₓ- (C₁-C₆)-alkyl ;
A is a 5- to 10-membered heterocycle, where the heterocycle may be fused to a further 5- to 10-membered ring;
B is a 4- to 8-membered cycloalkyl ring, a 4- to 10-membered heterocycle or a 6- to 10-membered aryl ring;
or a physiologically acceptable salt thereof,
the definition (C₀-C₆)-alkylene- being understood as meaning that either a bond or a (C₁-C₆)-alkylene group may be present and the definition -(C₀-C₆)-alkyl being understood as meaning that either a hydrogen or a (C₁-C₆)-alkyl group may be present,
a cycloalkyl radical being understood as meaning a ring system which comprises one or more rings and is saturated or partially unsaturated (with one or two double bonds) and is constructed exclusively of carbon atoms.

2. The compound of the formula I as claimed in claim 1, wherein
R1 is H, (C₁-C₆)-alkyl, (C₀-C₆)-alkylene-aryl, CO- (C₁-C₆)-alkyl, (C₂-C₆)-alkylene-COO-(C₀-C₆)- alkyl, (C₂-C₆)-alkylene-O-(C₁-C₆)-alkyl;
R2, R3 independently of one another are H, F, Cl, Br, CN, NO₂, (C₀-C₆)-alkylene-COO- (C₀-C₆)-alkyl, (C₀-C₆)-alkylene-O-(C₀-C₆)-alkyl, (C₁-C₆)-alkyl, (C₀-C₆)-alkylene-CO- (C₁-C₆)-alkyl, (C₀-C₆)- alkylene-phenyl, SCF₃, SF₅, SCH₃;
R4, R5 independently of one another are H, F, Cl, Br, CN, SCN, NO₂, (C₀-C₆)-alkylene-COO-(C₀-C₆)- alkyl, -CO-COO-(C₀-C₆)-alkyl, (C₀-C₆)-alkylene- O-(C₀-C₆)-alkyl, (C₁-C₆)-alkyl , (C₀-C₆)- alkylene-CO- (C₁-C₆)-alkyl, (C₀-C₆)-alkylene- CONH (C₀-C₆)-alkyl, (C₀-C₆)-alkylene-CON[(C₀-C₆)- alkyl]₂, (C₀-C₆)-alkylene-NH (C₀-C₆)-alkyl, (C₀- C₆)-alkylene-NH-COO- (C₀-C₆)-alkyl, (C₀-C₆)- alkylene-CON [(C₀-C₆)-alkyl]-O-(C₀-C₆)-alkyl, (C₀-C₆)-alkylene-N [(C₀-C₆)-alkyl]₂, (C₀-C₆)- alkylene-aryl, SF₅, (C₀-C₆)-alkyl-S(O)ₓ(C₁-C₆)- alkyl, S(O)ₓ(C₁-C₆)-alkylene-COO- (C₀-C₆)-alkyl, S(O)ₓ(C₂-C₆)-alkylene-O-(C₀-C₆)-alkyl, -SO₂-NH- (C₀-C₆)-alkyl, -SO₂-N-[(C₀-C₆)-alkyl]₂, S(O)ₓ(C₀-C₆)-alkylene-heterocycle, S(O)ₓ(C₁-C₆)- alkylene-CO-heterocycle, -NH-SO₂-(C₁-C₆)-alkyl, (C₀-C₆)-alkylene-cycloalkyl, (C₀-C₆)-alkylene- heterocycle, (C₀-C₆)-alkylene-aryl;
x is 0, 1, 2;
R6, R7 independently of one another are H, F, Cl, Br, CN, NO₂, =O, =S, =N-O-(C₀-C₆)-alkyl, (C₀-C₆)- alkylene-COO-(C₀-C₆)-alkyl, (C₀-C₆)-alkylene-O- (C₀-C₆)-alkyl, (C₀-C₆)-alkylene-O-CO-(C₁-C₆)- alkyl, (C₁-C₆)-alkyl, (C₀-C₆)-alkylene-CO-(C₁- C₆)-alkyl, (C₀-C₆)-alkylene-aryl, SF₅, S(O)ₓ- (C₁-C₆)-alkyl;
A is a 5- to 10-membered heterocycle, where the heterocycle may be fused to a further 5- to 10-membered ring;
B is a 4- to 8-membered cycloalkyl ring, a 4- to 10-membered heterocycle or a 6- to 10-membered aryl ring;
or a physiologically acceptable salt thereof.

3. The compound of the formula I as claimed in claim 1 or 2, wherein
R1 is H, (C₁-C₆)-alkyl, (C₀-C₆)-alkylene-aryl, CO- (C₁-C₆)-alkyl, (C₂-C₆)-alkylene-COO- (C₀-C₆)- alkyl, (C₂-C₆)-alkylene-O- (C₁-C₆)-alkyl ;
R2, R3 independently of one another are H, F, Cl, Br, CN, NO₂, (C₀-C₆)-alkylene-COO- (C₀-C₆)-alkyl , (C₀-C₆)-alkylene-O-(C₀-C₆)-alkyl, (C₁-C₆) -alkyl, (C₀-C₆)-alkylene-CO-(C₁-C₆)-alkyl, (C₀-C₆)- alkylene-phenyl, SCF₃, SF₅, SCH₃;
R4, R5 independently of one another are H, F, Cl, Br, CN, SCN, NO₂, (C₀-C₆)-alkylene-COO-(C₀-C₆)- alkyl, -CO-COO- (C₀-C₆)-alkyl, (C₀-C₆)-alkylene- O-(C₀-C₆)-alkyl, (C₁-C₆)-alkyl, (C₀-C₆)- alkylene-CO-(C₁-C₆)-alkyl, (C₀-C₆)-alkylene- CONH(C₀-C₆)-alkyl, (C₀-C₆)-alkylene-CON[(C₀-C₆)- alkyl]₂, (C₀-C₆)-alkylene-NH (C₀-C₆)-alkyl, (C₀- C₆)-alkylene-NH-COO- (C₀-C₆)-alkyl, (C₀-C₆)- alkylene-CON [(C₀-C₆)-alkyl]-O-(C₀-C₆)-alkyl, (C₀-C₆)-alkylene-N [(C₀-C₆)-alkyl]₂, (C₀-C₆)- alkylene-aryl, SF₅, (C₀-C₆)-alkyl-S(O)ₓ(C₁-C₆)- alkyl, S(O)ₓ(C₁-C₆)-alkylene-COO-(C₀-C₆)-alkyl, S(O)ₓ(C₂-C₆)-alkylene-O-(C₀-C₆)-alkyl, -SO₂-NH- (C₀-C₆)-alkyl, -SO₂-N-[(C₀-C₆)-alkyl]₂, S(O)ₓ(C₀-C₆)-alkylene-heterocycle, S(O)ₓ(C₁-C₆)- alkylene-CO-heterocycle, -NH-SO₂-(C₁-C₆)-alkyl, (C₀-C₆)-alkylene-cycloalkyl, (C₀-C₆)-alkylene- heterocycle, (C₀-C₆)-alkylene-aryl;
x is 0, 1, 2;
R6, R7 independently of one another are H, F, Cl, Br, CN, NO₂, =O, =S, =N-O-(C₀-C₆)-alkyl, (C₀-C₆)- alkylene-COO-(C₀-C₆)-alkyl, (C₀-C₆)-alkylene-O- (C₀-C₆)-alkyl, (C₀-C₆)-alkylene-O-CO- (C₁-C₆)- alkyl, (C₁-C₆)-alkyl, (C₀-C₆)-alkylene-CO- (C₁- C₆-alkyl, (C₀-C₆)-alkylene-aryl, SF₅, S(O)ₓ- (C₁-C₆)-alkyl;
A is a 5- to 10-membered heterocycle which contains a -C=N- bond in the alpha position, where the heterocycle may be fused to a further 5- to 10-membered ring;
B is a 4- to 8-membered cycloalkyl ring, a 4- to 10-membered heterocycle or a 6- to 10-membered aryl ring;
or a physiologically acceptable salt thereof.

4. The compound of the formula I as claimed in one or more of claims 1 to 3, wherein
R1 is H, (C₁-C₆)-alkyl, (C₀-C₆)-alkylene-aryl, CO- (C₁-C₆)-alkyl, (C₂-C₆)-alkylene-COO-(C₀-C₆)- alkyl, (C₂-C₆)-alkylene-O-(C₁-C₆)-alkyl;
R2, R3 are H;
R4, R5 independently of one another are H, F, Cl, Br, CN, SCN, NO₂, (C₀-C₆)-alkylene-COO-(C₀-C₆)- alkyl, -CO-COO- (C₀-C₆)-alkyl, (C₀-C₆)-alkylene- O-(C₀-C₆)-alkyl, (C₁-C₆)-alkyl, (C₀-C₆)- alkylene-CO- (C₁-C₆)-alkyl, (C₀-C₆)-alkylene- CONH(C₀-C₆)-alkyl, (C₀-C₆)-alkylene-CON[(C₀-C₆)- alkyl]₂, (C₀-C₆)-alkylene-NH(C₀-C₆)-alkyl, (C₀- C₆) -alkylene-NH-COO- (C₀-C₆)-alkyl, (C₀-C₆)- alkylene-CON [(C₀-C₆)-alkyl]-O-(C₀-C₆)-alkyl, (C₀-C₆)-alkylene-N[(C₀-C₆)-alkyl]₂, (C₀-C₆)- alkylene-aryl, SF₅, (C₀-C₆)-alkyl-S(O)ₓ(C₁-C₆)- alkyl, S(O)ₓ(C₁-C₆)-alkylene-COO-(C₀-C₆)-alkyl, S(O)ₓ(C₂-C₆)-alkylene-O-(C₀-C₆)-alkyl, -SO₂-NH- (C₀-C₆)-alkyl, -SO₂-N-[(C₀-C₆)-alkyl]₂, S(O)ₓ(C₀-C₆)-alkylene-heterocycle, S(O)ₓ(C₁-C₆)- alkylene-CO-heterocycle, -NH-SO₂-(C₁-C₆)-alkyl, (C₀-C₆)-alkylene-cycloalkyl, (C₀-C₆)-alkylene- heterocycle, (C₀-C₆)-alkylene-aryl;
x is 0, 1, 2;
R6, R7 independently of one another are H, F, Cl, Br, CN, NO₂, =O, =S, =N-O-(C₀-C₆)-alkyl, (C₀-C₆)- alkylene-COO- (C₀-C₆)-alkyl, (C₀-C₆)-alkylene-O- (C₀-C₆)-alkyl, (C₀-C₆)-alkylene-O-CO- (C₁-C₆)- alkyl, (C₁-C₆)-alkyl, (C₀-C₆)-alkylene-CO- (C₁- C₆)-alkyl, (C₀-C₆)-alkylene-aryl, SF₅, S(O)ₓ- (C₁-C₆)-alkyl;
A is a 5-membered heterocycle which contains a - C=N- bond in the alpha position, where the heterocycle may be fused to a further 5- to 10-membered ring;
B is 4- to 8-membered cycloalkyl ring, a 4- to 10-membered heterocycle or a 6- to 10-membered aryl ring;
or a physiologically acceptable salt thereof.

5. The compound according to one or more of claims 1 to 4 for use as a pharmaceutical.

6. A pharmaceutical which comprises one or more compounds according to one or more of claims 1 to 4.

7. A pharmaceutical which comprises one or more compounds according to one or more of claims 1 to 4 and at least one further active ingredient.

8. The pharmaceutical as claimed in claim 7, which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose-1,6-biphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

9. The use of a compound as claimed in one or more of claims 1 to 4 for manufacturing a medicament for the treatment of diabetes.

10. The use of a compound as claimed in one or more of claims 1 to 4 for manufacturing a medicament for lowering blood glucose levels.

11. The use of a compound as claimed in one or more of claims 1 to 4 for manufacturing a medicament for the treatment of metabolic syndrome.

12. The use of a compound as claimed in one or more of claims 1 to 4 for manufacturing a medicament for the treatment of nicotine addiction.

13. The use of a compound as claimed in one or more of claims 1 to 4 for manufacturing a medicament for the treatment of alcohol addiction.

14. The use of a compound as claimed in one or more of claims 1 to 4 for manufacturing a medicament for the treatment of CNS disorders.

15. The use of a compound as claimed in one or more of claims 1 to 4 for manufacturing a medicament for the treatment of schizophrenia.

16. The use of a compound as claimed in one or more of claims 1 to 4 for manufacturing a medicament for the treatment of Alzheimer's disease.

17. A method for manufacturing a pharmaceutical comprising one or more compounds according to one or more of claims 1 to 4, which comprises mixing the active ingredient with a pharmaceutically acceptable carrier, and converting this mixture into a form suitable for administration.

## Revendications

1. Composés de formule I, 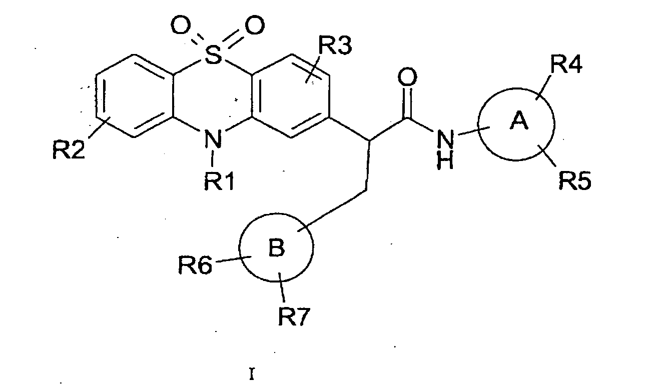 où
R1 signifie H, (C₁-C₆)-alkyle, (C₀-C₆)-alkylène-aryle, CO- (C₁-C₆)-alkyle, (C₂-C₆)-alkylène-COO-(C₀-C₆)- alkyle, (C₂-C₆)-alkylène-O-(C₁-C₆)-alkyle ;
R2, R3 signifient, indépendamment l'un de l'autre H, F, Cl, Br, CN, NO₂, (C₀-C₆)-alkylène-COO-(C₀-C₆)- alkyle, (C₀-C₆)-alkylène-O- (C₀-C₆)-alkyle, (C₁-C₆)- alkyle, (C₀-C₆)-alkylène-CO-(C₁-C₆)-alkyle, (C₀-C₆)- alkylène-phényle, SCF₃, SF₅, SCH₃ ;
R4, R5 signifient indépendamment l'un de l'autre H, F, Cl, Br, CN, SCN, NO₂, =O, (C₀-C₆)-alkylène-COO-(C₀- C₆)-alkyle, -CO-COO- (C₀-C₆)-alkyle, (C₀-C₆)- alkylène-O- (C₀-C₆)-alkyle, (C₁-C₆)-alkyle, (C₀-C₆)- alkylène-CO-(C₁-C₆)-alkyle, (C₀-C₆)-alkylène- CONH(C₀-C₆)-alkyle, (C₀-C₆)-alkylène-CON[(C₀-C₆)- alkyle]₂, (C₀-C₆)-alkylène-NH(C₀-C₆)-alkyle, (C₀-C₆)- x alkylène-NH-COO-(C₀-C₆)-alkyle, (C₀-C₆)-alkylène- CON [(C₀-C₆)-alkyl]-O-(C₀-C₆)-alkyle, (C₀-C₆)- alkylène-N[(C₀-C₆)-alkyle]₂, (C₀-C₆)-alkylène-aryle, SF₅, (C₀-C₆)-alkyl-S(O)ₓ(C₁-C₆)-alkyle, S(O)ₓ(C₁-C₆)- alkylène-COO- (C₀-C₆)-alkyle, S(O)ₓ(C₂-C₆)-alkylène- O-(C₀-C₆)-alkyle, -SO₂-NH-(C₀-C₆)-alkyle, -SO₂-N- [(C₀-C₆)-alkyle]₂,S(O)ₓ(C₀-C₆)-alkylène- hétérocycle, S(O)ₓ(C₁-C₆)-alkylène-CO-hétérocycle, -NH-SO₂-(C₁-C₆)-alkyle, (C₀-C₆)-alkylène- cycloalkyle, (C₀-C₆)-alkylène-hétérocycle, (C₀-C₆)- alkylène-aryle ; vaut 0, 1, 2 ;
R6, R7 signifient, indépendamment l'un de l'autre, H, F, Cl, Br, CN, NO₂, =O, =S, =N-O-(C₀-C₆)-alkyle, (C₀-C₆)-alkylène-COO- (C₀-C₆)-alkyle, (C₀-C₆)- alkylène-O-(C₀-C₆)-alkyle, (C₀-C₆)-alkylène-O-CO- (C₁-C₆)-alkyle, (C₁-C₆)-alkyle, (C₀-C₆)-alkylène-CO- (C₁-C₆)-alkyle, (C₀-C₆)-alkylène-aryle, SF₅, S(O)ₓ- (C₁-C₆)-alkyle ;
A signifie un hétérocycle de 5 à 10 chaînons, où l'hétérocycle peut être condensé avec un autre cycle de 5 à 10 chaînons ;
B signifie un cycle cycloalkyle de 4 à 8 chaînons, un hétérocycle de 4 à 10 chaînons ou un cycle aryle de 6 à 10 chaînons ;
ainsi que leurs sels physiologiquement acceptables,
- où on entend par la définition (C₀-C₆)-alkylène- que soit une liaison, soit un groupe (C₁-C₆)- alkylène peut être présent et où on entend par la définition -(C₀-C₆)-alkyle que soit un hydrogène, soit un groupe (C₁-C₆)-alkyle peut être présent,
- où on entend par radical cycloalkyle un système cyclique contenant un ou plusieurs cycles, qui peut être saturé ou partiellement insaturé (avec une ou deux doubles liaisons) qui est exclusivement constitué par des atomes de carbone.

2. Composés de formule I, selon la revendication 1, **caractérisés en ce que**
R1 signifie H, (C₁-C₆)-alkyle, (C₀-C₆)-alkylène-aryle, CO- (C₁-C₆)-alkyle, (C₂-C₆)-alkylène-COO-(C₀-C₆)- alkyle, (C₂-C₆)-alkylène-O-(C₁-C₆)-alkyle ;
R2, R3 signifient, indépendamment l'un de l'autre H, F, Cl, Br, CN, NO₂, (C₀-C₆)-alkylène-COO-(C₀-C₆)- alkyle, (C₀-C₆)-alkylène-O- (C₀-C₆)-alkyle, (C₁-C₆)- alkyle, (C₀-C₆)-alkylène-CO- (C₁-C₆)-alkyle, (C₀-C₆)- alkylène-phényle, SCF₃, SF₅, SCH₃ ;
R4, R5 signifient indépendamment l'un de l'autre H, F, Cl, Br, CN, SCN, NO₂, (C₀-C₆)-alkylène-COO-(C₀-C₆)- alkyle, -CO-COO-(C₀-C₆)-alkyle, (C₀-C₆)-alkylène-O- (C₀-C₆)-alkyle, (C₁-C₆)-alkyle, (C₀-C₆)-alkylène-CO- (C₁-C₆)-alkyle, (C₀-C₆)-alkylène-CONH(C₀-C₆)-alkyle, (C₀-C₆)-alkylène-CON[(C₀-C₆)-alkyle]₂, (C₀-C₆)- alkylène-NH(C₀-C₆)-alkyle, (C₀-C₆)-alkylène-NH-COO- (C₀-C₆)-alkyle, (C₀-C₆)-alkylène-CON [(C₀-C₆)-alkyl]- O-(C₀-C₆)-alkyle, (C₀-C₆)-alkylène-N[(C₀-C₆)- alkyle]₂, (C₀-C₆)-alkylène-aryle, SF₅, (C₀-C₆)- alkyl-S(O)ₓ(C₁-C₆)-alkyle, S(O)ₓ(C₁-C₆)-alkylène- COO-(C₀-C₆)-alkyle, S(O)ₓ(C₂-C₆)-alkylène-O-(C₀-C₆)- alkyle, -SO₂-NH-(C₀-C₆)-alkyle, -SO₂-N-[(C₀-C₆)- alkyle]₂, S(O)ₓ(C₀-C₆)-alkylène-hétérocycle, S(O)ₓ(C₁-C₆)-alkylène-CO-hétérocycle, -NH-SO₂-(C₁- C₆)-alkyle, (C₀-C₆)-alkylène-cycloalkyle, (C₀-C₆)- alkylène-hétérocycle, (C₀-C₆)-alkylène-aryle ;
x vaut 0, 1, 2 ;
R6, R7 signifient, indépendamment l'un de l'autre, H, F, Cl, Br, CN, NO₂, =O, =S, =N-O-(C₀-C₆)-alkyle, (C₀-C₆)-alkylène-COO- (C₀-C₆)-alkyle, (C₀-C₆)- alkylène-O-(C₀-C₆)-alkyle, (C₀-C₆)-alkylène-O-CO- (C₁-C₆)-alkyle, (C₁-C₆)-alkyle, (C₀-C₆)-alkylène-CO- (C₁-C₆)-alkyle, (C₀-C₆)-alkylène-aryle, SF₅, S(O)ₓ- (C₁-C₆)-alkyle ;
A signifie un hétérocycle de 5 à 10 chaînons, où l'hétérocycle peut être condensé avec un autre cycle de 5 à 10 chaînons ;
B signifie un cycle cycloalkyle de 4 à 8 chaînons, un hétérocycle de 4 à 10 chaînons ou un cycle aryle de 6 à 10 chaînons ;
ainsi que leurs sels physiologiquement acceptables.

3. Composés de formule I, selon la revendication 1 ou 2, **caractérisés en ce que**
R1 signifie H, (C₁-C₆)-alkyle, (C₀-C₆)-alkylène-aryle, CO- (C₁-C₆)-alkyle, (C₂-C₆)-alkylène-COO- (C₀-C₆)- alkyle, (C₂-C₆)-alkylène-O-(C₁-C₆)-alkyle ;
R2, R3 signifient, indépendamment l'un de l'autre H, F, Cl, Br, CN, NO₂, (C₀-C₆)-alkylène-COO-(C₀-C₆)- alkyle, (C₀-C₆)-alkylène-O-(C₀-C₆)-alkyle, (C₁-C₆)- alkyle, (C₀-C₆)-alkylène-CO-(C₁-C₆)-alkyle, (C₀-C₆)- alkylène-phényle, SCF₃, SF₅, SCH₃ ;
R4, R5 signifient indépendamment l'un de l'autre H, F, Cl, Br, CN, SCN, NO₂, (C₀-C₆)-alkylène-COO- (C₀-C₆)- alkyle, -CO-COO- (C₀-C₆)-alkyle, (C₀-C₆)-alkylène-O- (C₀-C₆)-alkyle, (C₁-C₆)-alkyle, (C₀-C₆)-alkylène-CO- (C₁-C₆)-alkyle, (C₀-C₆)-alkylène-CONH(C₀-C₆)-alkyle, (C₀-C₆)-alkylène-CON [(C₀-C₆)-alkyle]₂, (C₀-C₆)- alkylène-NH(C₀-C₆)-alkyle, (C₀-C₆)-alkylène-NH-COO- (C₀-C₆)-alkyle , (C₀-C₆)-alkylène-CON [(C₀-C₆)-alkyl]- O-(C₀-C₆)-alkyle, (C₀-C₆)-alkylène-N [(C₀-C₆)- alkyle]₂, (C₀-C₆)-alkylène-aryle, SF₅, (C₀-C₆)- alkyl-S(O)ₓ(C₁-C₆)-alkyle, S(O)ₓ(C₁-C₆)-alkylène- COO- (C₀-C₆)-alkyle, S(O)ₓ(C₂-C₆)-alkylène-O-(C₀-C₆)- alkyle, -SO₂-NH-(C₀-C₆)-alkyle, -SO₂-N-[ (C₀-C₆)- alkyle]₂, S(O)ₓ(C₀-C₆)-alkylène-hétérocycle, S(O)ₓ(C₁-C₆)-alkylène-CO-hétérocycle, -NH-SO₂-(C₁- C₆)-alkyle, (C₀-C₆)-alkylène-cycloalkyle, (C₀-C₆)- alkylène-hétérocycle, (C₀-C₆)-alkylène-aryle;
x vaut 0, 1, 2 ;
R6, R7 signifient, indépendamment l'un de l'autre, H, F, Cl, Br, CN, NO₂, =O, =S, =N-O-(C₀-C₆)-alkyle, (C₀-C₆)-alkylène-COO- (C₀-C₆)-alkyle, (C₀-C₆)- alkylène-O-(C₀-C₆)-alkyle, (C₀-C₆)-alkylène-O-CO- (C₁-C₆)-alkyle, (C₁-C₆)-alkyle, (C₀-C₆)-alkylène-CO- (C₁-C₆)-alkyle, (C₀-C₆)-alkylène-aryle, SF₅, S (O)ₓ- (C₁-C₆)-alkyle ;
A signifie un hétérocycle de 5 à 10 chaînons, qui contient en position alpha une liaison -C=N-, où l'hétérocycle peut être condensé avec un autre cycle de 5 à 10 chaînons ;
B signifie un cycle cycloalkyle de 4 à 8 chaînons, un hétérocycle de 4 à 10 chaînons ou un cycle aryle de 6 à 10 chaînons ;
ainsi que leurs sels physiologiquement acceptables.

4. Composés de formule I selon l'une ou plusieurs des revendications 1 à 3, **caractérisés en ce que**
R1 signifie H, (C₁-C₆)-alkyle, (C₀-C₆)-alkylène-aryle, CO- (C₁-C₆)-alkyle, (C₂-C₆)-alkylène-COO- (C₀-C₆)- alkyle, (C₂-C₆)-alkylène-O-(C₁-C₆)-alkyle ;
R2, R3 signifient H ;
R4, R5 signifient indépendamment l'un de l'autre H, F, Cl, Br, CN, SCN, NO₂, (C₀-C₆)-alkylène-COO-(C₀-C₆)- alkyle, -CO-COO- (C₀-C₆)-alkyle, (C₀-C₆)-alkylène-O- (C₀-C₆)-alkyle, (C₁-C₆)-alkyle, (C₀-C₆)-alkylène-CO- (C₁-C₆)-alkyle, (C₀-C₆)-alkylène-CONH(C₀-C₆)-alkyle, (C₀-C₆)-alkylène-CON[(C₀-C₆)-alkyle]₂, (C₀-C₆)- alkylène-NH (C₀-C₆)-alkyle, (C₀-C₆)-alkylène-NH-COO- (C₀-C₆)-alkyle, (C₀-C₆)-alkylène-CON [(C₀-C₆)-alkyl]- O-(C₀-C₆)-alkyle, (C₀-C₆)-alkylène-N [(C₀-C₆)- alkyle] ₂, (C₀-C₆)-alkylène-aryle, SF₅, (C₀-C₆)- alkyl-S(O)ₓ(C₁-C₆)-alkyle, S (O)ₓ(C₁-C₆)-alkylène- COO- (C₀-C₆)-alkyle, S (O)ₓ(C₂-C₆)-alkylène-O-(C₀-C₆)- alkyle, -SO₂-NH-(C₀-C₆)-alkyle, -SO₂-N-[ (C₀-C₆)- alkyle]₂, S(O)ₓ(C₀-C₆)-alkylène-hétérocycle, S (O)ₓ(C₁-C₆)-alkylène-CO-hétérocycle, -NH-SO₂-(C₁- C₆)-alkyle, (C₀-C₆)-alkylène-cycloalkyle, (C₀-C₆)- alkylène-hétérocycle, (C₀-C₆)-alkylène-aryle ;
x vaut 0, 1, 2 ;
R6, R7 signifient, indépendamment l'un de l'autre, H, F, Cl, Br, CN, NO₂, =O, =S, =N-O-(C₀-C₆)-alkyle, (C₀-C₆)-alkylène-COO- (C₀-C₆)-alkyle, (C₀-C₆)- alkylène-O-(C₀-C₆)-alkyle, (C₀-C₆)-alkylène-O-CO- (C₁-C₆)-alkyle, (C₁-C₆)-alkyle, (C₀-C₆)-alkylène-CO- (C₁-C₆)-alkyle, (C₀-C₆)-alkylène-aryle, SF₅, S (O)ₓ- (C₁-C₆)-alkyle ;
A signifie un hétérocycle de 5 chaînons, qui contient en position alpha une liaison -C=N-, où l'hétérocycle peut être condensé avec un autre cycle de 5 à 10 chaînons ;
B signifie un cycle cycloalkyle de 4 à 8 chaînons, un hétérocycle de 4 à 10 chaînons ou un cycle aryle de 6 à 10 chaînons ;
ainsi que leurs sels physiologiquement acceptables.

5. Composés selon l'une ou plusieurs des revendications 1 à 4, destinés à être utilisés comme médicament.

6. Médicament contenant un ou plusieurs des composés selon l'une ou plusieurs des revendications 1 à 4.

7. Médicament contenant un ou plusieurs des composés selon l'une ou plusieurs des revendications 1 à 4 et au moins une autre substance active.

8. Médicament selon la revendication 7, **caractérisé en ce qu'**il contient comme autre substance active un(e) ou plusieurs antidiabétiques, substances actives hypoglycémiques, inhibiteurs de la HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène-synthétase, antagonistes de la lipoprotéine(a), agonistes du récepteur HM74A, inhibiteurs de lipase, insulines, sulfonylurées, biguanides, méglitinides, thiazolidinediones, inhibiteurs de l'α-glucosidase, substances actives agissant sur le canal potassique dépendant d'ATP des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur 4 du glucose, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur 1 ou 2 du glucose dépendant du sodium, modulateurs du GPR40, inhibiteurs de la lipase sensible aux hormones, inhibiteurs de l'acétyl-CoA carboxylase, inhibiteurs de la phosphoénol-pyruvate-carboxykinase, inhibiteurs de la glycogène synthase kinase-3 bêta, inhibiteurs de la protéine kinase C bêta, antagonistes du récepteur de l'endothéline-A, inhibiteurs de la I kappaB kinase, modulateurs du récepteur des glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, antagonistes du récepteur de CB1, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, antagonistes de galanine, hormones de croissance, composés libérant l'hormone de croissance, agonistes de TRH, modulateurs 2 ou 3 de la protéine découplante, agonistes de leptine, agonistes de DA (Bromocriptine, Doprexine), inhibiteurs de lipase/amylase, modulateurs de PPAR, modulateurs de RXR, agonistes de TR-β ou amphétamines.

9. Utilisation des composés selon l'une ou plusieurs des revendications 1 à 4 pour la préparation d'un médicament destiné au traitement du diabète.

10. Utilisation des composés selon l'une ou plusieurs des revendications 1 à 4 pour la préparation d'un médicament destiné à abaisser la glycémie.

11. Utilisation des composés selon l'une ou plusieurs des revendications 1 à 4 pour la préparation d'un médicament destiné au traitement du syndrome métabolique.

12. Utilisation des composés selon l'une ou plusieurs des revendications 1 à 4 pour la préparation d'un médicament destiné au traitement de la dépendance à la nicotine.

13. Utilisation des composés selon l'une ou plusieurs des revendications 1 à 4 pour la préparation d'un médicament destiné au traitement de la dépendance à l'alcool.

14. Utilisation des composés selon l'une ou plusieurs des revendications 1 à 4 pour la préparation d'un médicament destiné au traitement de maladies du système nerveux central.

15. Utilisation des composés selon l'une ou plusieurs des revendications 1 à 4 pour la préparation d'un médicament destiné au traitement de la schizophrénie.

16. Utilisation des composés selon l'une ou plusieurs des revendications 1 à 4 pour la préparation d'un médicament destiné au traitement de la maladie d'Alzheimer.

17. Procédé pour la préparation d'un médicament contenant un ou plusieurs des composés selon l'une ou plusieurs des revendications 1 à 4, **caractérisé en ce que** la substance active est mélangée avec un support pharmaceutiquement approprié et ce mélange est amené dans une forme appropriée pour l'administration.
